(19) Europäisches Patentamt
European Patent Office
Office européen des brevets

(11) EP 4 592 296 A1

(12) **EUROPEAN PATENT APPLICATION**
published in accordance with Art. 153(4) EPC

(43) Date of publication:
**30.07.2025 Bulletin 2025/31**

(21) Application number: **23868630.7**

(22) Date of filing: **21.09.2023**

(51) International Patent Classification (IPC):
***C07D 498/22*** *(2006.01)* ***A61K 31/519*** *(2006.01)*
***A61P 35/00*** *(2006.01)*

(52) Cooperative Patent Classification (CPC):
**A61K 31/519; A61P 35/00; C07D 498/22**

(86) International application number:
**PCT/KR2023/014444**

(87) International publication number:
**WO 2024/063578 (28.03.2024 Gazette 2024/13)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **23.09.2022 KR 20220121129**
**23.12.2022 KR 20220183607**

(71) Applicant: **Idience Co., Ltd.**
**Seocho-gu**
**Seoul 06752 (KR)**

(72) Inventors:
- **HONG, Chang Hee**
  **Hwaseong-si, Gyeonggi-do 18449 (KR)**
- **LEE, Jin Hee**
  **Hwaseong-si, Gyeonggi-do 18449 (KR)**
- **LEE, Joo Yun**
  **Hwaseong-si, Gyeonggi-do 18449 (KR)**
- **PARK, Jong Seon**
  **Hwaseong-si, Gyeonggi-do 18449 (KR)**
- **KIM, Jin Woong**
  **Hwaseong-si, Gyeonggi-do 18449 (KR)**
- **HONG, Soo Jung**
  **Hwaseong-si, Gyeonggi-do 18449 (KR)**
- **HONG, Sung Jun**
  **Hwaseong-si, Gyeonggi-do 18449 (KR)**
- **YOON, Hong Chul**
  **Hwaseong-si, Gyeonggi-do 18449 (KR)**
- **AN, Kyung Mi**
  **Hwaseong-si, Gyeonggi-do 18449 (KR)**
- **PARK, Joon Tae**
  **Hwaseong-si, Gyeonggi-do 18449 (KR)**
- **LEE, Jung Woo**
  **Hwaseong-si, Gyeonggi-do 18449 (KR)**

(74) Representative: **Mewburn Ellis LLP**
**Aurora Building**
**Counterslip**
**Bristol BS1 6BX (GB)**

(54) **NOVEL TETRAHETEROCYCLE COMPOUND**

(57) The present invention relates to a novel tetraheterocycle compound and more specifically, to a novel tetraheterocycle compound useful as a KRAS protein inhibitor, an isomer, and a pharmaceutical composition for cancer treatment comprising same.



Processed by Luminess, 75001 PARIS (FR)

## Description

### Technical Field

The present disclosure relates to a novel tetraheterocyclic compound, and more specifically, to a novel tetraheterocyclic compound useful as a KRAS protein inhibitor, an isomer thereof, and a pharmaceutical composition including the same for the treatment of cancer.

### Background Art

The RAS gene is responsible for signal transduction within the mitogen activated protein kinase (MAPK) and phosphatidylinositol 3 kinase (PI3K) pathways, and is known as an oncogene due to its frequent mutations. The RAS gene family is classified into KRAS, NRAS, and HRAS, which encode four proteins, namely splice variants K-Ras4A and K-Ras4B, and N-Ras and H-Ras. K-Ras is the most frequently mutated isoform in Ras-induced cancers (86 %), followed by N-Ras (11 %) and H-Ras (3 %) (Non-patent Document 1). For example, oncogenic changes in KRAS are observed in 15.95 % of cancers, including pancreatic cancer, lung cancer, colon adenocarcinoma, colorectal cancer, and rectal adenocarcinomas (Non-patent Document 2).

Gain-of-function missense mutations, mostly located at codons 12, 13, and 61, constitutively activate RAS proteins and are detected in a variety of human cancers. 98 % of tumor Ras mutations are found in the active site amino acid residues G12, G13, and Q61, which impair intrinsic and GAP-mediated GTP hydrolysis, thereby aberrantly activating downstream signaling (Non-patent Document 3). K-Ras G12 mutations (89 %) predominate in human cancers, followed by G13 mutations (9 %) and Q61 mutations (1 %). The codon 12 mutation types include codon 12 Gly→Asp (G12D) (36 %), codon 12 Gly→Val (G12V) (23 %), and codon 12 Gly→Cys (G12C) (14 %), with G12D being the most common mutation among codon 12 mutations. Additionally, mutations of codon 13 Gly→Asp (G13D) (7 %) and codon 61 Gln→His (Q61H) (0.6 %) are also observed (Non-patent Document 1).

The well-known role of KRAS in malignancy and the reported occurrence of KRAS mutations in various tumor types suggest that KRAS may be an effective target for cancer therapy. The inventors of the present disclosure completed the present disclosure by developing a novel KRAS inhibitor.

[Prior Art Documents]

[Non-Patent Documents]

(Non-Patent Document 1) Scientific Reports 6(1):21949

(Non-Patent Document 2) J Cancer Metastasis Treat 2021;7:26

(Non-Patent Document 3) Cancer Biol Ther. 2006 August; 5(8): 928-932


## Disclosure of Invention

### Technical Problem

The present disclosure provides a novel tetraheterocyclic compound, an isomer thereof, and a pharmaceutically acceptable salt thereof, and a pharmaceutical composition including the same for treating cancer, a method of preparing the same, and intermediates therefor.

### Solution to Problem

To achieve the above objective, an aspect of the present disclosure provides a compound of Formula 1, or a stereoisomer, a diastereomer, an enantiomer, atropisomer, a solvate thereof, an isotopic variant thereof, a tautomer thereof, or a pharmaceutically acceptable salt thereof.

Another aspect of the present disclosure provides a pharmaceutical composition including a compound of Formula 1, or a stereoisomer, a diastereomer, an enantiomer, atropisomer, a solvate thereof, an isotopic variant thereof, a tautomer thereof, or a pharmaceutically acceptable salt thereof.

Another aspect of the present disclosure provides a method of preparing a compound of Formula 1, or a stereoisomer, a diastereomer, an enantiomer, an atropisomer, a solvate thereof, an isotopic variant thereof, a tautomer thereof, or a pharmaceutically acceptable salt thereof, and an intermediate used therein.

**Advantageous Effects of Invention**

The present disclosure provides a novel tetraheterocyclic compound useful as a KRAS protein inhibitor, or a stereoisomer, a diastereoisomer, an enantiomer, an atropisomer, a solvate thereof, an isotopic variant thereof, a tautomer thereof, or a pharmaceutically acceptable salt thereof, and a pharmaceutical composition including the same, which may exhibit KRAS protein inhibitory activity and may be effectively used in the treatment of cancer.

**Mode for the Invention**

All technical terms used herein, unless otherwise defined, are used as commonly understood by one of ordinary skill in the relevant field of the present disclosure. In addition, while suitable methods or samples are described herein, similar or equivalent methods are also included within the scope of the present disclosure.

**Tetraheterocyclic Compound**

An aspect of the present disclosure provides a compound of the following Formula 1, or a stereoisomer, a diastereomer, an enantiomer, atropisomer, a solvate thereof, an isotopic variant thereof, a tautomer thereof, or a pharmaceutically acceptable salt thereof:

[Formula 1]

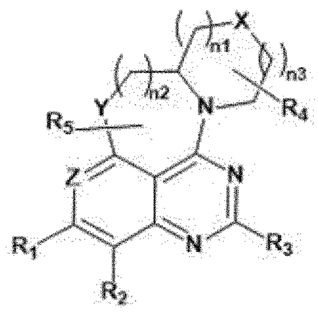

In the formula above,

$R^1$ is phenyl, pyridinyl, naphthyl, indazolyl, benzothiazolyl, or benzothiophenyl, unsubstituted or optionally substituted with one or more substituents independently selected from hydroxy, halogen, $C_{1-3}$ haloalkyl, $C_{1-3}$ alkyl, $C_{2-6}$ alkenyl, $C_{2-6}$ alkynyl, $C_{3-6}$ cycloalkyl, $C_{1-3}$ alkoxy, amino, and cyano;
$R^2$ is hydrogen or halogen;
$R^3$ is hydrogen, $C_{1-3}$ alkyl, $C_{3-6}$ cycloalkyl, 4-10 membered heterocycle, or $-O-(L)_m-A_1$, wherein $A_1$ is $C_{1-3}$ alkyl, $C_{3-6}$ cycloalkyl, 4-10 membered heterocycle, 6-10 membered aryl, 5-10 membered heteroaryl, or 4-10 membered fused heteroaryl, each of which is substituted or unsubstituted with one or more $R^7$;
L is $C_{1-3}$ alkylene or $C_{3-8}$ cycloalkylene, each of which is substituted or unsubstituted with $R^7$;
$R^7$ is each independently halogen, oxo(=O), $=CH_2$, $-OCF_3$, $-OCHF_2$, amino, cyano, $-N(C_{1-3}\ alkyl)_2$, $-NH(C_{1-3}\ alkyl)$, substituted or unsubstituted $C_{1-3}$ alkyl, substituted or unsubstituted $C_{1-3}$ haloalkyl, substituted or unsubstituted $C_{1-3}$ alkoxy, substituted or unsubstituted $C_{3-4}$ cycloalkyl, or substituted or unsubstituted heterocycle;
$R^4$ is each independently hydrogen, hydroxy, halogen, $C_{1-3}$ haloalkyl, $C_{1-3}$ alkyl, or $C_{1-3}$ alkoxy;
X is O, $CH_2$ or $NR^8$;
$R^8$ is hydrogen or $C_{1-6}$ alkyl, $C_{3-6}$ cycloalkyl, or $C_{3-6}$ heterocycloalkyl optionally substituted 1-3 times with $R^9$;
$R^9$ is independently oxygen, hydroxy, $-C_{1-4}$ alkyl or $-O-C_{1-4}$ alkyl in each case;
Y is NH, O, S, SO, or $SO_2$;
Z is $CR^6$ or N;
$R^5$ is hydrogen, $C_{1-3}$ alkyl, cyano, or amino;
$R^6$ is hydrogen, hydroxy, halogen, $C_{1-3}$ alkyl;
n1, n2, n3, and m are each an integer of 1 to 3;
Heterocycle, heteroaryl, and fused heteroaryl each include one or more of N, S or O as a heteroatom.

In an embodiment,

$R^1$ may be phenyl, naphthyl, benzothiazolyl, or benzothiophenyl, which is unsubstituted or optionally substituted with one or more substituents independently selected from hydroxy, halogen, $C_{1-3}$ haloalkyl, $C_{1-3}$ alkyl, $C_{2-6}$ alkenyl, $C_{2-6}$ alkynyl, $C_{3-6}$ cycloalkyl, $C_{1-3}$ alkoxy, amino, and cyano;
X may be O, $CH_2$, or NH;
Y may be O.

In an embodiment,
$R^3$ may be -O-$(L)_m$-$A_1$, wherein $A_1$ may be a 4-10 membered heterocycle or a 5-10 membered fused heteroaryl, each of which is substituted or unsubstituted with one or more $R^7$.

In an embodiment,

$R^1$ is unsubstituted or optionally substituted with one or more substituents independently selected from hydroxy, halogen, $C_{1-3}$ alkyl, $C_{2-6}$ alkenyl, $C_{2-6}$ alkynyl, $C_{3-6}$ cycloalkyl, amino, and cyano;
The compound of claim 1,
$R^1$ is

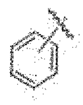 , 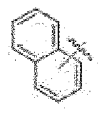 , 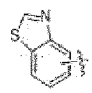 , or 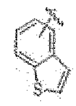 ,

which is unsubstituted or optionally substituted with one or more substituents independently selected from hydroxy, halogen, $C_{1-3}$ alkyl, $C_{2-6}$ alkenyl, $C_{2-6}$ alkynyl, $C_{3-6}$ cycloalkyl, amino, and cyano;
$R^3$ is -O-$(L)_m$-$A_1$, wherein $A_1$ is a 4-10 membered heterocycle or a 5-10 membered fused heteroaryl, each of which is substituted or unsubstituted with one or more $R^7$;
L is $C_{1-3}$ alkylene or $C_{3-8}$ cycloalkylene;
$R^7$ is each independently halogen, oxo (=O), =$CH_2$, -$OCF_3$, -$OCHF_2$, amino, cyano, $C_{1-3}$ alkyl, $C_{1-3}$ haloalkyl, or $C_{1-3}$ alkoxy;
$R^4$ is hydrogen;
X is O, $CH_2$, or NH;
Z is $CR^6$ or N;
$R^5$ is hydrogen or $C_{1-3}$ alkyl;
$R^6$ is halogen;
and n1, n2, n3, and m are each independently an integer of 1 to 3.

In an embodiment,

when Z is N,
$R^1$ may be phenyl or naphthyl, which is unsubstituted or optionally substituted with one or more substituents independently selected from hydroxy, halogen, $C_{1-3}$ haloalkyl, $C_{1-3}$ alkyl, $C_{2-6}$ alkenyl, $C_{2-6}$ alkynyl, $C_{3-6}$ cycloalkyl, $C_{1-3}$ alkoxy, amino, and cyano;
when Z is $CR^6$,
$R^1$ may be benzothiazolyl or benzothiophenyl, which is unsubstituted or optionally substituted with one or more substituents independently selected from hydroxy, halogen, $C_{1-3}$ haloalkyl, $C_{1-3}$ alkyl, $C_{2-6}$ alkenyl, $C_{2-6}$ alkynyl, $C_{3-6}$ cycloalkyl, $C_{1-3}$ alkoxy, amino, and cyano.

In an embodiment,

$R^3$ may be -O-$(L)_m$-$A_2$, wherein $A_2$ may be

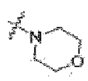 , 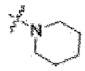 , 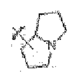 , 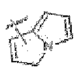 , 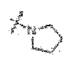 ,

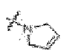 , or 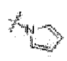 ,

each of which is unsubstituted or optionally substituted with one or more substituents independently selected from

halogen, $C_{1-3}$ alkyl, $C_{1-3}$ alkoxy, and $=CH_2$,

and $-(L)_m-$ may be methylene or

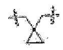.

In an embodiment,

$R^1$ may be

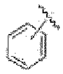, 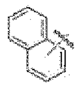, 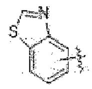, or 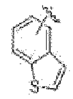,

which is unsubstituted or optionally substituted with one or more substituents independently selected from hydroxy, halogen, $C_{1-3}$ alkyl, $C_{2-6}$ alkenyl, $C_{2-6}$ alkynyl, $C_{3-6}$ cycloalkyl, amino, and cyano;
$R^2$ is halogen;
$R^3$ may be $-O-(L)_m-A_1$, wherein $A_1$ is

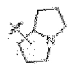, 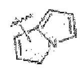, 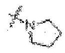, 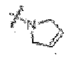, or 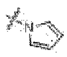,

each of which is unsubstituted or optionally substituted with one or more substituents independently selected from halogen, $C_{1-3}$ alkyl, $C_{1-3}$ alkoxy, and $=CH_2$, and $-(L)_m-$ may be methylene or

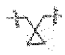;

$R^4$ may be hydrogen;
X may be O, $CH_2$, or NH;
Y may be O;
Z may be CH, $C(C_{1-3}$ alkyl), or N;
and $R^5$ may be hydrogen or $C_{1-3}$ alkyl.

In an embodiment, the compound of Formula 1 may be any one compound selected from the group consisting of the following compounds, or a stereoisomer, a diastereomer, an enantiomer, an atropisomer, a solvate thereof, an isotopic variant thereof, a tautomer thereof, or a pharmaceutically acceptable salt thereof:

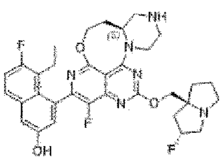, 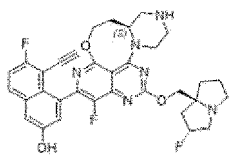,

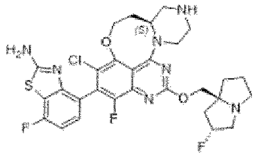, 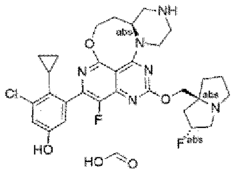,

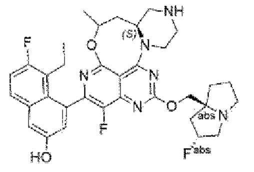,
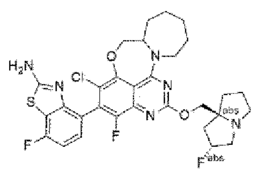,

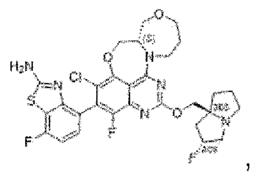,
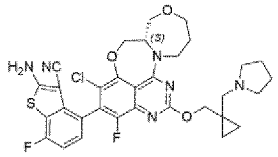,

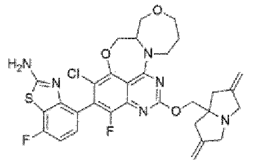,
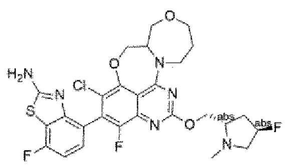,

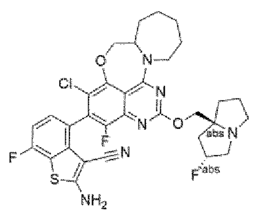,
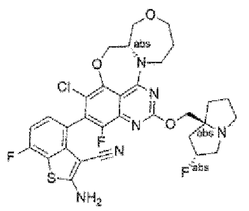,

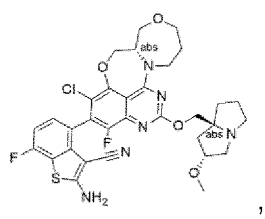,
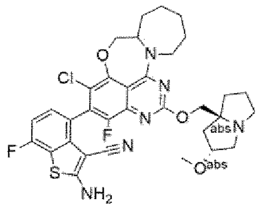,

and

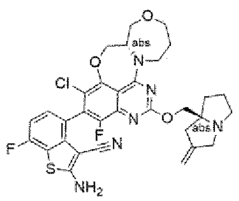.

Another aspect provides a pharmaceutical composition for treating cancer, including as an active ingredient any one of the compounds mentioned above, or a stereoisomer, a diastereomer, an enantiomer, atropisomer, a solvate thereof, an isotopic variant thereof, a tautomer thereof, or a pharmaceutically acceptable salt thereof.
In an embodiment, the composition may exhibit KRAS protein inhibitory activity.

## Definition

The term "halogen" as used herein, unless otherwise stated, refers to fluorine, chlorine, bromine or iodine.
The term "alkyl" as used herein, unless otherwise stated, refers to a straight-chain or branched saturated monovalent hydrocarbon group.

The term "alkylene" as used herein refers to a divalent straight-chain or branched hydrocarbon group having $(-CH_2-)_n$, including but not limited to methylene, ethylene, propylene, butylene, and isobutylene, etc.

The term "alkenyl" as used in the present disclosure, unless otherwise stated, refers to a monovalent hydrocarbon group including at least one carbon-carbon double bond, each double bond being capable of having an E- or Z-type stereo configuration.

The term "alkynyl" as used in the present disclosure, unless otherwise stated, refers to a monovalent hydrocarbon radical including at least one carbon-carbon triple bond.

The term "alkoxy" as used herein, unless otherwise stated, refers to a straight-chain or branched hydrocarbon moiety linked to oxygen.

The term "aryl" as used herein, unless otherwise stated, refers to an aromatic group which may be substituted or unsubstituted, and includes monocyclic or bicyclic, or more than bicyclic, substituted or unsubstituted aromatic groups, and includes unsaturated or partially saturated aryl, and may include, for example, $C_{6-15}$ aryl, including, for example, but not limited to, phenyl, biphenyl, naphthyl, toluyl, or naphthalenyl, etc.

The term "heteroaryl" as used herein, unless otherwise stated, refers to a monocyclic or bicyclic, or more than bicyclic, substituted or unsubstituted aromatic group including one or more heteroatoms selected from N, O, and S, including unsaturated or partially saturated heteroaryls, and may include, for example, $C_{4-15}$ heteroaryls. Examples may include, but are not limited to, morpholinyl, piperidinyl, pyrrolidinyl, or pyrrolizinyl.

The term "fused heteroaryl" as used herein refers to an unsaturated or partially saturated, substituted or unsubstituted ring system in which the heteroaryl group is linked in a fused manner to another aryl, heteroaryl or heterocycloalkyl group. For example, it may include a $C_{8-20}$ heteroaryl, and may be, for example, a 9 membered, 10 membered, 11 membered, 12 membered, 13 membered, 14 membered or 15 membered benzo-fused heteroaryl group. For example, a fused heteroaryl may form a 5+5 membered, 5+6 membered, 5+7 membered, 6+6 membered, or 6+7 membered fused ring system. Additionally, the fused heteroaryl may be, for example, pyrrolizin, benzothiazole, benzothiazolyl, benzothiophenyl, benzofuranyl, isobenzofuranyl, benzothionyl, indolyl, isindolinyl, indazolinyl, benzimidazolinyl, benzoxazolinyl, benzoisoxazolinyl, benzothiadiazolyl, benzoxadiazolinyl, benzotriazolinyl, quinolinyl, isoquinolinyl, or quinazolinyl, etc., but is not limited thereto.

The term "partially saturated" as used herein refers to an aryl, heteroaryl, or fused heteroaryl ring as defined above including at least one site of saturation, in other words, at least one single bond. As used herein, the term "unsaturated" refers to an aryl, heteroaryl, or fused heteroaryl ring as defined above that does not include a site of saturation, in other words, a single bond.

The term "cycloalkyl" as used herein, unless otherwise stated, refers to a substituted or unsubstituted, saturated or partially unsaturated, monocyclic, bicyclic, or polycyclic hydrocarbon ring, including bridged cycloalkyl, fused cycloalkyl, and spirocycloalkyl. For example, it may include $C_{3-10}$ cycloalkyl or $C_{3-6}$ cycloalkyl, including but not limited to cyclopropyl, cyclobutyl, cyclopentyl, cyclohexyl, or cycloheptyl.

The term "cycloalkylene" as used herein refers to a radical (divalent radical) derived from a cycloalkene.

The term "heterocycle" or "heterocycloalkyl" as used herein, unless otherwise stated, refers to a substituted or unsubstituted, saturated or partially unsaturated, monocyclic, bicyclic, or polycyclic non-aromatic ring including one or more heteroatoms selected from N, O, and S, including bridged heterocycles, fused heterocycles, and spiroheterocycles. The above term includes monocyclic or polycyclic, cyclic alkyl, for example, $C_{4-15}$ heterocycloalkyl, may include but not limited to piperidinyl, piperazinyl, pyrrolidinyl, morpholinyl, thiomorpholinyl, tetrahydrofuranyl, tetrahydro-2H-pyranyl, imidazolidinyl, pyrrolidin-2-one, pyrrolizinyl, or pyrrolyl, etc. A heterocycle may be a carbon linker or a heteroatom linker. For example, a heterocycle linked to the base molecule through a nitrogen atom among the ring atoms of the heterocycle may be, but is not limited to, N-morpholinyl, N-piperidinyl, N-pyrrolidinyl, or N-pyrrolyl.

As used herein, the term "fused heterocycle" or "fused cycloalkyl," unless otherwise stated, refers to a substituted or unsubstituted ring system. Depending on the number of rings, they may be classified as bicyclic, tricyclic, tetracyclic, or more polycyclic fused cycloalkyls. Fused cycloalkyl refers to a polycyclic cycloalkyl in which each ring shares an adjacent pair of carbon atoms with another ring, and one or more of the rings may share one or more double bonds, but none of these rings has a fully conjugated $\pi$-electron system, and may include, for example, a $C_{3-20}$ fused cycloalkyl. For example, a bicyclic fused cycloalkyl is also referred to as a "bicycloalkyl" and may be a 5+6 bicycloalkyl or a 6+6 bicycloalkyl, depending on the number of atoms forming each of the two rings that are fused.

The term "fused heterocycloalkyl" as used herein, unless otherwise stated, refers to a substituted or unsubstituted fused cycloalkyl including one or more heteroatoms selected from N, O, and S, and may include, for example, a $C_{8-20}$ heterofused cycloalkyl. For example, a bicyclic fused heterocycloalkyl is also referred to as a "heterobicycloalkyl" and may be a 5+6 fused heterobicycloalkyl or a 6+6 fused heterobicycloalkyl, depending on the number of atoms forming each of the two rings being fused. Examples include, but are not limited to, hexahydro-1H-pyrrolizine.

The term "stereoisomer" as used herein may refer to a compound of the present disclosure or a salt thereof which has the same formula or molecular formula but is optically or sterically different. The term "enantiomer" as used herein refers to various stereoisomers and geometric isomers that may exist for the compounds according to the present

disclosure.

The compounds described herein may have an asymmetric center, a geometrical center (for example, a double bond), or both. Unless a particular stereochemical or isomeric form is specifically indicated, all chiral, diastereomeric, racemic, and geometrically isomeric forms of the structure are intended.

Compounds of Formula 1 according to an aspect of the present disclosure may have an asymmetric carbon center (asymmetric carbon), and therefore may exist as enantiomers (*R* or *S* isomers), racemates, diastereomers, or any mixtures thereof, and all of these isomers and mixtures are included in the scope of the present disclosure.

It is understood that the compounds described herein may have chiral centers, and unless otherwise stated, each chiral center may independently be the R-configuration or the S-configuration or a mixture thereof. Accordingly, the compounds described herein include optical isomers which are enriched or resolved at any or all of the asymmetric atoms. Racemic mixtures of R-enantiomers and S-enantiomers, and enantio-enriched stereoisomeric mixtures including the R- and S-enantiomers as well as individual optical isomers, may be isolated or synthesized substantially free of their enantiomeric or diastereomeric partners, and all such stereoisomers are within the scope of the present technology.

Compounds of the present disclosure including asymmetrically substituted atoms may be isolated in optically active or racemic forms. Method of preparing optically active forms, such as resolution of racemic forms, synthesis from optically active starting materials, or use of chiral auxiliaries, are well known in the art.

The term "atropisomer" as used herein refers to all stereoisomers that may be separated from each other. This is an isomer that appears when a single bond between carbons in a compound cannot rotate freely due to a bulky substituent. It refers to stereoisomers generated from an asymmetric axis, which may be formed from restricted rotation about a single bond where rotational barriers are sufficiently high to allow discrimination of isomeric species, including complete isolation of stable non-interconverting diastereoisomers or enantiomeric species.

Compounds according to an embodiment may form rotational atropisomers with a high probability.

Compounds according to an embodiment may also include "tautomeric" forms. The tautomeric form arises due to the exchange of a single bond with an adjacent double bond, along with the concomitant transfer of a proton. The tautomeric form includes prototropic tautomers, which are isomeric protonated states with the same empirical formula and total charge. The tautomeric form may be in equilibrium or stereochemically fixed to one form by appropriate substitution.

Compounds according to an embodiment may include "isotopic variants." The present disclosure also includes isotopically labeled compounds which are identical to the compounds described herein except that one or more atoms are replaced by an atom having an atomic mass or mass number different from the atomic mass or mass number generally found in nature ("isotope"). The compounds of the present disclosure may also contain unnatural proportions of atomic isotopes at one or more of the atoms constituting such compounds. Examples of isotopes that may be incorporated into the compounds described herein include isotopes of hydrogen, carbon, nitrogen, oxygen, phosphorus, fluorine and chlorine, for example $^{2}H$ ("D"), $^{3}H$, $^{13}C$, $^{14}C$, $^{15}N$, $^{18}O$, $^{17}O$, $^{31}P$, $^{32}P$, $^{35}S$, $^{18}F$ and $^{36}Cl$. For example, the compounds described herein may have one or more H atoms replaced with deuterium. In general, references or descriptions to a particular element, such as hydrogen or H, are intended to include all isotopes of that element. For example, if the R group is defined as including hydrogen or H, deuterium and tritium are also included. Deuterated starting materials are readily available and are applicable to the synthetic methods described herein to provide for the synthesis of deuterium including compounds. A large number of deuterium-including reagents and building blocks are commercially available from chemical suppliers such as Aldrich Chemical Co.

The term "solvate" as used herein may include a molecular complex including a compound of Formula 1 and one or more pharmaceutically acceptable solvent molecules, for example ethanol or water. A complex in which the solvent molecule is water is also referred to as a "hydrate."

The term "pharmaceutically acceptable salt" as used herein includes any salt that has low toxicity to humans and does not adversely affect the biological activity and physicochemical properties of the parent compound.

## KRAS Protein Inhibitor

Kirsten Rat Sarcoma Oncogene Homolog (KRAS) is a GTPase that integrates extracellular signals into intracellular proliferation and survival signals, and is one of the small GTPase family including Ras, Rho, Rab, Arf, and Ran. It receives signals from various receptor tyrosine kinases, particularly upstream signals from EGFR, and transmits signals downstream mainly to Raf and PI3K through the GTPase cycle of KRAS, thereby regulating various processes including cell proliferation.

In the GTPase cycle, KRAS binds to effector proteins in the activated state bound to GTP through the action of Guanine nucleotide exchange factors (GEF), and becomes the inactivated state bound to GDP through the action of GTPase-activating protein (GAP).

In addition to the KRAS GTP/GDP binding site, switch I/II involved in protein structural transition related to GTP/GDP binding was reported, demonstrating that it may inhibit the GTPase cycle.

The association of KRAS with cancer has been reported, and although various attempts have been made to

inhibit KRAS activity, the potential as a direct drug target has been considered low. However, the approval of Sotorasib and Adagrasib for non-small cell lung cancer has confirmed the possibility of drug development.

The compounds of the present disclosure relate to novel KRAS inhibitor compounds that inhibit KRAS, wherein the compounds include a mutation in any one of KRAS codons 12, 13, 61, for example, but not limited to, at least one or more of KRAS G12D, KRAS G12V, KRAS G12C, KRAS G13D and KRAS Q61H mutations.

Pharmaceutical Composition

An aspect of the present disclosure provides a pharmaceutical composition for treating cancer, which includes a compound of Formula 1, or a stereoisomer, a diastereomer, an enantiomer, an atropisomer, a solvate, an isotopic variant, a tautomer, or a pharmaceutically acceptable salt thereof as an active ingredient.

According to an embodiment, the composition may include a therapeutically effective amount of a compound of Formula 1, or a stereoisomer, a diastereomer, an enantiomer, an atropisomer, a solvate, an isotopic variant, a tautomer, or a pharmaceutically acceptable salt thereof.

According to an embodiment, the composition may further include another therapeutic agent in addition to a compound of Formula 1, or a stereoisomer, a diastereomer, an enantiomer, an atropisomer, a solvate, an isotopic variant, a tautomer, or a pharmaceutically acceptable salt thereof.

According to an embodiment, the composition may further include a pharmaceutically acceptable carrier or excipient.

According to an embodiment, the composition may include any one of a compound of Formula 1, or a stereoisomer, a diastereomer, an enantiomer, an atropisomer, a solvate, an isotopic variant, a tautomer, or a pharmaceutically acceptable salt thereof, or another therapeutic agent in a range of 0.005 % to 100 %, and the remainder may include a pharmaceutically acceptable carrier or excipient.

According to an embodiment, a compound of Formula 1, or a stereoisomer, a diastereomer, an enantiomer, a atropisomer, a solvate, an isotopic variant, a tautomer, or a pharmaceutically acceptable salt thereof, or a pharmaceutical composition including the same, may be used for the treatment of cancer.

According to an embodiment, a compound of Formula 1, or a stereoisomer, a diastereomer, an enantiomer, a atropisomer, a solvate, an isotopic variant, a tautomer, or a pharmaceutically acceptable salt thereof, or a pharmaceutical composition including the same, may exhibit KRAS protein inhibitory activity.

## Route of Administration and Dosage Form

The compounds and pharmaceutical compositions of the present disclosure may be provided by any suitable route of administration and dosage form acceptable in the pharmaceutical art.

## Method of Preparation

The compounds of the present disclosure may be synthesized using organic synthesis techniques known to a person of ordinary skill in the art. Some compounds and/or intermediates of the present disclosure may be commercially available, may be known in the literature, or may be prepared by a person of ordinary skill in the art by selecting an appropriate synthesis method from known organic synthesis techniques. Some compounds of the present disclosure may be synthesized using the reaction equation, examples, or intermediates described herein. A person of ordinary skill in the art may recognize that the reaction time, number equivalents of reagents, and/or temperature may be modified from the synthesis methods described herein, and that different work-up and/or purification techniques may be used.

The structure of the synthesized compound may be verified by methods known to a person of ordinary skill in the art, for example by nuclear magnetic resonance (NMR) spectroscopy and/or mass spectroscopy.

## General Information

Unless otherwise stated, reagents and solvents obtained from commercial suppliers were used without purification or drying. $^1$H NMR was recorded using Broker 400 MHz and 500 MHz, and TMS was used as an internal standard. LCMS analysis was performed on a Waters UPLC with an SQD-2 mass detector (Single quadruple), and HPLC analysis was performed on an Acquit UPLC H CLASS (WATERS).

The numerical values described herein are to be construed as including the meaning of "about" even if not specified. As used herein, the term "about" refers to within 5 % of a given value or range, preferably within 1 % to 2 %.

As used herein, numerical ranges expressed using the term "to" include ranges that include the numerical values described before and after the term "to" as the lower and upper limits, respectively.

All references, publications, issued patents, and patent applications cited within the text of the present

specification are incorporated herein by reference in their entirety for all purposes.

Hereinafter, the present disclosure will be described in more detail through the following synthesis reaction equation, examples, or test examples. However, these are only provided to aid understanding of the present disclosure, and are not intended to limit the scope of the present disclosure in any way.

**Synthesis Reaction Equation of Example 1**

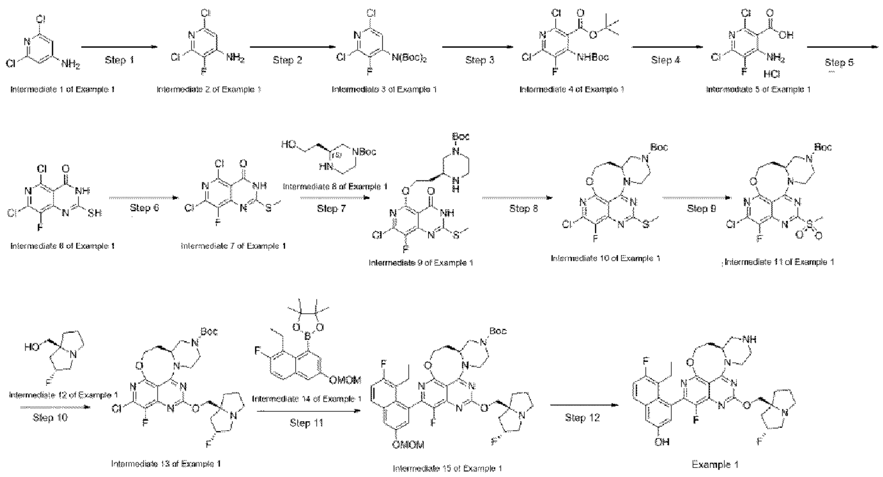


**Example 1: Preparation of 5-ethyl-6-fluoro-4-((S)-1-fluoro-12-(((2R,7aS)-2-fluorotetrahydro-1H-pyrrolizin-7a(5H)-yl)methoxy)-6,6a,7,8,9,10-hexahydro-5H-4-oxa-3,8,10a,11,13-pentaazabenzo[4,5]cycloocta[1,2,3-de]naphthalen-2-yl)naphthalen-2-ol**

Step 1) Intermediate 2 of Example 1: Preparation of 2,6-dichloro-3-fluoropyridin-4-amine

To a stirred solution of 2,6-dichloropyridin-4-amine (Intermediate 1 of Example 1) (25.0 g, 154 mmol) in a mixture of solvent MeOH and water (1:1, 500 mL, 20 vol), Selectfluoro (60.1 g, 169 mmol) was added at room temperature, and the reaction mixture was heated to 45 °C and stirred for 16 hours. The progress of the reaction was monitored by TLC. After completion of the reaction, the reaction mixture was concentrated under reduced pressure to obtain a residue, which was diluted with water and extracted with ethyl acetate. A separated organic layer was dried over anhydrous $Na_2SO_4$, filtered, and evaporated under reduced pressure to obtain a crude compound. The crude compound was purified by silica gel (100-200 mesh) column chromatography and eluted with 2 % ethyl acetate in petroleum ether (pet ether) to obtain 2,6-dichloro-3-fluoropyridin-4-amine (Intermediate 2 of Example 1) (20.0 g, 72 % yield) as a white solid. $^1$H NMR(400 MHz, $CDCl_3$) $\delta$ 4.52(brs, 2H), 6.62(d, $J$ = 5.2 Hz, 1H). MS(LCMS): 180.96 $m/z$ [M+H].

Step 2) Intermediate 3 of Example 1: Preparation of tert-butyl(tert-butoxycarbonyl)(2,6-dichloro-3-fluoropyridin-4-yl) carbamate

To a stirred solution of 2,6-dichloro-3-fluoropyridin-4-amine (Intermediate 2 of Example 1) (1.00 g, 2.63 mmol) in THF (10 mL, 10 vol), DMAP (0.06 g, 0.26 mmol) and Boc anhydride (1.10 mL, 5.26 mmol) were added at room temperature, and the reaction mixture was heated to 60 °C and maintained for 6 hours. The progress of the reaction was monitored by TLC. After completion of the reaction, the reaction mixture was diluted with water (50 mL), extracted with ethyl acetate, and a separated organic layer was dried over anhydrous $Na_2SO_4$, filtered, and concentrated under reduced pressure to obtain a crude compound. The crude compound was purified by silica gel (100-200 mesh) column chromatography and eluted with 1 % ethyl acetate in petroleum ether to obtain tert-butyl(tert-butoxycarbonyl)(2,6-dichloro-3-fluoropyridin-4-yl) carbamate (Intermediate 3 of Example 1) (0.50 g, 50 % yield). $^1$H NMR(400 MHz, $CDCl_3$) $\delta$ 1.47(s, 18H), 7.15(d, J = 4.40Hz, 1H).

Step 3) Intermediate 4 of Example 1: Preparation of tert-butyl 4-((tert-butoxycarbonyl)amino)-2,6-dichloro-5-fluoronicotinate

To a stirred solution of tert-butyl (tert-butoxycarbonyl)(2,6-dichloro-3-fluoropyridin-4-yl)carbamate (Intermediate 3 of Example 1) (28.0 g, 73.7 mmol) in THF (280 mL, 10 vol), LDA (1 M in THF) (147 mL, 147 mmol) was added at -78 °C and stirred for 30 minutes, followed by the addition of Boc anhydride (14.4 g, 66.3 mmol). The reaction mixture was stirred at -78 °C for 2 hours. The progress of the reaction was monitored by TLC. After completion of the reaction, the reaction mixture was diluted with water and extracted with ethyl acetate. A separated organic layer was dried over anhydrous $Na_2SO_4$, filtered, and evaporated under reduced pressure to obtain a crude compound. The crude compound was purified by silica gel (100-200 mesh) column chromatography and eluted with 5 % ethyl acetate in petroleum ether to obtain tert-butyl 4-((tert-butoxycarbonyl)amino)-2,6-dichloro-5-fluoronicotinate (Intermediate 4 of Example 1) (26.0 g, 92 % yield) as an grayish-white solid. $^1H$ NMR(400 MHz, $CDCl_3$) $\delta$ 1.50(s, 9H), 1.62(s, 9H), 7.21(brs, 1H). MS(LC-MS): 381.17 *m/z* [M+H].

Step 4) Intermediate 5 of Example 1: Preparation of 4-amino-2,6-dichloro-5-fluoronicotinic acid

To a stirred solution of tert-butyl 4-((tert-butoxycarbonyl)amino)-2,6-dichloro-5-fluoronicotinate (Intermediate 4 of Example 1) (26.0 g, 68.4 mmol) in 1,4-dioxane (130 mL, 5 vol) at 0 °C, concentrated HCl (130 mL, 5 vol) was added, and the reaction mixture was heated to room temperature and stirred for 16 hours. The progress of the reaction was monitored by TLC. After completion of the reaction, the reaction mixture was concentrated under reduced pressure, washed with DCM, and filtered to obtain a crude compound, 4-amino-2,6-dichloro-5-fluoronicotinic acid (Intermediate 5 of Example 1) (18.0 g, crude compound) as a white solid. The crude compound was used as is in the next step without further purification. $^1H$ NMR(400 MHz, $CDCl_3$) $\delta$ 5.71 (brs, 2H), 7.31(brs, 1H). MS(LC-MS): 224.82 *m/z* [M+H].

Step 5) Intermediate 6 of Example 1: Preparation of 5,7-dichloro-8-fluoro-2-mercaptopyrido[4,3-d]pyrimidin-4(3H)-one

A solution of 4-amino-2,6-dichloro-5-fluoronicotinic acid (Intermediate 5 of Example 1) (18.0 g, 80.3 mmol) in $SOCl_2$ (540 mL, 30 vol) was heated to 50 °C and stirred for 8 hours. The progress of the reaction was monitored by TLC. After completion of the reaction, the reaction mixture was concentrated under reduced pressure to obtain a residue, which was dissolved in acetone (20 mL), and $NH_4SCN$ (1.0 g in 20 mL acetone) was added dropwise at room temperature and stirred for 1 hour. After completion, the reaction mixture was diluted with water, filtered, washed with water, and dried under high vacuum to obtain a crude compound, 5,7-dichloro-8-fluoro-2-mercaptopyrido[4,3-d]pyrimidin-4(3H)-one (Intermediate 6 of Example 1) (10.0 g, 49 % yield) as a grayish-white solid. The above compound was used as is in the next step without further purification. $^1H$ NMR(400 MHz, $CDCl_3$) $\delta$ 13.31 (brs, 1H), 12.91 (s, 1H). MS(LC-MS): 265.94 *m/z* [M+H].

Step 6) Intermediate 7 of Example 1: Preparation of 5,7-dichloro-8-fluoro-2-(methylthio)pyrido[4,3-d]pyrimidin-4(3H)-one

To a stirred solution of 5,7-dichloro-8-fluoro-2-mercaptopyrido[4,3-d]pyrimidin-4(3H)-one (Intermediate 6 of Example 1) (10.0 g, 37.7 mmol) in MeOH (1500 mL, 150 vol), 0.1 M NaOH (1500 mL, 150 vol) and MeI (2.50 mL, 41.5 mmol) were added at room temperature, and the reaction mixture was stirred for 2 hours at room temperature. The progress of the reaction was monitored by TLC. After completion of the reaction, the reaction mixture was diluted with water (500 mL) and acidified with concentrated HCl to about pH 6 to obtain a solid, the solid was filtered and washed with water, then dried under high vacuum to obtain a crude compound, 5,7-dichloro-8-fluoro-2-(methylthio)pyrido[4,3-d]pyrimidin-4(3H)-one (Intermediate 7 of Example 1) (8.0 g, crude compound) as a light brown solid. The crude compound was used as is in the next step without further purification. $^1H$ NMR(400 MHz, $CDCl_3$) $\delta$ 2.60(s, 3H), 13.33(s, 1H). MS(LC-MS): 279.95 m/z [M+H].

Step 7) Intermediate 9 of Example 1: Preparation of tert-butyl (S)-3-(2-((7-chloro-8-fluoro-2-(methylthio)-4-oxo-3,4-dihydropyrido[4,3-d]pyrimidin-5-yl)oxy)ethyl)piperazine-1-carboxylate

To a stirred solution of 5,7-dichloro-8-fluoro-2-(methylthio)pyrido[4,3-d]pyrimidin-4(3H)-one (Intermediate 7 of Example 1) (8.00 g, 28.7 mmol) in THF (80 mL, 10 vol), NaH (4.90 g, 129 mmol) was added at 0 °C and stirred for 30 minutes, followed by the addition of tert-butyl (S)-3-(2-hydroxyethyl)piperazine-1-carboxylate (7.90 g, 34.4 mmol) at room temperature, and the reaction mixture was stirred at room temperature for 2 hours. The progress of the reaction was monitored by TLC. After completion of the reaction, the reaction mixture was diluted with cold ice water, extracted with ethyl acetate, and a separated organic layer was washed with a brine solution, dried over anhydrous $Na_2SO_4$, filtered and evaporated under reduced pressure to obtain a crude compound. The crude compound was purified by silica gel (100-200 mesh) column chromatography, eluted with 5 % methanol in DCM, to obtain tert-butyl (S)-3-(2-((7-chloro-8-fluoro-2-(methylthio)-4-oxo-3,4-dihydropyrido[4,3-d]pyrimidin-5-yl)oxy)ethyl)piperazine-1-carboxylate (Intermediate 9 of Example 1) (6.0 g, 44 % yield) as a brown solid. $^{1}H$ NMR(400 MHz, DMSO-$d_6$): δ 1.42(s, 9H), 2.06(brs, 2H), 2.42(s, 3H), 3.04(d, *J* = 10.1Hz, 3H), 3.46(brs, 2H), 4.02(brs, 2H), 4.27(brs, 1H), 4.52(d, *J* = 8.8, 1H), 9.45(brs, 1H). MS(LC-MS): 474.37 *m*/*z* [M+H].

Step 8) Intermediate 10 of Example 1: Preparation of tert-butyl (S)-2-chloro-1-fluoro-12-(methylthio)-5,6,6a,7,9,10-hexahydro-8H-4-oxa-3,8,10a,11,13-pentaazabenzo[4,5]cycloocta[1,2,3-de]naphthalen-8-carboxylate

To a stirred solution of tert-butyl (S)-3-(2-((7-chloro-8-fluoro-2-(methylthio)-4-oxo-3,4-dihydropyrido[4,3-d]pyrimidin-5-yl)oxy)ethyl)piperazine-1-carboxylate (Intermediate 9 of Example 1) (6.00 g, 12.7 mmol) in DCM (60 mL, 10 vol), DIPEA (32.9 mL, 190 mmol) was added at 0 °C and stirred for 10 minutes, then BOP-Cl (11.6 g, 45.6 mmol) was added at 0 °C. The reaction mixture was heated to room temperature and stirred for 5 hours. The progress of the reaction was monitored by TLC. After completion of the reaction, the reaction mixture was diluted with water, extracted with DCM, and a separated organic layer was washed with a brine solution, dried over anhydrous $Na_2SO_4$, and concentrated under reduced pressure to obtain a crude compound. The crude compound was purified by preparative HPLC to obtain tert-butyl (S)-2-chloro-1-fluoro-12-(methylthio)-5,6,6a,7,9,10-hexahydro-8H-4-oxa-3,8,10a,11,13-pentaazabenzo[4,5]cycloocta [1,2,3-de]naphthalen-8-carboxylate (Intermediate 10 of Example 1) (0.80 g, 14 % yield) as a brown solid. $^{1}H$ NMR(400 MHz, DMSO-$d_6$): δ 1.42(s, 9H), 1.97-2.10(m, 2H), 2.54(s, 3H), 3.07-3.22(m, 2H), 3.55(d, *J* = 5.60 Hz, 1H), 3.78(d, *J* = 12.8Hz, 1H), 3.86-3.91 (m, 1H), 3.97(d, *J* = 12.8 Hz, 1H), 4.22(t, *J* = 10.8Hz, 1H), 4.46(dd, *J* = 12.8, 4.8 Hz, 1H), 4.66-4.76(m, 1H). MS(LC-MS): 456.37 *m*/*z* [M+H].

Step 9) Intermediate 11 of Example 1: Preparation of tert-butyl (S)-2-chloro-1-fluoro-12-(methylsulfonyl)-5,6,6a,7,9,10-hexahydro-8H-4-oxa-3,8,10a,11,13-pentaazabenzo[4,5]cycloocta[1,2,3-de]naphthalen-8-carboxylate

To a stirred solution of tert-butyl (S)-2-chloro-1-fluoro-12-(methylthio)-5,6,6a,7,9,10-hexahydro-8H-4-oxa-3,8,10a,11,13-pentaazabenzo[4,5]cycloocta[1,2,3-de]naphthalen-8-carboxylate (Intermediate 10 of Example 1) (0.40 g, 0.88 mmol) in a mixture of solvent ACN and water (2:1, 48 mL), oxone (0.65 g, 1.05 mmol) was added at 0 °C, and the reaction mixture was stirred at room temperature for 20 minutes. The progress of the reaction was monitored by TLC. After completion of the reaction, the reaction mixture was concentrated under reduced pressure to obtain a crude compound, tert-butyl (S)-2-chloro-1-fluoro-12-(methylsulfonyl)-5,6,6a,7,9,10-hexahydro-8H-4-oxa-3,8,10a,11,13-pentaazabenzo[4,5]cycloocta[1,2,3-de]naphthalen-8-carboxylate (Intermediate 11 of Example 1) (0.34 g, crude compound) as a grayish-white solid. MS(LCMS): 488.32 *m*/*z* [M+H].

Step 10) Intermediate 13 of Example 1: Preparation of tert-butyl (S)-2-chloro-1-fluoro-12-(((2R,7aS)-2-fluorotetrahydro-1H-pyrrolizin-7a(5H)-yl)methoxy)-5,6,6a,7,9,10-hexahydro-8H-4-oxa-3,8,10a,11,13-pentaazabenzo[4,5]cycloocta [1,2,3-de]naphthalen-8-carboxylate

To a stirred solution of ((2R,7aS)-2-fluorotetrahydro-1H-pyrrolizin-7a(5H)-yl)methanol (0.01 g, 0.62 mmol) in THF (2.0 mL, 10 vol) at 0 °C, NaH (0.031 g, 0.82 mmol) was added, and the mixture was stirred for 30 minutes, followed by the addition of tert-butyl (S)-2-chloro-1-fluoro-12-(methylsulfonyl)-5,6,6a,7,9,10-hexahydro-8H-4-oxa-3,8,10a,11,13-pentaazabenzo[4,5]cycloocta[1,2,3-de]naphthalen-8-carboxylate (Intermediate 11 of Example 1) (0.20 g, 0.41 mmol) at 0 °C. The reaction mixture was heated to room temperature and stirred for 4 hours. The progress of the reaction was monitored by TLC. After completion of the reaction, the reaction mixture was diluted with water and extracted with ethyl acetate. A separated organic layer was washed with a brine solution, dried over anhydrous $Na_2SO_4$, filtered and evaporated under reduced pressure to obtain a crude compound. The crude compound was purified by silica gel (100-200 mesh) column chromatography, eluted with 2 % methanol in DCM, to obtain tert-butyl (S)-2-chloro-1-fluoro-12-(((2R,7aS)-2-fluorotetrahydro-1H-pyrrolizin-7a(5H)-yl)methoxy)-5,6,6a,7,9,10-hexahydro-8H-4-oxa-3,8,10a,11,13-pentaazabenzo[4,5]cycloocta[1,2,3-de]naphthalen-8-carboxylate (Intermediate 13 of Example 1) (0.02 g, 9 % yield) as a grayish-white solid. MS(LC-MS): 567.44 *m*/*z* [M+H].

Step 11) Intermediate 15 of Example 1: Preparation of tert-butyl (S)-2-(8-ethyl-7-fluoro-3-(methoxymethoxy)naphthalen-1-yl)-1-fluoro-12-(((2R,7aS)-2-fluorotetrahydro-1H-pyrrolizin-7a(5H)-yl)methoxy)-5,6,6a,7,9,10-hexahydro-8H-4-oxa-3,8,10a,11,13-pentaazabenzo[4,5]cycloocta[1,2,3-de]naphthalen-8-carboxylate

To a stirred solution of tert-butyl (S)-2-chloro-1-fluoro-12-(((2R,7aS)-2-fluorotetrahydro-1H-pyrrolizin-7a(5H)-yl) methoxy)-5,6,6a,7,9,1 0-hexahydro-8H-4-oxa-3,8,10a,11,13-pentaazabenzo[4,5]cycloocta[1,2,3-de]naphthalen-8-carboxylate (Intermediate 13 of Example 1) (0.20 g, 0.35 mmol) and 2-(8-ethyl-7-fluoro-3-(methoxymethoxy)naphthalen-1-yl)-4,4,5,5-tetramethyl-1,3,2-dioxaborolane (Intermediate 14 of Example 1) (0.19 g, 0.53 mmol) in a mixture of

solvent 1,4-dioxane and water (2.0 mL, 10 vol) $Cs_2CO_3$ (0.34 g, 1.06 mmol) was added at room temperature, and degassed under $N_2$ gas for 10 minutes. Pd(dppf)$Cl_2$·DCM (0.028 g, 0.03 mmol) was then added at room temperature, and the reaction mixture was heated to 80 °C and stirred for 16 hours. The progress of the reaction was monitored by TLC. After completion of the reaction, the reaction mixture was diluted with water and extracted with ethyl acetate. A separated organic layer was washed with a brine solution, dried over anhydrous $Na_2SO_4$, filtered and evaporated under reduced pressure to obtain a crude compound. The crude compound was purified by preparative HPLC, to obtain tert-butyl (S)-2-(8-ethyl-7-fluoro-3-(methoxymethoxy)naphthalen-1-yl)-1-fluoro-12-(((2R,7aS)-2-fluorotetrahydro-1H-pyrrolizin-7a(5H)-yl)methoxy)-5,6,6a,7,9,10-hexahydro-8H-4-oxa-3,8,10a,11,13-pentaazabenzo[4,5]cycloocta[1,2,3-de]naphthalen-8-carboxylate (Intermediate 15 of Example 1) (0.04 g, 15 % yield) as a light yellow solid. MS(LC-MS): 765.71 *m*/*z* [M+H].

Step 12) Example 1: Preparation of 5-ethyl-6-fluoro-4-((S)-1-fluoro-12-(((2R,7aS)-2-fluorotetrahydro-1H-pyrrolizin-7a(5H)-yl)methoxy)-6,6a,7,8,9,10-hexahydro-5H-4-oxa-3,8,10a,11,13-pentaazabenzo[4,5]cycloocta[1,2,3-de]naphthalen-2-yl)naphthalen-2-ol

To a stirred solution of tert-butyl (S)-2-(8-ethyl-7-fluoro-3-(methoxymethoxy)naphthalen-1-yl)-1-fluoro-12-(((2R,7aS)-2-fluorotetrahydro-1H-pyrrolizin-7a(5H)-yl)methoxy)-5,6,6a,7,9,10-hexahydro-8H-4-oxa-3,8,10a,11,13-pentaazabenzo[4,5]cycloocta[1,2,3-de]naphthalen-8-carboxylate (Intermediate 15 of Example 1) (0.04 g, 0.05 mmol) in DCM (1.2 mL, 30 vol), 4M HCl in 1,4-dioxane (0.80 mL) was added at 0°C, and the reaction mixture was heated to room temperature and stirred for 4 hours. The progress of the reaction was monitored by TLC. After completion of the reaction, the reaction mixture was concentrated under reduced pressure to obtain a crude compound. The crude compound was purified by preparative HPLC to obtain 5-ethyl-6-fluoro-4-((S)-1-fluoro-12-(((2R,7aS)-2-fluorotetrahydro-1H-pyrrolizin-7a(5H)-yl)methoxy)-6,6a,7,8,9,10-hexahydro-5H-4-oxa-3,8,10a,11,13-pentaazabenzo[4,5]cycloocta[1,2,3-de]naphthalen-2-yl)naphthalen-2-ol (Example 1) (0.006 g, 19 % yield) as a grayish-white solid.

$^1$H NMR(400 MHz, DMSO-$d_6$) δ 0.76(t, *J* = 7.2 Hz, 2H), 0.85(t, *J* = 7.2 Hz, 1H), 1.75-1.79(m, 2H), 1.79-1.81(m, 2H), 1.99(brs, 2H), 2.05(brs, 1H), 2.12-2.28(m, 2H), 2.61(d, *J* = 12.8Hz, 1H), 2.82-2.86(m, 2H), 2.93(brs , 1H), 3.02(s, 1H), 3.09(d, *J* = 4.12 Hz, 4H), 3.74-3.86(m, 1H), 3.98(d, *J* = 10.26 Hz, 1H), 4.02-4.20(m, 2H), 4.43-4.48(m, 1H), 5.03(t, *J* = 12.8 Hz, 1H), 5.21 & 5.35(brs, 1H), 6.91 & 7.11(d, *J* = 2.50 Hz, 1H), 7.28-7.30 (m, 1H), 7.31(d, *J* = 8.06 Hz, 1H), 7.74(dd, *J* = 8.88, 5.88 Hz, 1H), 9.92(brs, 1H). NH and -OH protons were not visible in $^1$H-NMR. MS(LC-MS): 621.40 *m*/*z* [M+H].

**Synthesis Reaction Equation of Example 2**

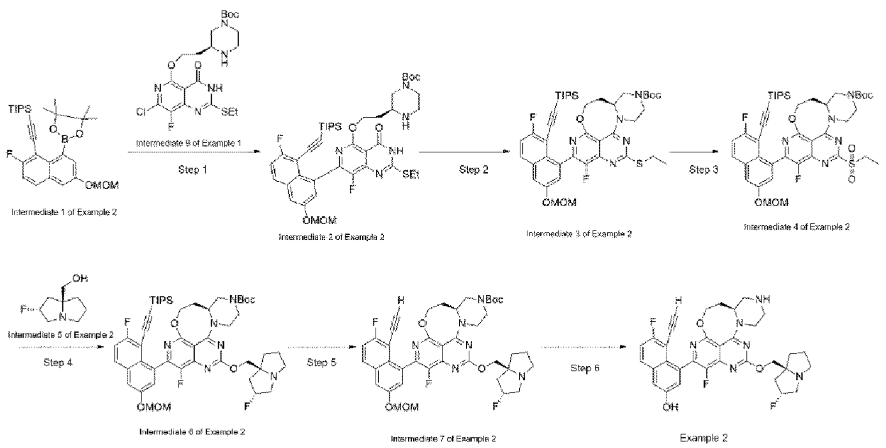


**Example 2: Preparation of 5-ethynyl-6-fluoro-4-((S)-1-fluoro-12-(((2R,7aS)-2-fluorotetrahydro-1H-pyrrolizin-7a(5H)-yl)methoxy)-6,6a,7,8,9,10-hexahydro-5H-4-oxa-3,8,10a,11,13-pentaazabenzo[4,5]cycloocta[1,2,3-de]naphthalen-2-yl)naphthalen-2-ol**

Step 1) Intermediate 2 of Example 2: Preparation of tert-butyl (S)-3-(2-((2-(ethylthio)-8-fluoro-7-(7-fluoro-3-(methoxymethoxy)-8-((triisopropylsilyl)ethynyl)naphthalen-1-yl)-4-oxo-3,4-dihydropyrido[4,3-d]pyrimidin-5-yl)oxy)ethyl)piperazine-1-carboxylate

To a stirred solution of tert-butyl (S)-3-(2-((7-chloro-2-(ethylthio)-8-fluoro-4-oxo-3,4-dihydropyrido[4,3-d]pyrimidin-5-yl)oxy)ethyl)piperazine-1-carboxylate (Intermediate 9 of Example 1) (0.10 g, 0.20 mmol) and ((2-fluoro-6-(methoxymethoxy)-8-(4,4,5,5-tetramethyl-1,3,2-dioxaborolan-2-yl)naphthalen-1-yl)ethynyl)triisopropylsilane (Intermediate 1 of Example 2) (0.13 g, 0.25 mmol) in a mixture of solvent 1,4-dioxane and water (4:1, 2.5 mL), $K_3PO_4$ (0.13 g, 0.62 mmol) was added at room temperature, and was degassed under $N_2$ gas for 10 minutes. Ruphos-Pd-G3 (0.02 g, 0.02 mmol) was then added at room temperature, and the reaction mixture was heated to 90 °C and stirred for 3 hours. The progress of the reaction was monitored by TLC. After completion of the reaction, the reaction mixture was diluted with water and extracted with ethyl acetate (2 times), and the combined organic layers were washed with a brine solution, dried over anhydrous $Na_2SO_4$, filtered, and evaporated under reduced pressure to obtain a crude compound. The crude compound was purified by silica gel (100-200 mesh) column chromatography and eluted with 5 % methanol in DCM to obtain tert-butyl (S)-3-(2-((2-(ethylthio)-8-fluoro-7-(7-fluoro-3-(methoxymethoxy)-8-((triisopropylsilyl)ethynyl)naphthalen-1-yl)-4-oxo-3,4-dihydropyrido[4,3-d]pyrimidin-5-yl)oxy)ethyl)piperazine-1-carboxylate (Intermediate 2 of Example 2) (0.35 g, total yield from 4 batches (4x100 mg), crude compound) as a light yellow solid. MS(LC-MS): 838.54 *m/z* [M+H].

Step 2) Intermediate 3 of Example 2: Preparation of tert-butyl (S)-12-(ethylthio)-1-fluoro-2-(7-fluoro-3-(methoxymethoxy)-8-((triisopropylsilyl)ethynyl)naphthalen-1-yl)-5,6,6a,7,9,10-hexahydro-8H-4-oxa-3,8,10a,11,13-pentaazabenzo[4,5]cycloocta[1,2,3-delnaphthalen-8-carboxylate

To a stirred solution of tert-butyl (S)-3-(2-((2-(ethylthio)-8-fluoro-7-(7-fluoro-3-(methoxymethoxy)-8-((triisopropylsilyl)ethynyl)naphthalen-1-yl)-4-oxo-3,4-dihydropyrido[4,3-d]pyrimidin-5-yl)oxy)ethyl)piperazine-1-carboxylate (Intermediate 2 of Example 2) (0.15 g, 0.28 mmol) in DCM (3.0 mL, 20 vol), BOP-Cl (0.254 g, 1.00 mmol) and DIPEA (0.70 mL, 4.04 mmol) were added at room temperature, and the reaction mixture was heated to 50 °C and stirred for 5 hours. The progress of the reaction was monitored by TLC. After completion of the reaction, the reaction mixture was concentrated under reduced pressure to obtain a crude compound. The crude compound was purified by silica gel (100-200 mesh) column chromatography and eluted with 5 % MeOH in DCM to obtain tert-butyl (S)-12-(ethylthio)-1-fluoro-2-(7-fluoro-3-(methoxymethoxy)-8-((triisopropylsilyl)ethynyl)naphthalen-1-yl)-5,6,6a,7,9,10-hexahydro-8H-4-oxa-3,8,10a,11,13-pentaazabenzo[4,5]cycloocta[1,2,3-de]naphthalen-8-carboxylate (Intermediate 3 of Example 2) (0.13 g, total yield from 2 batches, 57 % yield) as a brown solid. MS(LCMS): 820.49 *m/z* [M+H].

Step 3) Intermediate 4 of Example 2: Preparation of tert-butyl (S)-12-(ethylsulfonyl)-1-fluoro-2-(7-fluoro-3-(methoxymethoxy)-8-((triisopropylsilyl)ethynyl)naphthalen-1-yl)-5,6,6a,7,9,10-hexahydro-8H-4-oxa-3,8,10a,11,13-pentaazabenzo[4,5]cycloocta[1,2,3-de]naphthalen-8-carboxylate

To a stirred solution of tert-butyl (S)-12-(ethylthio)-1-fluoro-2-(7-fluoro-3-(methoxymethoxy)-8-((triisopropylsilyl)ethynyl)naphthalen-1-yl)-5,6,6a,7,9,10-hexahydro-8H-4-oxa-3,8,10a,11,13-pentaazabenzo[4,5]cycloocta[1,2,3-de]naphthalen-8-carboxylate (Intermediate 3 of Example 2) (0.13 g, 0.16 mmol) in a mixture of solvent ACN and water (2:1, 15.6 mL), oxone (0.24 g, 0.79 mmol) was added at room temperature, and the reaction mixture was stirred for 1 hour at room temperature. The progress of the 2-hour reaction was monitored by TLC. After completion of the reaction, the reaction mixture was diluted with water (10 mL) and ethyl acetate (2 x 20 mL), and the combined organic layers were washed with a brine solution, dried over anhydrous $Na_2SO_4$, and concentrated under reduced pressure to obtain a crude compound, tert-butyl (S)-12-(ethylsulfonyl)-1-fluoro-2-(7-fluoro-3-(methoxymethoxy)-8-((triisopropylsilyl)ethynyl)naphthalen-1-yl)-5,6,6a,7,9,10-hexahydro-8H-4-oxa-3,8,10a,11,13-pentaazabenzo[4,5]cycloocta[1,2,3-de]naphthalen-8-carboxylate (Intermediate 4 of Example 2) (0.135 g, crude compound) as a pale yellow solid. The crude compound was used as is in the next step without further purification. MS(LC-MS): 852.61 *m/z* [M+H].

Step 4) Intermediate 6 of Example 2: Preparation of tert-butyl (S)-1-fluoro-2-(7-fluoro-3-(methoxymethoxy)-8-((triisopropylsilyl)ethynyl)naphthalen-1-yl)-12-(((2R,7aS)-2-fluorotetrahydro-1H-pyrrolizin-7a(5H)-yl)methoxy)-5,6,6a,7,9,10-hexahydro-8H-4-oxa-3,8,10a,11,13-pentaazabenzo[4,5]cycloocta[1,2,3-de]naphthalen-8-carboxylate

To a stirred solution of ((2R,7aS)-2-fluorotetrahydro-1H-pyrrolizin-7a(5H)-yl)methanol (Intermediate 5 of Example 2) (0.031 g, 0.20 mmol) in THF (2.0 mL, 15 vol), $NaO^tBu$ (0.025 g, 0.26 mmol) was added at 0 °C and stirred for 10 minutes, followed by the addition of tert-butyl (S)-12-(ethylsulfonyl)-1-fluoro-2-(7-fluoro-3-(methoxymethoxy)-8-((triisopropylsilyl)ethynyl)naphthalen-1-yl)-5,6,6a,7,9,10-hexahydro-8H-4-oxa-3,8,10a,11,13-pentaazabenzo[4,5]cycloocta[1,2,3-de]naphthalen-8-carboxylate (Intermediate 4 of Example 2) (0.135 g, 0.16 mmol) at 0 °C. The reaction mixture was

stirred at 0 °C for 20 minutes. The progress of the reaction was monitored by TLC. After completion of the reaction, the reaction mixture was concentrated under reduced pressure to obtain a residue, the residue was diluted with water and extracted with ethyl acetate (twice) and the combined organic layers were washed with a brine solution, dried over anhydrous $Na_2SO_4$, filtered, and evaporated. Under reduced pressure, a crude compound tert-butyl (S)-1-fluoro-2-(7-fluoro-3-(methoxymethoxy)-8-((triisopropylsilyl)ethynyl)naphthalen-1-yl)-12-(((2R,7aS)-2-fluorotetrahydro-1H-pyrrolizin-7a(5H)-yl)methoxy)-5,6,6a,7,9,10-hexahydro-8H-4-oxa-3,8,10a,11,13-pentaazabenzo[4,5]cycloocta[1,2,3-de]naphthalen-8-carboxylate (Intermediate 6 of Example 2) (0.135 g, crude compound) was obtained as a pale yellow solid. The crude compound was used as is in the next step without further purification. MS(LC-MS): 917.64 *m*/*z* [M+H].

Step 5) Intermediate 7 of Example 2: Preparation of tert-butyl (S)-2-(8-ethynyl-7-fluoro-3-(methoxymethoxy)naphthalen-1-yl)-1-fluoro-12-(((2R,7aS)-2-fluorotetrahydro-1H-pyrrolizin-7a(5H)-yl)methoxy)-5,6,6a,7,9,10-hexahydro-8H-4-oxa-3,8,10a,11,13-pentaazabenzo[4,5]cycloocta[1,2,3-de]naphthalen-8-carboxylate

To a stirred solution of tert-butyl (S)-1-fluoro-2-(7-fluoro-3-(methoxymethoxy)-8-((triisopropylsilyl)ethynyl)naphthalen-1-yl)-12-(((2R,7aS)-2-fluorotetrahydro-1H-pyrrolizin-7a(5H)-yl)methoxy)-5,6,6a,7,9,10-hexahydro-8H-4-oxa-3,8,10a,11,13-pentaazabenzo[4,5]cycloocta[1,2,3-de]naphthalen-8-carboxylate (Intermediate 6 of Example 2) (0.135 g, 0.15 mmol) in THF (3.4 mL, 25 vol), TBAF (2.0 mL, 15 vol) was added at 0 °C, and the reaction mixture was stirred at 0 °C for 1 hour. The progress of the reaction was monitored by TLC. After completion of the reaction, the reaction mixture was quenched with an aqueous $NaHCO_3$ solution (5 mL), extracted with ethyl acetate (twice), and the combined organic layers were washed with a brine solution, dried over anhydrous $Na_2SO_4$, filtered, and evaporated under reduced pressure to obtain a crude compound. The crude compound was purified by preparative HPLC to obtain tert-butyl (S)-2-(8-ethynyl-7-fluoro-3-(methoxymethoxy)naphthalen-1-yl)-1-fluoro-12-(((2R,7aS)-2-fluorotetrahydro-1H-pyrrolizin-7a(5H)-yl)methoxy)-5,6,6a,7,9,10-hexahydro-8H-4-oxa-3,8,10a,11,13-pentaazabenzo[4,5]cycloocta[1,2,3-de]naphthalen-8-carboxylate (Intermediate 7 of Example 2) (0.03 g, 26 % yield) as a grayish-white solid. MS(LC-MS): 761.55 *m*/*z* [M+H].

Step 6) Example 2: Preparation of 5-ethynyl-6-fluoro-4-((S)-1-fluoro-12-(((2R,7aS)-2-fluorotetrahydro-1H-pyrrolizin-7a(5H)-yl)methoxy)-6,6a,7,8,9,10-hexahydro-5H-4-oxa-3,8,10a,11,13-pentaazabenzo[4,5]cycloocta[1,2,3-de]naphthalen-2-yl)naphthalen-2-ol

To a stirred solution of tert-butyl (S)-2-(8-ethynyl-7-fluoro-3-(methoxymethoxy)naphthalen-1-yl)-1-fluoro-12-(((2R,7aS)-2-fluorotetrahydro-1H-pyrrolizin-7a(5H)-yl)methoxy)-5,6,6a,7,9,10-hexahydro-8H-4-oxa-3,8,10a,11,13-pentaazabenzo[4,5]cycloocta[1,2,3-de]naphthalen-8-carboxylate (Intermediate 7 of Example 2) (0.12 g, 0.16 mmol) in 1,4-dioxane (1.2 mL), 4M HCl was added at 0 °C. The reaction mixture was stirred at 0 °C for 30 minutes. The progress of the reaction was monitored by TLC. After completion of the reaction, the reaction mixture was concentrated under reduced pressure to obtain a crude compound. The crude compound was purified by preparative HPLC to obtain 5-ethynyl-6-fluoro-4-((S)-1-fluoro-12-(((2R,7aS)-2-fluorotetrahydro-1H-pyrrolizin-7a(5H)-yl)methoxy)-6,6a,7,8,9,10-hexahydro-5H-4-oxa-3,8,10a,11,13-pentaazabenzo[4,5]cycloocta[1,2,3-de]naphthalen-2-yl)naphthalen-2-ol (Example 2) (0.0061 g, 6 % yield) as a grayish-white solid.

$^1$H NMR(401 MHz, DMSO-$d_6$) $\delta$ 1.72-2.23(m, 8H), 2.58-2.63(m, 1H), 2.82-2.87(m, 2H), 2.98-3.16(m, 6H), 3.73(dd, *J* = 10.88, 1.38 Hz, 1H), 3.94-3.98(m, 1H), 4.06-4.17(m, 3H), 4.42(dd, *J* = 12.51, 5.50 Hz, 1H), 5.03(d, *J* = 12.51 Hz, 1H), 5.21-5.34(m, 1H), 7.04-7.24(m, 1H), 7.35-7.36(m, 1H), 7.42-7.48(m, 1H), 7.95(dd, *J* = 9.13, 5.88 Hz, 1H), no exchangeable protons were observed in $^1$H NMR. MS(LC-MS): 617.31 *m*/*z* [M+H].

**Synthesis Reaction Equation of Example 3**

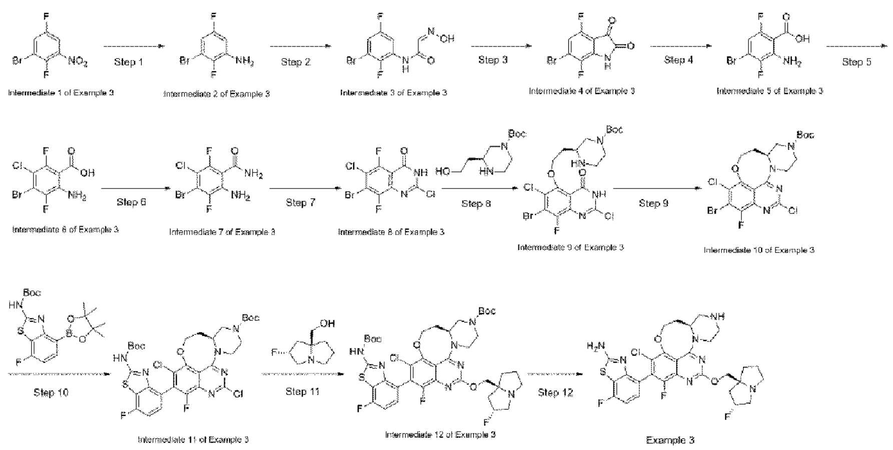


**Example 3: Preparation of 4-((14aS)-11-chloro-9-fluoro-7-(((2R,7aS)-2-fluorotetrahydro-1H-pyrrolizin-7a(5H)-yl) methoxy)-1,3,4,13,14,14a-hexahydro-2H-pyrazino[1',2':5,6][1,5]oxazocino[4,3,2-de]quinazolin-10-yl)-7-fluorobenzo[d]thiazol-2-amine**

Step 1) Intermediate 2 of Example 3: Preparation of 3-bromo-2,5-difluoroaniline

To a stirred solution of 1-bromo-2,5-difluoro-3-nitrobenzene (Intermediate 1 of Example 3) (10.0 g, 42.0 mmol) in a mixture of solvent EtOH and $H_2O$ (4:1, 250 mL), Fe (7.03 g, 126 mmol) and $NH_4Cl$ (13.5 g, 252 mmol) were added at room temperature, and the reaction mixture was heated to 80 °C and stirred for 6 hours. The progress of the reaction was monitored by TLC. After completion of the reaction, the reaction mixture was filtered through a Celite pad using ethyl acetate (100 mL) as a washing solvent to obtain a filtrate, and the filtrate was washed with water (50 mL), dried with anhydrous $Na_2SO_4$, filtered, and concentrated under reduced pressure to obtain a crude compound. The crude compound was purified by silica gel (100-200 mesh) column chromatography and eluted with 10 % ethyl acetate in petroleum ether to obtain 3-bromo-2,5-difluoroaniline (Intermediate 2 of Example 3) (10.0 g, crude compound) as a pale yellow viscous liquid. $^1$H NMR(400 MHz, $CDCl_3$) δ 3.93(brs, 2H), 6.40-6.45(m, 1H), 6.59-6.64(m, 1H). MS(LC-MS): 208.02 *m/z* [M+H].

Step 2) Intermediate 3 of Example 3: Preparation of (Z)-N-(3-bromo-2,5-difluorophenyl)-2-(hydroxyimino)acetamide

To a stirred solution of 3-bromo-2,5-difluoroaniline (Intermediate 2 of Example 3) (5.00 g, 24.1 mmol) in $H_2O$ (100 mL, 20 vol), $NH_2OH \cdot HCl$ (5.02 g, 72.5 mmol), $Na_2SO_4$ (27.4 g, 217 mmol), and 1,2,2-trichloroethan-1,1-diol (5.99 g, 36.2 mmol) in EtOH (14.0 mL) were added, followed by the addition of concentrated HCl (3.5 mL) at 0 °C, and the reaction mixture was heated to 60 °C and stirred for 16 hours. The progress of the reaction was monitored by TLC. After completion of the reaction, the reaction mixture was cooled to room temperature to form a solid, and the solid was filtered using water (50 mL) as a washing solvent, then dried under high vacuum to obtain a crude compound, (Z)-N-(3-bromo-2,5-difluorophenyl)-2-(hydroxyimino)acetamide (Intermediate 3 of Example 3) (10.0 g, total yield from 2 batches (2 x 5 g), crude compound) as a brown solid. The crude compound was used as is in the next step without further purification. $^1$H NMR(401MHz, DMSO-$d_6$) δ 7.50-7.54(m, 1H), 7.78(s, 1H), 7.82-7.87(m, 1H), 10.11(s, 1H), 12.45(s, 1H). MS(LC-MS): 279.02 *m/z* [M+H].

Step 3) Intermediate 4 of Example 3: Preparation of 6-bromo-4,7-difluoroindoline-2,3-dione

To $H_2SO_4$ (50 mL, 10 vol), (Z)-N-(3-bromo-2,5-difluorophenyl)-2-(hydroxyimino)acetamide (Intermediate 3 of Example 3) (5.00 g, 17.9 mmol) was added at 60 °C, and the reaction mixture was stirred at 90 °C for 2 hours. The progress of the reaction was monitored by TLC. After completion of the reaction, the reaction mixture was diluted with ice-cold water (50 mL) to obtain a solid, and the solid was filtered and dried in vacuum to obtain a crude compound, 6-bromo-4,7-difluoroindoline-2,3-dione (Intermediate 4 of Example 3) (4.0 g, crude compound) as a brown solid. The crude compound was used as is in the next step without further purification. MS(LC-MS): 259.88 *m/z* [MH].

Step 4) Intermediate 5 of Example 3: Preparation of 2-amino-4-bromo-3,6-difluorobenzoic acid

To a stirred solution of 6-bromo-4,7-difluoroindoline-2,3-dione (Intermediate 4 of Example 3) (4.00 g, 15.3 mmol) in 2M NaOH solution (84 mL, 11 vol), 30 % $H_2O_2$(9.53 mL) was added at 0 °C, and the reaction mixture was heated to room

temperature and stirred for 16 hours. The progress of the reaction was monitored by TLC. After completion of the reaction, the reaction mixture was acidified to about pH 3 with concentrated HCl at 0 °C to obtain a solid, and the solid was filtered and dried under high vacuum to obtain a crude compound, 2-amino-4-bromo-3,6-difluorobenzoic acid (Intermediate 5 of Example 3) (4.0 g, crude compound, total yield of 2 batches (2 x 4 g)) as a brown solid. The crude compound was used as is in the next step without further purification. $^1$H NMR(400 MHz, DMSO-$d_6$) δ 6.72(dd, *J* = 10.76, 5.25 Hz, 1H), 6.77-7.00(m, 2H), 13.42(brs, 1H). MS(LCMS): 252.06 *m*/*z* [M+H].

Step 5) Intermediate 6 of Example 3: Preparation of 2-amino-4-bromo-5-chloro-3,6-difluorobenzoic acid

To a stirred solution of 2-amino-4-bromo-3,6-difluorobenzoic acid (Intermediate 5 of Example 3) (3.00 g, 11.9 mmol) in DMF (40 mL, 13 vol), NCS (2.48 g, 14.3 mmol) was added at room temperature, and the reaction mixture was heated to 90 °C and stirred for 2 hours. The progress of the reaction was monitored by TLC. After completion of the reaction, the reaction mixture was diluted with water (100 mL) to obtain a solid, and the solid was filtered and dried under high vacuum to obtain a crude compound, 2-amino-4-bromo-5-chloro-3,6-difluorobenzoic acid (Intermediate 6 of Example 3) (3.0 g, crude compound) as a brown solid. The crude compound was used as is in the next step without further purification. MS(LCMS): 286.09 *m*/*z* [M+H].

Step 6) Intermediate 7 of Example 3: Preparation of 2-amino-4-bromo-5-chloro-3,6-difluorobenzamide

To a stirred solution of 2-amino-4-bromo-5-chloro-3,6-difluorobenzoic acid (Intermediate 6 of Example 3) (1.50 g, 5.26 mmol) in DMF (15 mL, 10 vol), $NH_4Cl$ (0.56 g, 10.5 mmol), HATU (2.20 g, 5.79 mmol), and DIPEA (2.03 g, 15.8 mmol) were added at 0 °C, and the reaction mixture was left to stand. Heated to room temperature and stirred for 2 hours. The progress of the reaction was monitored by TLC. After completion of the reaction, the reaction mixture was diluted with water (20 mL) to obtain a solid, and the solid was filtered and dried under high vacuum to obtain a crude compound, 2-amino-4-bromo-5-chloro-3,6-difluorobenzamide (Intermediate 7 of Example 3) (0.80 g, crude compound) as a brown solid. The crude compound was used as is in the next step without further purification. MS(LCMS): 285.08 *m*/*z* [M+H].

Step 7) Intermediate 8 of Example 3: Preparation of 7-bromo-2,6-dichloro-5,8-difluoroquinazolin-4(3H)-one

To a stirred solution of 2-amino-4-bromo-5-chloro-3,6-difluorobenzamide (Intermediate 7 of Example 3) (0.80 g, 2.82 mmol) in 1,4-dioxane (8.0 mL, 10 vol), thiophosgene (0.96 g, 8.45 mmol) was added at 0 °C, and the reaction mixture was stirred at room temperature for 1 hour. The reaction mixture was heated to 105 °C and stirred for 2 hours. The progress of the reaction was monitored by TLC. After completion of the reaction, the reaction mixture was concentrated under reduced pressure to obtain a residue, and the residue was washed with 20 % ethyl acetate in petroleum ether to obtain a solid, and the solid was filtered and dried under high vacuum to obtain a crude compound, 7-bromo-2,6-dichloro-5,8-difluoroquinazolin-4(3H)-one (Intermediate 8 of Example 3) (0.70 g, crude compound) as a pale yellow solid. The crude compound was used as is in the next step without further purification. MS(LC-MS): 329.02 *m*/*z* [M+H].

Step 8) Intermediate 9 of Example 3: Preparation of tert-butyl (S)-3-(2-((7-bromo-2,6-dichloro-8-fluoro-4-oxo-3,4-dihydroquinazolin-5-yl)oxy)ethyl)piperazine-1-carboxylate

To a stirred solution of tert-butyl (S)-3-(2-hydroxyethyl)piperazine-1-carboxylate (1.19 g, 3.65 mmol) in THF (10 mL, 14 vol), NaH (0.58 g, 24.3 mmol) was added at 0 °C, and the mixture was stirred at 0 °C for 30 minutes. To this, a solution of 7-bromo-2,6-dichloro-5,8-difluoroquinazolin-4(3H)-one (Intermediate 8 of Example 3) (0.70 g, 3.04 mmol) in THF (7.0 mL, 10 vol) was added at 0 °C, and the reaction mixture was heated to room temperature and stirred for 3 hours. The progress of the reaction was monitored by TLC. After completion of the reaction, the reaction mixture was diluted with saturated $NH_4Cl$ solution (20 mL) and extracted with ethyl acetate (2 × 20 mL) and the combined organic layers were dried over anhydrous $Na_2SO_4$, filtered, and concentrated under reduced pressure to obtain a crude compound, tert-butyl (S)-3-(2-((7-bromo-2,6-dichloro-8-fluoro-4-oxo-3,4-dihydroquinazolin-5-yl)oxy)ethyl)piperazine-1-carboxylate (Intermediate 9 of Example 3) (0.45 g, crude compound), as a brown solid. The crude compound was used as is in the next step without further purification. $^1$H NMR(400 MHz, DMSO-$d_6$) δ 1.16-1.23(m, 2H), 1.37-1.43(m, 9H), 1.99-2.10(m, 2H), 2.81-3.13(m, 3H), 3.96-4.13 (m, 4H), no exchangeable protons were observed in $^1$H NMR. MS(LC-MS): 539.16 *m*/*z* [M+H].

Step 9) Intermediate 10 of Example 3: Preparation of tert-butyl (S)-10-bromo-7,11-dichloro-9-fluoro-1,3,4,13,14,14a-hexahydro-2H-pyrazino[1',2':5,6][1,5]oxazocino[4,3,2-de]quinazolin-2-carboxylate

To a stirred solution of tert-butyl (S)-3-(2-((7-bromo-2,6-dichloro-8-fluoro-4-oxo-3,4-dihydroquinazolin-5-yl)oxy)ethyl)piperazine-1-carboxylate (Intermediate 9 of Example 3) (0.45 g, 0.77 mmol) in DCM (5 mL, 11 vol), BOP-Cl (0.68 g, 2.69 mmol) and DIPEA (1.48 g, 11.5 mmol) were added at 0 °C and the reaction mixture was heated to room temperature and stirred for 16 hours. The progress of the reaction was monitored by TLC. After completion of the reaction, the reaction mixture was diluted with DCM (15 mL) and washed with water (10 mL) and the separated organic layer was dried over anhydrous $Na_2SO_4$, filtered, and concentrated under reduced pressure to obtain a crude compound. The crude compound was purified by silica gel (100-200 mesh) and eluted with 40 % ethyl acetate in petroleum ether to obtain tert-butyl (S)-10-bromo-7,11-dichloro-9-fluoro-1,3,4,13,14,14a-hexahydro-2H-pyrazino[1',2':5,6][1,5]oxazocino[4,3,2-de]quinazolin-2-carboxylate (Intermediate 10 of Example 3) (0.15 g, crude compound) as a brown solid. MS(LC-MS): 521.23 *m/z* [M+H].

Step 10) Intermediate 11 of Example 3: Preparation of tert-butyl (14aS)-10-(2-((tert-butoxycarbonyl)amino)-7-fluorobenzo[d]thiazol-4-yl)-7,11-dichloro-9-fluoro-1,3,4,13,14,14a-hexahydro-2H-pyrazino[1',2':5,6][1,5]oxazocino[4,3,2-de]quinazolin-2-carboxylate

To a stirred solution of tert-butyl (S)-10-bromo-7,11-dichloro-9-fluoro-1,3,4,13,14,14a-hexahydro-2H-pyrazino[1',2':5,6][1,5]oxazocino[4,3,2-de]quinazolin-2-carboxylate (Intermediate 10 of Example 3) (0.15 g, 0.28 mmol) and tert-butyl (7-fluoro-4-(4,4,5,5-tetramethyl-1,3,2-dioxaborolan-2-yl)benzo[d]thiazol-2-yl)carbamate (0.095 g, 0.31 mmol) in a mixture of solvent 1,4-dioxane and water (2:1, 3.0 mL), $Cs_2CO_3$ (0.27 g, 0.84 mmol) was added at room temperature and degassed for 5 minutes. To this, dichloro[bis(diphenylphosphinophenyl)ether]palladium(II) (0.02 g, 0.03 mmol) was added at room temperature, and the reaction mixture was heated to 80 °C and stirred for 4 hours. The progress of the reaction was monitored by TLC. After completion of the reaction, the reaction mixture was diluted with ice-cold water (10 mL) and extracted with ethyl acetate (2 x 15 mL) and the combined organic layers were dried over anhydrous $Na_2SO_4$, filtered, and concentrated under reduced pressure to obtain a crude compound. The crude compound was purified by silica gel (100-200 mesh) and eluted with 40 % ethyl acetate in petroleum ether to obtain tert-butyl (14aS)-10-(2-((tert-butoxycarbonyl)amino)-7-fluorobenzo[d]thiazol-4-yl)-7,11-dichloro-9-fluoro-1,3,4,13,14,14a-hexahydro-2H-pyrazino[1',2':5,6][1,5]oxazocino[4,3,2-de]quinazolin-2-carboxylate (Intermediate 11 of Example 3) (0.07 g, 35 % yield) as a pale yellow viscous solid. MS(LCMS): 709.35 *m/z* [M+H].

Step 11) Preparation of Intermediate 12 of Example 3: tert-butyl (14aS)-10-(2-((tert-butoxycarbonyl)amino)-7-fluorobenzo[d]thiazol-4-yl)-11-chloro-9-fluoro-7-(((2R,7aS)-2-fluorotetrahydro-1H-pyrrolizin-7a(5H)-yl)methoxy)-1,3,4,13,14,14a-hexahydro-2H-pyrazino[1',2':5,6][1,5]oxazocino[4,3,2-de]quinazolin-2-carboxylate

To a stirred solution of ((2R,7aS)-2-fluorotetrahydro-1H-pyrrolizin-7a(5H)-yl)methanol (0.04 g, 0.25 mmol) in DMF (1.0 mL, 6 vol), NaH (0.008 g, 0.32 mmol) was added at 0 °C, and the mixture was stirred for 1 hour. To this, tert-butyl (14aS)-10-(2-((tert-butoxycarbonyl)amino)-7-fluorobenzo[d]thiazol-4-yl)-7,11-dichloro-9-fluoro-1,3,4,13,14,14a-hexahydro-2H-pyrazino[1',2':5,6][1,5]oxazocino[4,3,2-de]quinazolin-2-carboxylate (Intermediate 11 of Example 3) (0.15 g, 0.21 mmol) was added at 0 °C, and the reaction mixture was heated to room temperature and stirred for 3 hours. The progress of the reaction was monitored by TLC. After completion of the reaction, the reaction mixture was diluted with saturated $NH_4Cl$ solution (10 mL) and extracted with ethyl acetate (2 x 15 mL) and the combined organic layers were dried over anhydrous $Na_2SO_4$, filtered, and concentrated under reduced pressure to obtain a crude compound. The crude compound was purified by silica gel (100-200 mesh) and eluted with 5 % methanol in DCM to obtain tert-butyl (14aS)-10-(2-((tert-butoxycarbonyl)amino)-7-fluorobenzo[d]thiazol-4-yl)-11-chloro-9-fluoro-7-(((2R,7aS)-2-fluorotetrahydro-1H-pyrrolizin-7a(5H)-yl)methoxy)-1,3,4,13,14,14a-hexahydro-2H-pyrazino[1',2':5,6][1,5]oxazocino[4,3,2-de]quinazolin-2-carboxylate (Intermediate 12 of Example 3) (0.05 g, 28 % yield) as a brown solid. MS(LCMS): 832.41 *m/z* [M+H].

Step 12) Example 3: Preparation of 4-((14aS)-11-chloro-9-fluoro-7-(((2R,7aS)-2-fluorotetrahydro-1H-pyrrolizin-7a(5H)-yl)methoxy)-1,3,4,13,14,14a-hexahydro-2H-pyrazino[1',2':5,6][1,5]oxazocino[4,3,2-de]quinazolin-10-yl)-7-fluorobenzo[d]thiazol-2-amine

A solution of tert-butyl (14aS)-10-(2-((tert-butoxycarbonyl)amino)-7-fluorobenzo[d]thiazol-4-yl)-11-chloro-9-fluoro-7-(((2R,7aS)-2-fluorotetrahydro-1H-pyrrolizin-7a(5H)-yl)methoxy)-1,3,4,13,14,14a-hexahydro-2H-pyrazino[1',2':5,6][1,5]oxazocino[4,3,2-de]quinazolin-2-carboxylate (Intermediate 12 of Example 3) (0.05 g, 0.06 mmol) in 4M HCl in dioxane (1.0 mL, 20 vol) was stirred at room temperature for 5 hours. The progress of the reaction was monitored by TLC. After completion of the reaction, the reaction mixture was concentrated under reduced pressure to obtain a crude compound. The crude compound was purified by preparative HPLC to obtain 4-((14aS)-11-chloro-9-fluoro-7-(((2R,7aS)-2-fluorotetrahydro-1H-pyrrolizin-7a(5H)-yl)methoxy)-1,3,4,13,14,14a-hexahydro-2H-pyrazino[1',2':5,6][1,5]oxazocino[4,3,2-de]quinazolin-10-yl)-7-fluorobenzo[d]thiazol-2-amine (Example 3) (0.0075 g, 20 % yield) as a grayish-white solid.

$^1H$ NMR(400 MHz, DMSO-$d_6$) $\delta$ 1.70-1.91 (m, 4H), 1.93-2.16(m, 3H), 2.54-2.56(m, 1H), 2.64-2.70(m, 1H),

2.77-2.82 (m, 3H), 3.00-3.10(m, 4H), 3.19-3.24(m, 1H), 3.64-3.74(m, 1H), 3.94(dd, *J* = 10.51, 2.25Hz, 1H), 4.06(dd , *J* = 10.51, 2.75 Hz, 1H), 4.24(q, *J* = 11. 6Hz, 1H), 4.43(brs, 1H), 4.55-4.93(m, 1H), 5.20-5.34(m, 1H), 7.01- 7.06(m, 1H), 7.14-7.23(m, 1H), 7.89(d, *J* = 16.76 Hz, 2H), -NH protons were not observed in $^{1}$H NMR. MS(LCMS): 632.20 *m/z* [M+H].

**Synthesis Reaction Equation of Example 4**

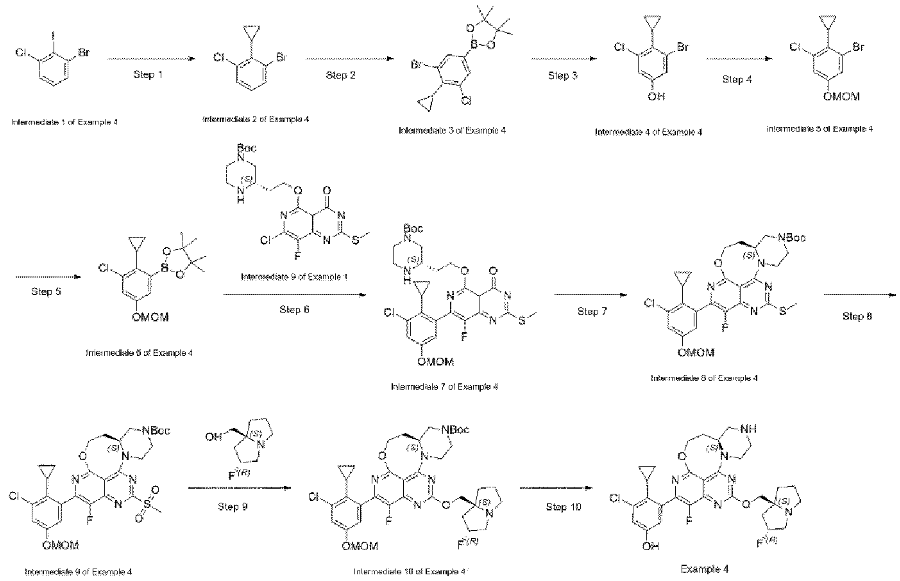


**Example 4: Preparation of 3-chloro-4-cyclopropyl-5-((S)-1-fluoro-12-(((2R,7aS)-2-fluorotetrahydro-1H-pyrrolizin-7a(5H)-yl)methoxy)-6,6a,7,8,9,10-hexahydro-5H-4-oxa-3,8,10a,11,13-pentaazabenzo[4,5]cycloocta[1,2,3-de] naphthalen-2-yl)phenol**

Step 1) Intermediate 2 of Example 4: Preparation of 1-bromo-3-chloro-2-cyclopropylbenzene

A mixture of 1-bromo-3-chloro-2-iodobenzene (22 g, 69.65 mmol), cyclopropylboronic acid (15.21 g, 90.54 mmol), Pd(dppf)$Cl_2$ (2.3 g, 3.16 mmol), and $K_3PO_4$ (24.2 g, 113.96 mmol) in dioxane:water (100 mL:30 mL) was stirred under $N_2$ gas at 100 °C for 7 hours. In TLC (PE/EA = 1/0), it was found that SM1 (Rf = 0.6) was consumed and a new spot (Rf = 0.7) was formed. The reaction mixture was diluted with water and extracted with ethyl acetate (200 mL × 2). The combined organic layers were washed with 70 mL of brine, dried over $Na_2SO_4$, filtered, and concentrated in vacuum to obtain a residue. The residue was purified by column chromatography (PE/EA = 1/0) to obtain 1-bromo-3-chloro-2-cyclopropylbenzene (13 g, 81.25 % yield) as a colorless liquid.

$^{1}$H NMR(400 MHz, $CDCl_3$) δ 7.46(dd, *J* = 8.0, 0.9 Hz, 1H), 7.30(dd, *J* = 8.0, 0.9Hz, 1H), 6.99(t, *J* = 8.0 Hz, 1H), 1.81 - 1.71 (m, 1H), 1.22 - 1.14(m, 2H), 0.81 - 0.73(m, 2H).

Step 2) Intermediate 3 of Example 4: Preparation of 2-(3-bromo-5-chloro-4-cyclopropylphenyl)-4,4,5,5-tetramethyl-1,3,2-dioxaborolane

A mixture of 1-bromo-3-chloro-2-cyclopropylbenzene (13 g, 56.52mmol), [Ir(OMe)(COD)]$_2$(3.74 g, 5.65 mmol), 4,4'-di-tert-butyl-2,2'-bipyridine (1.82g, 6.78 mmol), and 4,4,5,5-tetramethyl-1,3,2-dioxaborolane (21.70 g, 169.56 mmol) in hexane (130 mL) was stirred under $N_2$ gas at 60 °C for 3 hours. In TLC (PE/EA = 50/0), it was found that SM1 (Rf = 0.3) was consumed and a new spot (Rf = 0.5) was formed. The reaction mixture was concentrated under reduced pressure to remove a solvent. The residue was purified by column chromatography (PE:EA = 50:1) to obtain 2-(3-bromo-5-chloro-4-cyclopropylphenyl)-4,4,5,5-tetramethyl-1,3,2-dioxaborolane (10.7 g, 53.18 % yield) as a colorless oil.

$^{1}$H NMR(400 MHz, $CDCl_3$) δ 7.87(s, 1H), 7.70(s, 1H), 1.78(tt, *J* = 8.5, 5.8 Hz, 1H), 1.33(s, 12H), 1.22 - 1.14(m, 2H), 0.77(q, *J* = 5.8 Hz, 2H).

Step 3) Intermediate 4 of Example 4: Preparation of 2-(3-bromo-5-chloro-4-cyclopropylphenyl)-4,4,5,5-tetramethyl-1,3,2-dioxaborolane

To a solution of 2-(3-bromo-5-chloro-4-cyclopropylphenyl)-4,4,5,5-tetramethyl-1,3,2-dioxaborolane (10.5 g, 29.49 mmol) in THF/$H_2O$ (100/50 mL), AcOH (113.4 g, 1888.42 mmol, 107.5 mL) and $H_2O_2$ (2.05 g, 4.79 mmol, 15.96 mL, 30 % purity) were added at 0 °C, and the mixture was stirred at 0 °C for 1 hour. In TLC (PE/EA = 15/0), it was found that SM1 (Rf = 0.7) was consumed and a new spot (Rf = 0.4) was formed. The reaction mixture was quenched at 0 °C with $Na_2S_2O_3$ (10 % aq, 80 mL). The mixture was extracted with EA (100 mL × 2). The combined organic layers were washed with brine (100 mL) and dried over $Na_2SO_4$. The mixture was filtered and concentrated under reduced pressure to obtain a residue. The residue was purified by column chromatography (PE:EA = 10:1) to obtain 3-bromo-5-chloro-4-cyclopropylphenol as a colorless oil (5.7 g, 78.62 % yield).

$^1$H NMR(400 MHz, $CDCl_3$) δ 7.00(d, *J* = 2.6 Hz, 1H), 6.83(d, *J* = 2.6 Hz, 1H), 3.73(d, *J* = 47.7 Hz, 1H), 1.66(ddd, *J* = 14.0, 7.0, 4.2Hz, 1H), 1.15 - 1.08(m, 2H), 0.71(q, *J* = 5.7Hz, 2H).

Step 4) Intermediate 5 of Example 4: Preparation of 1-bromo-3-chloro-2-cyclopropyl-5-(methoxymethoxy)benzene

To a solution of 3-bromo-5-chloro-4-cyclopropylphenol (5.7 g, 23.17 mmol) in DCM (57 mL), DIEA (4.49 g, 34.76 mmol) and methoxymethyl hypobromite (3.19 g, 25.49 mmol) were added at 0 °C. The reaction mixture was then stirred at 25 °C for 2 hours. The reaction mixture was diluted with water and extracted with DCM (50 mL × 2). The combined organic layers were washed with 70 mL of brine, dried over $Na_2SO_4$, filtered, and concentrated in vacuum to obtain a residue. The residue was purified by column chromatography (PE:EA = 20:1) to obtain 1-bromo-3-chloro-2-cyclopropyl-5-(methoxymethoxy)benzene (4 g, 59.52 % yield) as a colorless oil.

$^1$H NMR(400 MHz, $CDCl_3$) δ 7.12(d, *J* = 2.5 Hz, 1H), 6.96(d, *J* = 2.5 Hz, 1H), 5.04(s, 2H), 3.38(s, 3H), 1.61 (s, 1H), 1.08 - 1.02(m, 2H), 0.65(dd, *J* = 5.6, 1.1 Hz, 2H).

Step 5) Intermediate 6 of Example 4: Preparation of 2-(3-chloro-2-cyclopropyl-5-(methoxymethoxy)phenyl)-4,4,5,5-tetramethyl-1,3,2-dioxaborolane

To a solution of 1-bromo-3-chloro-2-cyclopropyl-5-(methoxymethoxy)benzene (4 g, 13.79 mmol), KOAc (4.06 g, 41.38 mmol), and 4,4,5,5-tetramethyl-2-(4,5,5-trimethyl-4-methyl-1,3,2-dioxaborolan-2-yl)-1,3,2-dioxaborolane (7.0 g, 27.59 mmol) in dioxane (80 mL), Pd(dppf)$Cl_2$ (1.0 g, 1.38 mmol) was added. The mixture was stirred at 100 °C for 16 hours. The reaction mixture was diluted with water and extracted with ethyl acetate (50 mL × 3). The combined organic layers were washed with 70 mL of brine, dried over $Na_2SO_4$, filtered, and concentrated in vacuum to obtain a residue. The residue was purified by column chromatography (PE:EA = 10:1) to obtain 2-(3-chloro-2-cyclopropyl-5-(methoxymethoxy)phenyl)-4,4,5,5-tetramethyl-1,3,2-dioxaborolane (1.4 g, 30.04 % yield) as a colorless oil. MS: *m*/*z* = 339.1(M+H$^+$, ESI+)

Step 6) Intermediate 7 of Example 4: Preparation of tert-butyl (3S)-3-(2-((7-(3-chloro-2-cyclopropyl-5-(methoxymethoxy)phenyl)-8-fluoro-2-(methylthio)-4-oxo-4,4a-dihydropyrido[4,3-d]pyrimidin-5-yl)oxy)ethyl)piperazine-1-carboxylate

A solution of tert-butyl (3S)-3-(2-((7-chloro-8-fluoro-2-(methylthio)-4-oxo-4,4a-dihydropyrido[4,3-d]pyrimidin-5-yl)oxy)ethyl)piperazine-1-carboxylate (intermediate 9 of Example 1, 1.4 g, 2.96 mmol), 2-(3-chloro-2-cyclopropyl-5-(methoxymethoxy)phenyl)-4,4,5,5-tetramethyl-1,3,2-dioxaborolane (2.0 g, 5.92 mmol), Ruphos-Pd-$G_3$ (742 mg, 0.89 mmol), and $K_3PO_4$ (1.88 g, 8.88 mmol) in a mixture of dioxane/$H_2O$ (16 mL/4 mL) was stirred at 90 °C for 3 hours under $N_2$ gas. LCMS showed that the starting material was consumed and the desired mass was formed. The reaction mixture was quenched with water (60 mL) and extracted with ethyl acetate (60 mL × 3). The combined organic layers were washed with 60 mL of brine, dried over $Na_2SO_4$, filtered, and concentrated in vacuum to obtain a residue. The residue was purified by column chromatography (PE:EA = 0:1) to obtain tert-butyl (3S)-3-(2-((7-(3-chloro-2-cyclopropyl-5-(methoxymethyl)phenyl)-8-fluoro-2-(methylthio)-4-oxo-4,4a-dihydropyrido[4,3-d]pyrimidin-5-yl)oxy)ethyl)piperazine-1-carboxylate (700 mg, 36.46 % yield) as a yellow solid. MS: *m*/*z* = 650.3(M+H$^+$, ESI+)

Step 7) Intermediate 8 of Example 4: Preparation of tert-butyl (S)-2-(3-chloro-2-cyclopropyl-5-(methoxymethoxy)phenyl)-1-fluoro-12-(methylthio)-5,6,6a,7,9,10-hexahydro-8H-4-oxa-3,8,10a,11,13-pentaazabenzo[4,5]cycloocta[1,2,3-de]naphthalen-8-carboxylate

To a solution of tert-butyl (3S)-3-(2-((7-(3-chloro-2-cyclopropyl-5-(methoxymethyl)phenyl)-8-fluoro-2-(methylthio)-4-oxo-4,4a-dihydropyrido[4,3-d]pyrimidin-5-yl)oxy)ethyl)piperazine-1-carboxylate (700 mg, 1.08 mmol) in DCM (47 mL), BoPCl (822 mg, 3.24 mmol) and DIEA (1.25 g, 9.71 mmol) were added. The mixture was then stirred at 25

°C for 16 hours. LCMS showed that the starting material was consumed and the desired mass was formed. The reaction mixture was diluted with water (20 mL) and extracted with DCM (40 mL x 2). The combined organic layers were washed with 60 mL of brine, dried over $Na_2SO_4$, filtered, and concentrated in vacuum to obtain a residue. The residue was purified by column chromatography (PE:EA = 1:1) to obtain tert-butyl (S)-2-(3-chloro-2-cyclopropyl-5-(methoxymethoxy)phenyl)-1-fluoro-12-(methylthio)-5,6,6a,7,9,10-hexahydro-8H-4-oxa-3,8,10a,11,13-pentaazabenzo[4,5]cycloocta[1,2,3-de]naphthalen-8-carboxylate (140 mg, 68.06 % yield) as a yellow solid. MS: *m/z* = 632.4(M+H$^+$, ESI+)

Step 8) Intermediate 9 of Example 4: Preparation of tert-butyl (6aS)-2-(3-chloro-2-cyclopropyl-5-(methoxymethoxy)phenyl)-1-fluoro-12-(methylsulfinyl)-5,6,6a,7,9,10-hexahydro-8H-4-oxa-3,8,10a,11,13-pentaazabenzo[4,5]cycloocta[1,2,3-de]naphthalen-8-carboxylate

To a solution of tert-butyl (S)-2-(3-chloro-2-cyclopropyl-5-(methoxymethoxy)phenyl)-1-fluoro-12-(methylthio)-5,6,6a,7,9,10-hexahydro-8H-4-oxa-3,8,10a,11,13-pentaazabenzo[4,5]cycloocta[1,2,3-de]naphthalen-8-carboxylate(110 mg, 0.17 mmol) in ACN/$H_2O$ (10 mL/10 mL), oxane (536 mg, 0.87 mmol) was added and stirred at 25 °C for 1 hour. LCMS showed that the starting material was consumed and the desired mass was detected. The reaction mixture was diluted with water (20 mL) and extracted with DCM (30 mL x 2). The combined organic layers were washed with brine (20 mL), dried over $Na_2SO_4$, filtered, and concentrated in vacuum to obtain tert-butyl (6aS)-2-(3-chloro-2-cyclopropyl-5-(methoxymethoxy)phenyl)-1-fluoro-12-(methylsulfinyl)-5,6,6a,7,9,10-hexahydro-8H-4-oxa-3,8,10a,11,13-pentaazabenzo[4,5]cycloocta[1,2,3-de]naphthalen-8-carboxylate (120 mg, crude compound) as a yellow solid. MS: *m/z* = 648.2(M+H$^+$, ESI+)

Step 9) Intermediate 10 of Example 4: Preparation of tert-butyl (S)-2-(3-chloro-2-cyclopropyl-5-(methoxymethoxy)phenyl)-1-fluoro-12-(((2R,7aS)-2-fluorotetrahydro-1H-pyrrolizin-7a(5H)-yl)methoxy)-5,6,6a,7,9,10-hexahydro-8H-4-oxa-3,8,10a,11,13-pentaazabenzo[4,5]cycloocta[1,2,3-de]naphthalen-8-carboxylate

To a solution of tert-butyl (6aS)-2-(3-chloro-2-cyclopropyl-5-(methoxymethoxy)phenyl)-1-fluoro-12-(methylsulfinyl)-5,6,6a,7,9,10-hexahydro-8H-4-oxa-3,8,10a,11,13-pentaazabenzo[4,5]cycloocta[1,2,3-de]naphthalen-8-carboxylate (120 mg, 0.18 mmol) and ((2R,7aS)-2-fluorotetrahydro-1H-pyrrolizin-7a(5H)-yl)methanol (44 mg, 0.28 mmol) in THF (20 mL), t-BuONa (36 mg, 0.37 mmol) was added, and the mixture was stirred at 0 °C for 1 hour. LCMS showed that the starting material was consumed and the desired mass was detected. The reaction mixture was diluted with water (30 mL) and extracted with DCM (30 mL x 2). The combined organic layers were washed with 20 mL of brine, dried over $Na_2SO_4$, filtered, and concentrated in vacuum to obtain a residue. The residue was purified by pre-TLC (DCM/MeOH = 10/1) to obtain tert-butyl (S)-2-(3-chloro-2-cyclopropyl-5-(methoxymethoxy)phenyl)-1-fluoro-12-(((2R,7aS)-2-fluorotetrahydro-1H-pyrrolizin-7a(5H)-yl)methoxy)-5,6,6a,7,9,10-hexahydro-8H-4-oxa-3,8,10a,11,13-pentaazabenzo[4,5]cycloocta[1,2,3-de]naphthalen-8-carboxylate (70 mg, 52.24 % yield) as a yellow solid. MS: *m/z* = 743.3(M+H$^+$, ESI+)

Step 10) Example 4: Preparation of 3-chloro-4-cyclopropyl-5-((S)-1-fluoro-12-(((2R,7aS)-2-fluorotetrahydro-1H-pyrrolizin-7a(5H)-yl)methoxy)-6,6a,7,8,9,10-hexahydro-5H-4-oxa-3,8,10a,11,13-pentaazabenzo[4,5]cycloocta[1,2,3-de]naphthalen-2-yl)phenol

To a solution of tert-butyl (S)-2-(3-chloro-2-cyclopropyl-5-(methoxymethoxy)phenyl)-1-fluoro-12-(((2R,7aS)-2-fluorotetrahydro-1H-pyrrolizin-7a(5H)-yl)methoxy)-5,6,6a,7,9,10-hexahydro-8H-4-oxa-3,8,10a,11,13-pentaazabenzo[4,5]cycloocta[1,2,3-de]naphthalen-8-carboxylate (70 mg, 0.09 mmol) in EA (2 mL), 3M HCl (7 mL) was added, and the mixture was stirred at 0°C for 1 hour, LCMS showed that the starting material was consumed, and the desired mass was formed. The reaction mixture was concentrated in vacuum to obtain a residue. The residue was purified by pre-HPLC (FA conditions) to obtain 3-chloro-4-cyclopropyl-5-((S)-1-fluoro-12-(((2R,7aS)-2-fluorotetrahydro-1H-pyrrolizin-7a(5H)-yl)methoxy)-6,6a,7,8,9,10-hexahydro-5H-4-oxa-3,8,10a,11,13-pentaazabenzo[4,5]cycloocta[1,2,3-de]naphthalen-2-yl)phenol (Example 4) (2.76 mg, 4.9 % yield) as a white solid.

$^1$H NMR (400 MHz, MeOD) δ 8.35 (brs, 2H, FA), 6.95 (d, *J* = 2.4 Hz, 1H), 6.77 (s, 1H), 5.52 (d, *J* = 52.6 Hz, 1H), 5.35 - 5.17 (m, 1H), 4.69 - 4.45 (m, 3H), 4.40 - 4.25 (m, 1H), 4.20 - 4.10 (m, 1H), 4.01 - 3.57 (m, 3H), 3.48 - 3.32 (m, 3H), 3.25 - 3.13 (m, 2H), 3.06 - 2.93 (m, 1H), 2.73 - 2.44 (m, 3H), 2.40 - 2.22 (m, 3H), 2.20 - 2.02 (m, 2H), 1.90 - 1.77 (m, 1H), 0.15 - 0.57 (m, 2H), 0.23 - 0.03 (m, 2H). MS: *m/z* = 599.6(M+H$^+$, ESI+)

**Synthesis Reaction Equation of Example 5**

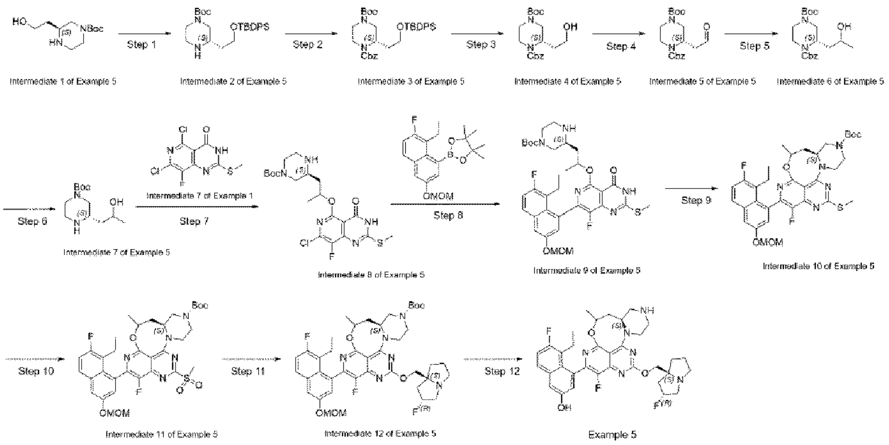


**Example 5: Preparation of 5-ethyl-6-fluoro-4-((6aS)-1-fluoro-12-(((2R,7aS)-2-fluorotetrahydro-1H-pyrrolizin-7a(5H)-yl)methoxy)-5-methyl-6,6a,7,8,9,10-hexahydro-5H-4-oxa-3,8,10a,11,13-pentaazabenzo[4,5]cycloocta[1,2,3-de]naphthalen-2-yl)naphthalen-2-ol**

Step 1) Intermediate 2 of Example 5: Preparation of tert-butyl (S)-3-(2-((tert-butyldiphenylsilyl)oxy)ethyl)piperazine-1-carboxylate

To a mixture of tert-butyl (S)-3-(2-hydroxyethyl)piperazine-1-carboxylate (8 g, 34.78 mmol), DMAP (848 mg, 6.95 mmol), and imidazole (4.73 g, 69.65 mmol) in DCM (80 mL), TBDPSCI (19.11 g, 69.65 mmol) was added, and the reaction mixture was stirred at 25 °C for 16 hours. TLC (PE/EA = 2/1) showed that SM1 (Rf = 0.2) was consumed and a new spot (Rf = 0.4) was formed. The reaction mixture was diluted with $H_2O$ (100 mL) and extracted with DCM (150 mL x 3). The combined organic layers were washed with brine (150 mL) and dried over $Na_2SO_4$. A residue was obtained by filtration and concentration in vacuum. The residue was purified by column chromatography (PE:EA = 3:1) to obtain tert-butyl (S)-3-(2-((tert-butyl diphenylsilyl)oxy)ethyl)piperazine-1-carboxylate (14.55 g, 91 % yield) as a colorless oil. MS: *m/z* = 469.4(M+H$^+$, ESI+)

Step 2) Intermediate 3 of Example 5: Preparation of 1-benzyl 4-(tert-butyl)(S)-2-(2-((tert-butyldiphenylsilyl)oxy)ethyl) piperazine-1,4-dicarboxylate

To a mixture of tert-butyl (S)-3-(2-((tert-butyl diphenylsilyl)oxy)ethyl)piperazine-1-carboxylate (14.55 g, 31.08 mmol) and DIEA (6.01 g, 46.62mmol) in DCM (150 mL), CBZCI (13.21 g, 77.7 mmol) was added at 0 °C, and the reaction mixture was stirred under $N_2$ gas at 0 °C for 1 hour. The reaction mixture was diluted with $H_2O$ (100 mL) and extracted with EA (150 mL x 3). The combined organic layers were washed with brine (150 mL) and dried over $Na_2SO_4$. A residue was obtained by filtration and concentration in vacuum. The residue was purified by column chromatography (PE:EA = 50:1) to obtain 1-benzyl 4-(tert-butyl)(S)-2-(2-((tert-butyldiphenylsilyl)oxy)ethyl)piperazine-1,4-dicarboxylate (13.806 g, 73 % yield) as a colorless oil. MS: *m/z* = 688.2 (M+H$^+$, ESI+)

$^1$H NMR(400 MHz, $CDCl_3$) $\delta$ 7.64(d, *J* = 6.7 Hz, 4H), 7.35(ddd, J = 40.3, 20.2, 11.1 Hz, 11H), 5.11(d, *J* = 17.9 Hz, 2H), 4.44 (s, 1H), 4.02 - 3.56 (m, 5H), 2.92 (td, J = 12.8, 2.9 Hz, 3H), 1.89 - 1.70 (m, 2H), 1.40 (s, 9H), 1.03 (s, 9H).

Step 3) Intermediate 4 of Example 5: Preparation of 1-benzyl 4-(tert-butyl) (S)-2-(2-hydroxyethyl)piperazine-1,4-dicarboxylate

To a solution of 1-benzyl 4-(tert-butyl)(S)-2-(2-((tert-butyldiphenylsilyl)oxy)ethyl)piperazine-1,4-dicarboxylate (13.806 g, 22.93 mmol) in THF (150 mL), TBAF (91.7 mL, 1M in THF) was added, and the mixture was stirred under $N_2$ gas at room temperature for 2 hours. LCMS showed that the starting material was consumed and the target compound was detected. The reaction mixture was diluted with $H_2O$ (100 mL) and extracted with EA (150 mL x 3). The combined organic layers were washed with brine (150 mL) and dried over $Na_2SO_4$. A residue was obtained by filtration and concentration in vacuum. The residue was purified by column chromatography (PE:EA = 5:1) to obtain 1-benzyl 4-(tert-butyl)(S)-2-(2-hydroxyethyl)piperazine-1,4-dicarboxylate (7.4 g, 92 % yield) as a colorless oil. MS: *m/z* = 387.2(M+H$^+$, ESI+)

Step 4) Intermediate 5 of Example 5: Preparation of 1-benzyl 4-(tert-butyl) (S)-2-(2-hydroxyethyl)piperazine-1,4-dicarboxylate

To a solution of oxalyl chloride (6.41 g, 50.5 mmol) in DCM (60 mL), a solution of dimethyl sulfoxide (5.51 mg, 70.7 mmol) in dichloromethane (50 mL) was added at -78 °C. After stirring at -78 °C for 0.5 hours, and then a solution of (S)-1-benzyl 4-tert-butyl 2-(2-hydroxyethyl)piperazine-1,4-dicarboxylate (7.4 g, 20.2 mmol) in DCM (20 mL) was added to the mixture. After stirring at -78 °C for 3 hours, TEA (15.3 g, 151.5 mmol) was added dropwise. The reaction mixture was further heated to 25 °C for 0.5 hours. LCMS showed that the starting material was consumed and the target compound was detected. The reaction mixture was diluted with $H_2O$ (60 mL) and extracted with DCM (60 mL x 3). The combined organic layers were washed with brine (30 mL) and dried over $Na_2SO_4$. A residue was obtained by filtration and concentration in vacuum. The residue was purified by column chromatography (PE:EA = 5:1) to obtain 1-benzyl 4-(tert-butyl)(S)-2-(2-hydroxyethyl)piperazine-1,4-dicarboxylate (5.4 g, 74.5 % yield) as a colorless oil. MS: *m/z* = 363.2(M+$H^+$, ESI+)

Step 5) Intermediate 6 of Example 5: Preparation of 1-benzyl 4-(tert-butyl)(2S)-2-(2-hydroxypropyl)piperazine-1,4-dicarboxylate

To a stirred solution of 1-benzyl 4-(tert-butyl)(S)-2-(2-hydroxyethyl)piperazine-1,4-dicarboxylate (5.4 g, 14.9 mmol) dissolved in dry THF (25 mL), a 1 M solution of $CH_3MgBr$ (14.9 mL, 14.9 mmol) in THF was added at 0 °C. The reactant was stirred at 25 °C for 2 hours, and LCMS showed that the starting material was consumed and the target compound was detected. The reaction mixture was quenched with saturated $NH_4Cl$ (40 mL) and extracted with DCM (60 mL x 3). The combined organic layers were washed with brine (30 mL) and dried over $Na_2SO_4$. A residue was obtained by filtration and concentration in vacuum. The residue was purified by column chromatography (PE:EA = 5:1) to obtain 1-benzyl 4-(tert-butyl)(2S)-2-(2-hydroxypropyl)piperazine-1,4-dicarboxylate (2 g, 35.5 % yield) as a colorless oil. MS: *m/z* = 401.2(M+Na+, ESI+)

Step 6) Intermediate 7 of Example 5: Preparation of tert-butyl (3S)-3-(2-hydroxypropyl)piperazine-1-carboxylate

To a suspension of 1-benzyl 4-(tert-butyl)(2S)-2-(2-hydroxypropyl)piperazine-1,4-dicarboxylate (2 g, 5.29 mmol) in MeOH (20 mL), $Pd(OH)_2$ (200 mg) was added, and the mixture was stirred under a hydrogen atmosphere at 25 °C for 2 hours. The mixture was filtered through a Celite pad, and the filtrate was evaporated to obtain tert-butyl (3S)-3-(2-hydroxypropyl)piperazine-1-carboxylate (1.01 g, 83 % yield) as a colorless oil. MS: *m/z* = 245.2(M+$H^+$, ESI+)

Step 7) Intermediate 8 of Example 5: Preparation of tert-butyl (3S)-3-(2-((7-chloro-8-fluoro-2-(methylthio)-4-oxo-3,4-dihydropyrido[4,3-d]pyrimidin-5-yl)oxy)propyl)piperazine-1-carboxylate

To a mixture of tert-butyl (3S)-3-(2-hydroxypropyl)piperazine-1-carboxylate (1.01 g, 4.14 mmol) in THF (15 mL), NaH (553 mg, 13.8 mmol, 60 % suspension in mineral oil) was added, and the mixture was stirred at 0 °C for 0.5 hours. 5,7-dichloro-8-fluoro-2-(methylthio)pyrido[4,3-d]pyrimidin-4(3H)-one (1.29 g, 4.61 mmol) was added to the mixture, and the mixture was stirred at 60 °C for an additional 1 hour. TLC (EA/MeOH = 15/1) showed that SM1 (Rf = 0.2) was consumed and a new spot (Rf = 0.4) was formed. The reaction mixture was quenched with water (40 mL) and extracted with ethyl acetate (50 mL × 3). The combined organic layers were washed with 60 mL of brine, dried over $Na_2SO_4$, filtered, and concentrated in vacuum to obtain a residue. The residue was purified by column chromatography (PE/EA = 0/1) to obtain tert-butyl (3S)-3-(2-((7-chloro-8-fluoro-2-(methylthio)-4-oxo-3,4-dihydropyrido[4,3-d]pyrimidin-5-yl)oxy)propyl)piperazine-1-carboxylate (934 mg, 46.3 % yield) as a white solid. MS: *m/z* = 488.1(M+$H^+$, ESI+)

Step 8) Intermediate 9 of Example 5: Preparation of tert-butyl (3S)-3-(2-((7-(8-ethyl-7-fluoro-3-(methoxymethoxy) naphthalen-1-yl)-8-fluoro-2-(methylthio)-4-oxo-3,4-dihydropyrido[4,3-d]pyrimidin-5-yl)oxy)propyl)piperazine-1-carboxylate

A mixture of tert-butyl (3S)-3-(2-((7-chloro-8-fluoro-2-(methylthio)-4-oxo-3,4-dihydropyrido[4,3-d]pyrimidin-5-yl)oxy)propyl)piperazine-1-carboxylate (934 mg, 1.92 mmol), 2-(8-ethyl-7-fluoro-3-(methoxymethoxy)naphthalen-1-yl)-4,4,5,5-tetramethyl-1,3,2-dioxaborolane (691.2 mg, 1.92 mmol), Ruphos-Pd-$G_3$ (481.5 mg, 0.576 mmol), and $K_3PO_4$ (1.22 g, 5.76 mmol) in dioxane/$H_2O$ (8 mL/2 mL) was stirred under $N_2$ gas at 90 °C for 3 hours. LCMS showed that the starting material was consumed and the target compound was detected. The reaction mixture was diluted with water (30 mL) and extracted with DCM (40 mL x 2). The combined organic layers were washed with 60 mL of brine, dried over $Na_2SO_4$, filtered, and concentrated in vacuum to obtain a residue. The residue was purified by column chromatography (PE/EA = 0/1) to obtain tert-butyl (3S)-3-(2-((7-(8-ethyl-7-fluoro-3-(methoxymethoxy)naphthalen-1-yl)-8-fluoro-2-(methylthio)-4-oxo-3,4-dihydropyrido[4,3-d]pyrimidin-5-yl)oxy)propyl)piperazine-1-carboxylate (235 mg, 20 % yield)

as a light yellow solid. MS: m/z = 686.3(M+H+, ESI+)

Step 9) Intermediate 10 of Example 5: Preparation of tert-butyl (6aS)-2-(8-ethyl-7-fluoro-3-(methoxymethoxy)naphthalen-1-yl)-1-fluoro-5-methyl-12-(methylthio)-5,6,6a,7,9,10-hexahydro-8H-4-oxa-3,8,10a,11,13-pentaazabenzo[4,5]cycloocta[1,2,3-delnaphthalen-8-carboxylate

To a solution of tert-butyl (3S)-3-(2-((7-(8-ethyl-7-fluoro-3-(methoxymethoxy)naphthalen-1-yl)-8-fluoro-2-(methylthio)-4-oxo-3,4-dihydropyrido[4,3-d]pyrimidin-5-yl)oxy)propyl)piperazine-1-carboxylate (100 mg, 0.15 mmol) in DCM (4 mL), DIEA (169 mg, 1.31 mmol) and BOPCl (114 mg, 0.45 mmol) were added. The mixture was then stirred at 25 °C for 16 hours. LCMS showed that the starting material was consumed and the target compound was detected. The reaction mixture was diluted with water (20 mL) and extracted with DCM (30 mL x 2). The combined organic layers were washed with 60 mL of brine, dried over $Na_2SO_4$, filtered, and concentrated in vacuum to obtain a residue. The residue was purified by column chromatography (PE/EA = 1/1) to obtain tert-butyl (6aS)-2-(8-ethyl-7-fluoro-3-(methoxymethoxy) naphthalen-1-yl)-1-fluoro-5-methyl-12-(methylthio)-5,6,6a,7,9,10-hexahydro-8H-4-oxa-3,8,10a,11,13-pentaazabenzo [4,5]cycloocta[1,2,3-de]naphthalen-8-carboxylate (40 mg, 41.2 % yield) as a white solid. MS: m/z = 668.3(M+H+, ESI+)

Step 10) Intermediate 11 of Example 5: Preparation of tert-butyl (6aS)-2-(8-ethyl-7-fluoro-3-(methoxymethoxy) naphthalen-1-yl)-1-fluoro-5-methyl-12-(methylsulfonyl)-5,6,6a,7,9,10-hexahydro-8H-4-oxa-3,8,10a,11,13-pentaazabenzo[4,5]cycloocta[1,2,3-delnaphthalen-8-carboxylate

To a solution of tert-butyl (6aS)-2-(8-ethyl-7-fluoro-3-(methoxymethoxy)naphthalen-1-yl)-1-fluoro-5-methyl-12-(methylthio)-5,6,6a,7,9,10-hexahydro-8H-4-oxa-3,8,10a,11,13-pentaazabenzo[4,5]cycloocta[1,2,3-de] naphthalen-8-carboxylate (40 mg, 0.11 mmol) in $MeCN/H_2O$ (1 mL/1 mL), oxone (190.3 mg, 0.55 mmol) was added, and the mixture was stirred at 25 °C for 1 hour. LCMS showed that the starting material was consumed and the desired mass was detected. The reaction mixture was diluted with water (20 mL) and extracted with DCM (30 mL x 2). The combined organic layer was washed with 20 mL of brine, dried over $Na_2SO_4$, filtered, and concentrated in vacuum to obtain tert-butyl (6aS)-2-(8-ethyl-7-fluoro-3-(methoxymethoxy)naphthalen-1-yl)-1-fluoro-5-methyl-12-(methylsulfonyl)-5,6,6a,7,9,10-hexahydro-8H-4-oxa-3,8,10a,11,13-pentaazabenzo[4,5]cycloocta[1,2,3-de]naphthalen-8-carboxylate (50 mg, 65 % yield, crude compound) as a white solid. MS: m/z = 700.2(M+H+, ESI+)

Step 10) Intermediate 12 of Example 5: Preparation of tert-butyl (6aS)-2-(8-ethyl-7-fluoro-3-(methoxymethoxy) naphthalen-1-yl)-1-fluoro-12-(((2R,7aS)-2-fluorotetrahydro-1H-pyrrolizin-7a(5H)-yl)methoxy)-5-methyl-5,6,6a,7,9,10-hexahydro-8H-4-oxa-3,8,10a,11,13-pentaazabenzo[4,5]cycloocta[1,2,3-de]naphthalen-8-carboxylate

To a solution of tert-butyl (6aS)-2-(8-ethyl-7-fluoro-3-(methoxymethoxy)naphthalen-1-yl)-1-fluoro-5-methyl-12-(methylsulfonyl)-5,6,6a,7,9,10-hexahydro-8H-4-oxa-3,8,10a,11,13-pentaazabenzo[4,5]cycloocta[1,2,3-de] naphthalen-8-carboxylate (50 mg, 0.071 mmol) and ((2R,7aS)-2-fluorotetrahydro-1H-pyrrolizin-7a(5H)-yl)methanol (16.9 mg, 0.106 mmol) in dry THF (2 mL), t-BuONa (11 mg, 0.14 mmol) was added, and the mixture was stirred under $N_2$ gas at -60 °C for 1 hour. LCMS showed that the starting material was consumed and the desired mass was formed. The reaction mixture was quenched with water (60 mL) and extracted with ethyl acetate (60 mL × 3). The combined organic layers were washed with 60 mL of brine, dried over $Na_2SO_4$, filtered, and concentrated in vacuum to obtain a residue. The residue was purified by pre-TLC (PE/EA = 0/1) to obtain tert-butyl (6aS)-2-(8-ethyl-7-fluoro-3-(methoxymethoxy)naphthalen-1-yl)-1-fluoro-12-(((2R,7aS)-2-fluorotetrahydro-1H-pyrrolizin-7a(5H)-yl)methoxy)-5-methyl-5,6,6a,7,9,10-hexahydro-8H-4-oxa-3,8,10a,11,13-pentaazabenzo[4,5]cycloocta[1,2,3-de]naphthalen-8-carboxylate (20 mg, 36 % yield). LC-MS MS: *m*/*z* = 779.3(M+H+, ESI+)

Step 11) Example 5: Preparation of 5-ethyl-6-fluoro-4-((6aS)-1-fluoro-12-(((2R,7aS)-2-fluorotetrahydro-1H-pyrrolizin-7a(5H)-yl)methoxy)-5-methyl-6,6a,7,8,9,10-hexahydro-5H-4-oxa-3,8,10a,11,13-pentaazabenzo[4,5]cycloocta[1,2,3-de]naphthalen-2-yl)naphthalen-2-ol

To a solution of tert-butyl (6aS)-2-(8-ethyl-7-fluoro-3-(methoxymethoxy)naphthalen-1-yl)-1-fluoro-12-(((2R,7aS)-2-fluorotetrahydro-1H-pyrrolizin-7a(5H)-yl)methoxy)-5-methyl-5,6,6a,7,9,10-hexahydro-8H-4-oxa-3,8,10a,11,13-pentaazabenzo[4,5]cycloocta[1,2,3-de]naphthalen-8-carboxylate (20 mg, 0.25 mmol) in EA (1 mL), 3M HCl (2 mL) was added, and the mixture was stirred at 0 °C *f*or 1 minute. After 1 hour, LCMS showed that the starting material was consumed and the desired mass was formed. The mixture was concentrated in vacuum to obtain a residue. The residue was purified by pre-HPLC to obtain tert-butyl (6aS)-2-(8-ethyl-7-fluoro-3-(methoxymethoxy)naphthalen-1-yl)-1-fluoro-12-(((2R,7aS)-2-fluorotetrahydro-1H-pyrrolizin-7a(5H)-yl)methoxy)-5-methyl-5,6,6a,7,9,10-hexahydro-8H-4-oxa-3,8,10a,11,13-pentaazabenzo[4,5]cycloocta[1,2,3-de]naphthalen-8-carboxylate(2.63 mg, 16.1 % yield).

$^1H$ NMR (400 MHz, MeOD) δ 8.49 (brs, 1H, FA), 7.71 - 7.57 (m, 1H), 7.32 - 6.90 (m, 3H), 5.55 - 5.20 (m, 2H), 4.64 - 4.52 (m, 1H), 4.41 (dd, *J* = 59.4, 11.3 Hz, 2H), 4.02 (dd, *J* = 30.6, 12.5 Hz, 1H), 3.66 - 3.40 (m, 3H), 3.26 - 2.92 (m, 5H), 2.91 - 2.65 (m, 2H), 2.60 - 2.34 (m, 3H), 2.28 - 2.08 (m, 4H), 2.05 - 1.92 (m, 1H), 1.88 - 1.74 (m, 1H), 1.47 (d, *J* = 5.9 Hz, 3H), 0.98 - 0.82 (m, 3H). MS: *m/z* = 635.4(M+H$^+$, ESI+)

**Synthesis Reaction Equation of Example 6**

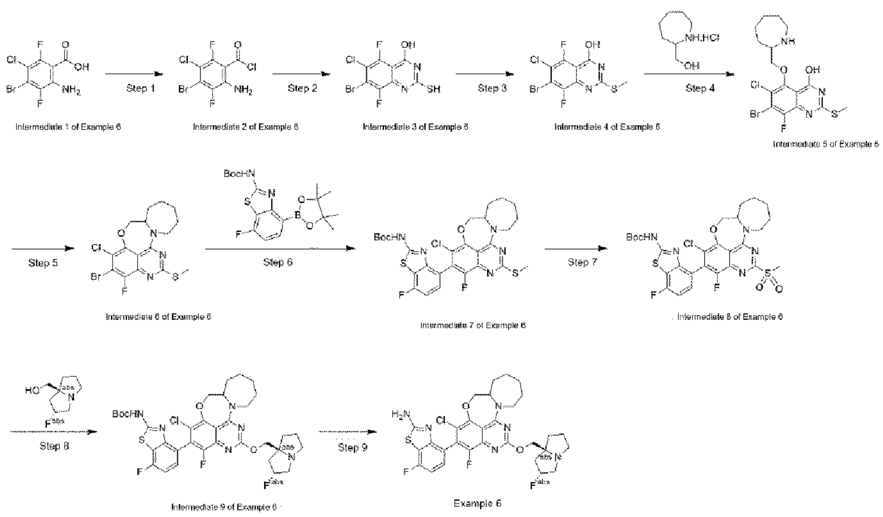


**Example 6: Preparation of 4-(3-chloro-1-fluoro-13-(((2R,7aS)-2-fluorotetrahydro-1H-pyrrolizin-7a(5H)-yl)methoxy)-5a,6,7,8,9,10-hexahydro-5H-azepino[2',1':3,4][1,4]oxazepino[5,6,7-de]quinazolin-2-yl)-7-fluorobenzo[d]thiazol-2-amine**

Step 1) Intermediate 2 of Example 6: Preparation of 2-amino-4-bromo-5-chloro-3,6-difluorobenzoyl chloride

A mixture of 2-amino-4-bromo-5-chloro-3,6-difluorobenzoic acid (2 g, 6.99 mmol) in $SOCl_2$ (20 mL) was stirred at 50 °C for 3 hours. The reaction mixture was concentrated under reduced pressure to obtain a desired product, 2-amino-4-bromo-5-chloro-3,6-difluorobenzoyl chloride (2.13 g, 100 % yield), as a brown oil. The crude product was used in the next step without further purification.

Step 2) Intermediate 3 of Example 6: Preparation of 7-bromo-6-chloro-5,8-difluoro-2-mercaptoquinazolin-4-ol

To a mixture of $NH_4SCN$ (1.6 g, 21.02 mmol) dissolved in acetone (20 mL), a solution of 2-amino-4-bromo-5-chloro-3,6-difluorobenzoyl chloride (2.13 g, 6.98 mmol) dissolved in acetone (10 mL) was added. The reaction mixture was stirred at room temperature for 1 hour. The reaction mixture was quenched with water (200 mL) and the solution was extracted with EtOAc (100 mL x 3). The combined organic phase was washed with brine (50 mL), dried over $Na_2SO_4$(s), filtered, and concentrated to obtain a desired product, 7-bromo-6-chloro-5,8-difluoro-2-mercaptoquinazolin-4-ol (2.78 g, crude compound), as a yellow solid. MS: *m/z* = 326.9(M+H$^+$, ESI+). The crude product was used in the next step without further purification.

Step 3) Intermediate 4 of Example 6: Preparation of 7-bromo-6-chloro-5,8-difluoro-2-(methylthio)quinazolin-4-ol

To a mixture of 7-bromo-6-chloro-5,8-difluoro-2-mercaptoquinazolin-4-ol (2.78 g, 8.53 mmol) in MeOH (30 mL), NaOH (1M, 17 mL, 17 mmol) and MeI (1.06 mL, 17.05 mmol) were added at room temperature. The reaction mixture was stirred at room temperature for 1.5 hours. The reaction mixture was adjusted to pH=7 using 1 M HCl, then the mixture was diluted with water (150 mL) and extracted with EtOAc (100 mL x 3). The combined organic phases were washed with brine (50 mL), dried over $Na_2SO_4$(s), filtered and concentrated to obtain a residue, which was purified by silica gel chromatography with PE/EA = 5:1 to obtain a desired product, 7-bromo-6-chloro-5,8-difluoro-2-(methylthio)quinazolin-4-ol (1.85 g, 64 % yield) as a yellow solid. MS: *m/z* = 340.9(M+H$^+$, ESI+)

Step 4) Intermediate 5 of Example 6: Preparation of 5-(azepan-2-ylmethoxy)-7-bromo-6-chloro-8-fluoro-2-(methylthio) quinazolin-4-ol

To a mixture of azepan-2-ylmethanol hydrochloride (387 mg, 2.34 mmol) in THF (5 mL), NaH (60 % in oil, 280 mg, 11.67 mmol) was added at 0 °C. The reaction mixture was stirred for 10 minutes at 0 °C under $N_2$ gas, and a solution of 7-bromo-6-chloro-5,8-difluoro-2-(methylthio)quinazolin-4-ol (794 mg, 2.33 mmol) in THF (15 mL) was added to the above mixture. The reaction mixture was stirred at 25 °C under $N_2$ gas for 16 hours. The reaction mixture was quenched with concentrated $NH_4Cl$(aq) (150 mL) and the mixture was extracted with EA (80 mL x 3). The organic phase was washed with brine (50 mL), dried over $Na_2SO_4$(s), filtered and concentrated to dryness in vacuum to obtain a yellow solid. The yellow solid was purified by silica gel chromatography and eluted with DCM/MeOH = 5:1 to obtain a desired product, 5-(azepan-2-ylmethoxy)-7-bromo-6-chloro-8-fluoro-2-(methylthio)quinazolin-4-ol (706 mg, 67.4 % yield), as a yellow solid. MS: *m/z* = 451.9(M+H$^+$, ESI+)

Step 5) Intermediate 6 of Example 6: Preparation of 2-bromo-3-chloro-1-fluoro-13-(methylthio)-5a,6,7,8,9,10-hexahydro-5H-azepino[2',1':3,4][1,4]oxazepino[5,6,7-de]quinazoline

To a mixture of 5-(azepan-2-ylmethoxy)-7-bromo-6-chloro-8-fluoro-2-(methylthio)quinazolin-4-ol (706 mg, 1.57 mmol) and DIEA (2.4 mL, 14.08 mmol) in DCM (20 mL), BOPCl (1.$_2$g, 4.71 mmol) was added at 0 °C under $N_2$ gas. The reaction mixture was stirred at room temperature under $N_2$ gas for 2 hours. The reactant was diluted with water (200 mL) and the solution was extracted with EtOAc (100 mL x 3). The combined organic phase was washed with brine (80 mL), dried over $Na_2SO_4$(s), filtered, and concentrated to dryness in vacuum to obtain a yellow solid. The yellow solid was purified by silica gel chromatography, eluted with PE/EA = 10:1, to obtain a desired product, 2-bromo-3-chloro-1-fluoro-13-(methylthio)-5a,6,7,8,9,10-hexahydro-5H-azepino[2',1':3,4][1,4]oxazepino[5,6,7-de]quinazoline (640 mg, 74.5 % yield), as a yellow solid. MS: *m/z* = 433.9(M+H$^+$, ESI+)

Step 6) Intermediate 7 of Example 6: Preparation of tert-butyl(4-(3-chloro-1-fluoro-13-(methylthio)-5a,6,7,8,9,10-hexahydro-5H-azepino[2',1':3,4][1,4]oxazepino[5,6,7-de]quinazolin-2-yl)-7-fluorobenzo[d]thiazol-2-yl)carbamate

To a mixture of 2-bromo-3-chloro-1-fluoro-13-(methylthio)-5a,6,7,8,9,10-hexahydro-5H-azepino[2',1':3,4][1,4] oxazepino[5,6,7-de]quinazoline (300 mg, 0.7 mmol), $K_3PO_4$ (443 mg, 2.1 mmol), and tert-butyl (7-fluoro-4-(4,4,5,5-tetramethyl-1,3,2-dioxaborolan-2-yl)benzo[d]thiazol-2-yl)carbamate (548 mg, 1.39 mmol) in dioxane/$H_2O$ (9:1, 5 mL), Ruphos-Pd-$G_3$ (174 mg, 0.21 mmol) was added. The solution was purged three times with $N_2$ and stirred at 100 °C for 2 hours under $N_2$ gas. The reactant was diluted with water (100 mL) and the solution was extracted with EtOAc (50 mL x 3). The combined organic phase was washed with brine (50 mL), dried over $Na_2SO_4$(s), filtered, and concentrated to dryness in vacuum to obtain a yellow solid. The yellow solid was purified by silica gel chromatography, eluted with PE/EA = 10:1, to obtain a desired product, tert-butyl(4-(3-chloro-1-fluoro-13-(methylthio)-5a,6, 1,8,9,1 0-hexahydro-5H-azepino[2',1':3,4] [1,4]oxazepino[5,6,7-de]quinazolin-2-yl)-7-fluorobenzo[d]thiazol-2-yl)carbamate (454 mg, crude compound), as a yellow solid. MS: *m/z* = 620.3(M+H$^+$, ESI+)

Step 7) Intermediate 8 of Example 6: Preparation of tert-butyl (4-(3-chloro-1-fluoro-13-(methylsulfonyl)-5a,6,7,8,9,10-hexahydro-5H-azepino[2',1':3,4][1,4]oxazepino[5,6,7-de]quinazolin-2yl)-7-fluorobenzo[d]thiazol-2-yl)carbamate

To a mixture of tert-butyl(4-(3-chloro-1-fluoro-13-(methylthio)-5a,6,7,8,9,10-hexahydro-5H-azepino[2',1':3) in ACN:$H_2O$ (3:1, 6 mL), oxone (2.2 g, 3.58 mmol) was added at 0 °C. The reaction mixture was stirred at room temperature for 5 hours. The reaction was quenched with aqueous $Na_2SO_3$ (60 mL) and extracted with EtOAc (30 mL x 3). The combined organic phase was washed with aqueous $Na_2SO_3$ (20 mL $\times$ 2) and brine (20 mL), dried over $Na_2SO_4$(s), filtered, and concentrated to dryness in vacuum to obtain a desired product, tert-butyl(4-(3-chloro-1-fluoro-13-(methylsulfonyl)-5a,6,7,8,9,10-hexahydro-5H-azepino[2',1':3,4][1,4]oxazepino[5,6,7-de]quinazolin-2-yl)-7-fluorobenzo[d]thiazol-2-yl) carbamate (434 mg, crude compound), as a yellow solid. MS: *m/z* = 652.2 (M+H$^+$, ESI+)

Step 8) Intermediate 9 of Example 6: Preparation of tert-butyl(4-(3-chloro-1-fluoro-13-(((2R,7aS)-2-fluorotetrahydro-1H-pyrrolizin-7a(5H)-yl)methoxy)-5a,6,7,8,9,10-hexahydro-5H-azepino[2',1':3,4][1,4]oxazepino[5,6,7-de]quinazolin-2-yl)-7-fluorobenzo[d]thiazol-2-yl)carbamate

To a mixture of ((2R,7aS)-2-fluorotetrahydro-1H-pyrrolizin-7a(5H)-yl)methanol (51 mg, 0.32 mmol) in THF (2 mL), NaH (60 % in oil, 39 mg, 1.62 mmol) was added at 0 °C. The reaction mixture was stirred under $N_2$ gas at 0 °C for 10 minutes, and then tert-butyl(4-(3-chloro-1-fluoro-13-(methylsulfonyl)-5a,6,7,8,9,10-hexahydro-5H-azepino[2',1':3,4] [1,4]oxazepino[5,6,7-de]quinazolin-2-yl)-7-fluorobenzo[d]thiazol-2-yl)carbamate (210 mg, 0.32 mmol) in THF (1 mL)

was added to the solution. The reaction mixture was stirred at 25 °C under $N_2$ gas for 1 hour. The reaction mixture was quenched with concentrated $NH_4Cl$(aq) (40 mL) and the mixture was extracted with EA (20 mL x 3). The organic phase was washed with brine (20 mL), dried over $Na_2SO_4$(s), filtered and concentrated to dryness in vacuum to obtain a brown solid. The brown solid was purified by silica gel chromatography, eluted with PE/EA = 1:2, to obtain a desired product, tert-butyl(4-(3-chloro-1-fluoro-13-(((2R,7aS)-2-fluorotetrahydro-1H-pyrrolizin-7a(5H)-yl)methoxy)-5a,6,7,8,9,10-hexahydro-5H-azepino[2',1':3,4][1,4]oxazepino[5,6,7-de]quinazolin-2-yl)-7-fluorobenzo[d]thiazol-2-yl)carbamate (65 mg, 27.6 % yield) as a white solid. *m/z* = 731.3(M+H$^+$, ESI+)

Step 9) Example 6: Preparation of 4-(3-chloro-1-fluoro-13-(((2R,7aS)-2-fluorotetrahydro-1H-pyrrolizin-7a(5H)-yl)methoxy)-5a,6,7,8,9,10-hexahydro-5H-azepino[2',1':3,4][1,4]oxazepino[5,6,7-de]quinazolin-2-yl)-7-fluorobenzo[d]thiazol-2-amine

To a mixture of tert-butyl (4-(3-chloro-1-fluoro-13-(((2R,7aS)-2-fluorotetrahydro-1H-pyrrolizin-7a(5H)-yl)methoxy)-5a,6,7,8,9,10-hexahydro-5H-azepino[2',1':3,4][1,4]oxazepino[5,6,7-de]quinazolin-2-yl)-7-fluorobenzo[d]thiazol-2-yl)carbamate (65 mg, 0.089 mmol) in DCM (1.5 mL), HCl/dioxane (1.5 mL) was added at room temperature. The reaction mixture was stirred at room temperature for 23 hours. The reactant was concentrated to dryness in vacuum to obtain a yellow solid. The yellow solid was purified by preparative HPLC (Waters 2767/Qda, Column: SunFire C18, 19*250 mm, 10 μm; Mobile phase A: 0.1 % FA/$H_2O$, B: ACN, Flow rate: 20 mL/min, Gradient: 20 % to 30 %) to obtain a desired product, Example 6 (22.82 mg, 40.7 % yield) as a white solid.

$^1$H NMR (400 MHz, DMSO-$d_6$) δ 8.33 (s, 0.31H, FA), 7.96 - 7.85 (m, 2H), 7.20 (t, *J* = 6.6 Hz, 1H), 7.04 (q, J = 8.0 Hz, 1H), 5.27 (d, J = 55.1 Hz, 1H), 4.87 - 4.59 (m, 2H), 4.48 - 4.37 (m, 1H), 4.11 - 3.95 (m, 3H), 3.14 - 2.96 (m, 4H), 2.87 - 2.76 (m, 1H), 2.14 - 1.55 (m, 13H), 1.30 - 1.16 (m, 1H). *m/z* = 631.4(M+H$^+$, ESI+)

**Synthesis Reaction Equation of Example 7**

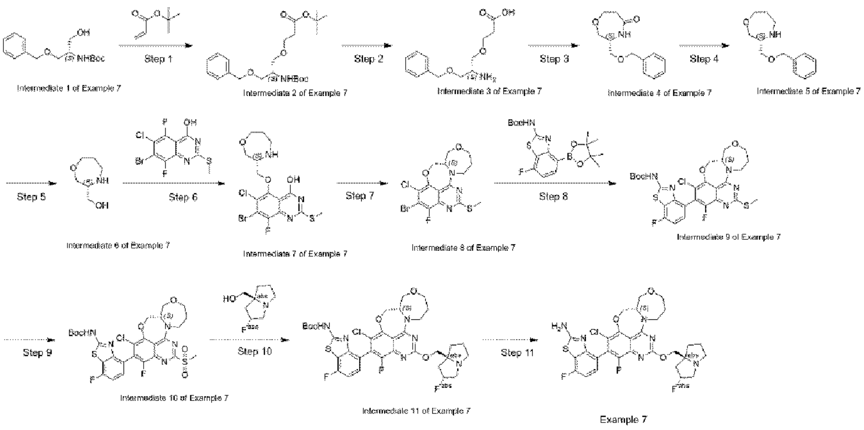


**Example 7: Preparation of 4-((14aS)-12-chloro-10-fluoro-8-(((2R,7aS)-2-fluorotetrahydro-1H-pyrrolizin-7a(5H)-yl)methoxy)-4,5,14,14a-tetrahydro-1H,3H-[1,4]oxazepino[3',4':3,4][1,4]oxazepino[5,6,7-de]quinazolin-11-yl)-7-fluorobenzo[d]thiazol-2-amine**

Step 1) Intermediate 2 of Example 7: Preparation of tert-butyl (S)-3-(3-(benzyloxy)-2-((tert-butoxycarbonyl)amino)propoxy)propanoate

To a solution of tert-butyl(S)-(1-(benzyloxy)-3-hydroxypropan-2-yl)carbamate (9 g, 31.99 mmol) and $Cs_2CO_3$ (10.6 g, 32.53 mmol) in t-BuOH (72 mL), tert-butyl acrylate (72 mL) was added. The reaction mixture was stirred at room temperature for 16 hours. The reaction mixture was diluted with water (250 mL) and the solution was extracted with ethyl acetate (150 mL x 3). The combined organic phases were washed with brine (50 mL), dried over anhydrous $Na_2SO_4$, filtered and concentrated in vacuum. The residue was purified by silica gel chromatography and purified with petroleum ether (pet ether)/ethyl acetate = 10:1 to obtain a desired product, tert-butyl (S)-3-(3-(benzyloxy)-2-((tert-butoxycarbonyl) amino)propoxy)propanoate (10.05 g, 76.8 % yield) as a colorless oil. MS: *m/z* = 410.2(M+H$^+$, ESI+)

Step 2) Intermediate 3 of Example 7: Preparation of (S)-3-(2-amino-3-(benzyloxy)propoxy)propanoic acid

A mixture of tert-butyl (S)-3-(3-(benzyloxy)-2-((tert-butoxycarbonyl)amino)propoxy)propanoate (2 g, 4.89 mmol) in HCl/dioxane (20 mL) was stirred at room temperature for 2 hours. The reactant was concentrated in vacuum to obtain a crude product (S)-3-(2-amino-3-(benzyloxy)propoxy)propanoic acid (1.81 g, HCl salt) as a yellow oil. MS: *m*/*z* = 254.0(M+$H^+$, ESI+). The crude product was used in the next step without further purification.

Step 3) Intermediate 4 of Example 7: Preparation of (S)-3-((benzyloxy)methyl)-1,4-oxazepan-5-one

To a solution of (S)-3-(2-amino-3-(benzyloxy)propoxy)propanoic acid (1.71 g, 6.72 mmol) and TEA (7.5 mL, 53.96 mmol) in DCM (18 mL), HATU (3.08 g, 8.1 mmol) was added. The reaction mixture was stirred at room temperature for 1 hour. The reaction mixture was diluted with water (150 mL) and the solution was extracted with ethyl acetate (50 mL x 3). The combined organic phases were washed with brine (50 mL), dried over anhydrous $Na_2SO_4$, filtered and concentrated in vacuum. The residue was purified by silica gel chromatography and purified with petroleum ether/ethyl acetate = 1:1 to obtain a desired product, (S)-3-((benzyloxy)methyl)-1,4-oxazepan-5-one (1.43 g, 90.5 % yield) as a yellow solid. MS: *m*/*z* = 236.1(M+$H^+$, ESI+)

Step 4) Intermediate 5 of Example 7: Preparation of (S)-3-((benzyloxy)methyl)-1,4-oxazepane

To a mixture of LAH (1 M in THF, 11.5 mL, 11.33 mmol) in THF (5 mL), a solution of (S)-3-((benzyloxy)methyl)-1,4-oxazepan-5-one (1.33 g, 5.66 mmol) in THF (5 mL) was added at 0 °C under $N_2$ gas. The reaction mixture was stirred at 25 °C under $N_2$ gas for 6 hours. The reaction mixture was quenched with $H_2O$ (1 mL) at 0 °C, and then $Na_2SO_4$ was added to the solution. The mixture was stirred at room temperature for 10 minutes. The mixture was filtered, the filtrate was collected and concentrated in vacuum. The residue was purified by silica gel chromatography and purified with petroleum ether/ethyl acetate = 1:2 to obtain a desired product, (S)-3-((benzyloxy)methyl)-1,4-oxazepane (1 g, 80 % yield) as a yellow oil. MS: *m*/*z* = 222.2(M+$H^+$, ESI+)

Step 5) Intermediate 6 of Example 7: Preparation of (R)-(1,4-oxazepan-3-yl)methanol

To a mixture of (S)-3-((benzyloxy)methyl)-1,4-oxazepane (0.95 g, 4.30 mmol) in MeOH (10 mL), Pd/C (0.5 g, 60 %) was added at room temperature. The reaction mixture was stirred at 25 °C under $H_2$ (15 psi) for 40 hours. The reactant was filtered through Celite, the filtrate was collected and concentrated in vacuum to obtain a crude product (R)-(1,4-oxazepan-3-yl)methanol (637 mg, crude compound) as a white solid. MS: *m*/*z* = 132.1(M+$H^+$, ESI+). The crude product was used in the next step without further purification.

Step 6) Intermediate 7 of Example 7: Preparation of (S)-5-((1,4-oxazepan-3-yl)methoxy)-7-bromo-6-chloro-8-fluoro-2-(methylthio)quinazolin-4-ol

To a mixture of (R)-(1,4-oxazepan-3-yl)methanol (300 mg, 2.29 mmol) in THF (4 mL), NaH (60 % in oil, 275 mg, 11.46 mmol) was added at 0°C. The reaction mixture was stirred at 0 °C under $N_2$ gas for 10 minutes, and the above solution was added to a solution of 7-bromo-6-chloro-5,8-difluoro-2-(methylthio)quinazolin-4-ol (779 mg, 2.29 mmol) in THF (4 mL). The reaction mixture was stirred at 25 °C under $N_2$ gas for 16 hours. The reaction mixture was quenched with concentrated $NH_4Cl$(aq) (150 mL) and the mixture was extracted with ethyl acetate (50 mL x 3). The organic phase was washed with brine (50 mL), dried over anhydrous $Na_2SO_4$, filtered and concentrated in vacuum. The residue was purified by silica gel chromatography and eluted with DCM/MeOH = 5:1 to obtain a desired product, (S)-5-((1,4-oxazepan-3-yl)methoxy)-7-bromo-6-chloro-8-fluoro-2-(methylthio)quinazolin-4-ol (319 mg, 30.9 % yield) as a yellow solid. MS: *m*/*z* = 454.0(M+$H^+$, ESI+)

Step 7) Intermediate 8 of Example 7: Preparation of (S)-11-bromo-12-chloro-10-fluoro-8-(methylthio)-4,5,14,14a-tetrahydro-1H,3H-[1,4]oxazepino[3',4':3,4][1,4]oxazepino[5,6,7-de]quinazoline

To a mixture of (S)-5-((1,4-oxazepan-3-yl)methoxy)-7-bromo-6-chloro-8-fluoro-2-(methylthio)quinazolin-4-ol (319 mg, 0.71 mmol) and a mixture of DIEA (1 mL, 6.05 mmol) in DCM (5 mL), BOPCl (540 mg, 2.12 mmol) was added under $N_2$ gas at 0 °C. The reaction mixture was stirred at room temperature under $N_2$ gas for 16 hours. The reactant was quenched with water (150 mL) and the solution was extracted with ethyl acetate (70 mL x 3). The combined organic phases were washed with brine (50 mL), dried over $Na_2SO_4$, filtered and concentrated in vacuum. The residue was purified by silica gel chromatography and eluted with petroleum ether/ethyl acetate = 4:1 to obtain a desired product, (S)-11-bromo-12-chloro-10-fluoro-8-(methylthio)-4,5,14,14a-tetrahydro-1H,3H-[1,4]oxazepino[3',4':3,4][1,4]oxazepino[5,6,7-de]quinazoline (142 mg, 46.4 % yield) as a yellow solid. MS: *m/z* = 435.9(M+H+, ESI+)

Step 8) Intermediate 9 of Example 7: Preparation of tert-butyl (4-((14aS)-12-chloro-10-fluoro-8-(methylthio)-4,5,14,14a-tetrahydro-1H,3H-[1,4]oxazepino[3',4':3,4][1,4]oxazepino[5,6,7-de]quinazolin-11-yl)-7-fluorobenzo[d]thiazol-2-yl)carbamate

To a mixture of (S)-11-bromo-12-chloro-10-fluoro-8-(methylthio)-4,5,14,14a-tetrahydro-1H,3H-[1,4]oxazepino[3',4':3,4][1,4]oxazepino[5,6,7-de]quinazoline (142 mg, 0.33 mmol), $K_3PO_4$ (209 mg, 0.98 mmol), and tert-butyl (7-fluoro-4-(4,4,5,5-tetramethyl-1,3,2-dioxaborolan-2-yl)benzothiazol-2-yl)carbamate (259 mg, 0.66 mmol) in dioxane/$H_2O$ (9:1, 5 mL), Ruphos-Pd-$G_3$ (82 mg, 0.10 mmol) was added. The solution was purged three times with $N_2$ and stirred at 100 °C for 2 hours under $N_2$ gas. The reactant was diluted with water (100 mL) and the solution was extracted with ethyl acetate (50 mL x 3). The combined organic phases were washed with brine (50 mL), dried over $Na_2SO_4$, filtered and concentrated in vacuum. The residue was purified by silica gel chromatography and eluted with petroleum ether/ethyl acetate = 1:1 to obtain a desired product, tert-butyl (4-((14aS)-12-chloro-10-fluoro-8-(methylthio)-4,5,14,14a-tetrahydro-1H,3H-[1,4]oxazepino[3',4':3,4][1,4]oxazepino[5,6,7-de]quinazolin-11-yl)-7-fluorobenzo[d]thiazol-2-yl)carbamate (143 mg, 70 % yield) as a yellow solid. MS: *m/z* = 622.2(M+H+, ESI+)

Step 9) Intermediate 10 of Example 7: Preparation of tert-butyl (4-((14aS)-12-chloro-10-fluoro-8-(methylsulfonyl)-4,5,14,14a-tetrahydro-1H,3H-[1,4]oxazepino[3',4':3,4][1,4]oxazepino[5,6,7-de]quinazolin-11-yl)-7-fluorobenzo[d]thiazol-2-yl)carbamate

To a mixture of tert-butyl (4-((14aS)-12-chloro-10-fluoro-8-(methylthio)-4,5,14,14a-tetrahydro-1H,3H-[1,4]oxazepino[3',4':3,4][1,4]oxazepino[5,6,7-de]quinazolin-11-yl)-7-fluorobenzo[d]thiazol-2-yl)carbamate (140 mg, 0.23 mmol) in THF:$H_2O$ (1:1, 4 mL), oxone (693 mg, 1.13 mmol) was added at 0 °C. The reaction mixture was stirred at room temperature for 1 hour. The reaction was quenched with aqueous $Na_2SO_3$ (80 mL) and the solution was extracted with ethyl acetate (30 mL x 3). The combined organic phase was washed with aqueous $Na_2SO_3$ (20 mL × 2) and brine (20 mL), dried over $Na_2SO_4$, filtered, and concentrated in vacuum to obtain a crude product, tert-butyl (4-((14aS)-12-chloro)-1 0-fluoro-8-(methylsulfonyl)-4,5, 14, 14a-tetrahydro-1H,3H-[1,4]oxazepino[3',4':3,4][1,4]oxazepino[5,6,7-de]quinazolin-11-yl)-7-fluorobenzo[d]thiazol-2-yl)carbamate (140 mg, crude compound) as a yellow solid. MS: *m/z* = 654.2(M+H+, ESI+)

Step 10) Intermediate 11 of Example 7: Preparation of tert-butyl (4-((14aS)-12-chloro-10-fluoro-8-(((2R,7aS)-2-fluorotetrahydro-1H-pyrrolizin-7a(5H)-yl)methoxy)-4,5,14,14a-tetrahydro-1H,3H-[1,4]oxazepino[3',4':3,4][1,4]oxazepino[5,6,7-de]quinazolin-11-yl)-7-fluorobenzo[d]thiazol-2-yl)carbamate

To a mixture of ((2R,7aS)-2-fluorotetrahydro-1H-pyrrolizin-7a(5H)-yl)methanol (34 mg, 0.21 mmol) in THF (2 mL), NaH (60 % in oil, 26 mg, 1.08 mmol) was added at 0 °C. The reaction mixture was stirred under $N_2$ gas at 0 °C for 10 minutes, and the above solution was added to a solution of tert-butyl (4-((14aS)-12-chloro-10-fluoro-8-(methylsulfonyl)-4,5,14,14a-tetrahydro-1H,3H-[1,4]oxazepino[3',4':3,4][1,4]oxazepino[5,6,7-de]quinazolin-11-yl)-7-fluorobenzo[d]thiazol-2-yl)carbamate (140 mg, 0.21 mmol) in THF (3 mL). The reaction mixture was stirred at 25 °C under $N_2$ gas for 16 hours. The reaction mixture was quenched with concentrated $NH_4Cl$(aq) (40 mL) and the mixture was extracted with ethyl acetate (20 mL x 3). The organic phase was washed with brine (20 mL), dried over $Na_2SO_4$, filtered and concentrated in vacuum. The residue was purified by Pre-TLC (ethyl acetate, Rf=0.4) to obtain a desired product, tert-butyl (4-((14aS)-12-chloro-10-fluoro-8-(((2R,7aS)-2-fluorotetrahydro-1H-pyrrolizin-7a(5H)-yl)methoxy)-4,5,14,14a-tetrahydro-1H,3H-[1,4]oxazepino[3',4':3,4][1,4]oxazepino[5,6,7-de]quinazolin-11-yl)-7-fluorobenzo[d]thiazol-2-yl)carbamate (30 mg, 19 % yield) as a white solid. MS: *m/z* = 733.3(M+H+, ESI+)

Step 11) Example 7: Preparation of 4-((14aS)-12-chloro-10-fluoro-8-(((2R,7aS)-2-fluorotetrahydro-1H-pyrrolizin-7a(5H)-yl)methoxy)-4,5,14,14a-tetrahydro-1H,3H-[1,4]oxazepino[3',4':3,4][1,4]oxazepino[5,6,7-de]quinazolin-11-yl)-7-fluorobenzo[d]thiazol-2-amine

A mixture of tert-butyl (4-((14aS)-12-chloro-10-fluoro-8-(((2R,7aS)-2-fluorotetrahydro-1H-pyrrolizin-7a(5H)-yl)methoxy)-4,5,14,14a-tetrahydro-1H,3H-[1,4]oxazepino[3',4':3,4][1,4]oxazepino[5,6,7-de]quinazolin-11-yl)-7-fluorobenzo[d]thiazol-2-yl)carbamate (30 mg, 0.04 mmol) in HCl/dioxane (2 mL) was stirred at room temperature for 16 hours. The reactant was concentrated in vacuum. Preparative HPLC (Waters 2767/Qda, column: SunFireSunfire C18, 19*250 mm, 10 μm; mobile phase A: 0.1 % FA/$H_2O$, B: ACN, flow rate: 20 mL/min; gradient: 20 % to 30 %) was added to the residue to obtain a desired product, Example 7 (2.47 mg, 9.5 % yield) as a white solid.

$^1H$ NMR (400 MHz, $CD_3OD$) δ 8.56 (s, 0.72H-FA), 7.24 - 7.14 (m, 1H), 6.97 (t, *J* = 8.8 Hz, 1H), 5.39 - 5.25 (m, 1H), 5.11 - 5.00 (m, 1H), 4.83 - 4.70 (m, 3H), 4.62 - 4.54 (m, 1H), 4.47 - 4.42 (m, 1H), 4.39 - 4.12 (m, 4H), 4.08 - 3.97 (m, 1H), 3.85 -

3.76 (m, 1H), 3.60 - 3.46 (m, 2H), 3.14 -3.05 (m, 1H), 2.36 - 2.17 (m, 4H), 2.06 - 1.87 (m, 4H). MS: *m/z* = 633.3(M+H$^+$, ESI+)

<u>**Synthesis Reaction Equation of Example 8**</u>

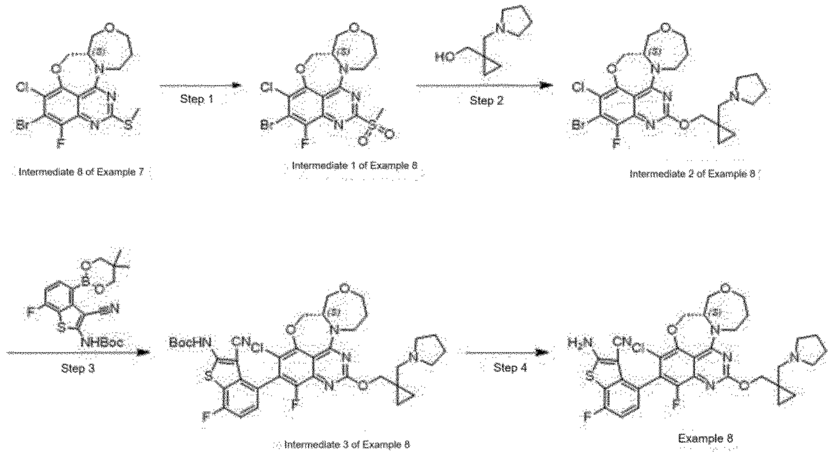


**Example 8: Preparation of 2-amino-4-((S)-12-chloro-10-fluoro-8-((1-(pyrrolidin-1-ylmethyl)cyclopropyl)methoxy)-4,5,14,14a-tetrahydro-1H,3H-[1,4]oxazepino[3',4':3,4][1,4]oxazepino[5,6,7-de]quinazolin-11-yl)-7-fluorobenzo[b]thiophene-3-carbonitrile**

<u>Step 1) Intermediate 1 of Example 8: Preparation of (S)-11-bromo-12-chloro-10-fluoro-8-(methylsulfonyl)-4,5,14,14a-tetrahydro-1H,3H-[1,4]oxazepino[3',4':3,4][1,4]oxazepino[5,6,7-de]quinazoline</u>

To a mixture of (S)-11-bromo-12-chloro-10-fluoro-8-(methylthio)-4,5,14,14a-tetrahydro-1H,3H-[1,4]oxazepino[3',4':3,4][1,4]oxazepino[5,6,7-de]quinazoline (197 mg, 0.45 mmol) in THF/$H_2O$ (1:1, 8 mL) oxone (1.4 g, 2.28 mmol) was added at 0 °C. The reaction mixture was stirred at room temperature for 1 hour. The reaction was quenched with aqueous $Na_2SO_3$ (80 mL) and the solution was extracted with ethyl acetate (30 mL x 3). The combined organic phase was washed with aqueous $Na_2SO_3$ (20 mL x 2) and brine (20 mL), dried over anhydrous $Na_2SO_4$, filtered, and the solvent was concentrated to dryness in vacuum to obtain a crude product (S)-11-bromo-12-chloro-10-fluoro-8-(methylsulfonyl)-4,5,14,14a-tetrahydro-1H,3H-[1,4]oxazepino[3',4':3,4][1,4]oxazepino[5,6,7-de]quinazoline (227 mg, crude compound) as a yellow solid. MS: *m/z* = 467.9(M+H$^+$, ESI+). The crude product was used in the next step without further purification.

<u>Step 2) Intermediate 2 of Example 8: (S)-11-bromo-12-chloro-10-fluoro-8-((1-(pyrrolidin-1-ylmethyl)cyclopropyl)methoxy)-4,5,14,14a-tetrahydro-1H,3H-[1,4]oxazepino[3',4':3,4][1,4]oxazepino[5,6,7-de]quinazoline</u>

To a stirred solution of (1-(pyrrolidin-1-ylmethyl)cyclopropyl)methanol (79 mg, 0.51 mmol) in THF (2 mL), NaH (60 % in mineral oil, 20 mg, 0.51 mmol) was added under nitrogen gas at 0 °C, and the mixture was stirred for 20 minutes. Followed by the addition of (S)-11-bromo-12-chloro-10-fluoro-8-(methylsulfonyl)-4,5,14,14a-tetrahydro-1H,3H-[1,4]oxazepino[3',4':3,4][1,4]oxazepino[5,6,7-de]quinazoline (160 mg, 0.34 mmol), and the mixture was stirred at room temperature for 16 hours. After the reaction was completed, an aqueous solution of ammonium chloride (20 mL) was added to terminate the reaction, and ethyl acetate (3 x 25 mL) was added to extract the organic layer. The obtained organic layer was washed with brine (60 mL), dried over anhydrous $Na_2SO_4$, filtered, and concentrated under reduced pressure to obtain a residue. The obtained residue was purified by silica gel column chromatography with methanol/DCM = 1:6 as the elution condition, yielding the desired compound (S)-11-bromo-12-chloro-10-fluoro-8-((1-(pyrrolidin-1-ylmethyl)cyclopropyl)methoxy)-4,5,14,14a-tetrahydro-1H,3H-[1,4]oxazepino[3',4':3,4][1,4]oxazepino[5,6,7-de]quinazoline (50 mg, 26.6 % yield).

Step 3) Intermediate 3 of Example 8: tert-butyl (4-((S)-12-chloro-10-fluoro-8-((1-(pyrrolidin-1-ylmethyl)cyclopropyl)methoxy)-4,5,14,14a-tetrahydro-1H,3H-[1,4]oxazepino[3',4':3,4][1,4]oxazepino[5,6,7-de]quinazolin-11-yl)-3-cyano-7-fluorobenzo[b]thiophene-2-yl)carbamate

To a stirred solution of (S)-11-bromo-12-chloro-10-fluoro-8-((1-(pyrrolidin-1-ylmethyl)cyclopropyl)methoxy)-4,5,14,14a-tetrahydro-1H,3H-[1,4]oxazepino[3',4':3,4][1,4]oxazepino[5,6,7-de]quinazoline (50 mg, 0.09 mmol) in 1,4-dioxane (3 mL), tert-butyl (3-cyano-4-(5,5-dimethyl-1,3,2-dioxaborinan-2-yl)-7-fluorobenzothiophene-2-yl)carbamate (73 mg, 0.18 mmol), $K_3PO_4$ (57 mg, 0.27 mmol), KF (10 mg, 0.18 mmol), and DPEphos-$PdCl_2$ (14 mg, 0.02 mmol) were added, and the mixture was stirred under nitrogen gas at 105 °C for 4 hours. After completion of the reaction, the reaction mixture was cooled to room temperature and concentrated under reduced pressure to obtain a residue. The residue was purified by reversed-phase chromatography (column: SunFire Sunfire C18, 19*250 mm, 10 μm; mobile phase A: 0.1 % HCOOH/$H_2O$, B: acetonitrile; flow rate: 20 mL/min; gradient: 35 % to 50 %) to obtain a desired compound tert-butyl (4-((S)-12-chloro-10-fluoro-8-((1-(pyrrolidin-1-ylmethyl)cyclopropyl)methoxy)-4,5,14,14a-tetrahydro-1H,3H-[1,4]oxazepino[3',4':3,4][1,4]oxazepino[5,6,7-de]quinazolin-11-yl)-3-cyano-7-fluorobenzo[b]thiophene-2-yl)carbamate (12 mg, 17.3 % yield) as a white solid. MS: *m/z* = 753.4 (M+H$^+$, ESI+)

Step 4) Example 8: 2-amino-4-((S)-12-chloro-10-fluoro-8-((1-(pyrrolidin-1-ylmethyl)cyclopropyl)methoxy)-4,5,14,14a-tetrahydro-1H,3H-[1,4]oxazepino[3',4':3,4][1,4]oxazepino[5,6,7-de]quinazolin-11-yl)-7-fluorobenzo[b]thiophene-3-carbonitrile

A stirred solution of tert-butyl (4-((S)-12-chloro-10-fluoro-8-((1-(pyrrolidin-1-ylmethyl)cyclopropyl)methoxy)-4,5,14,14a-tetrahydro-1H,3H-[1,4]oxazepino[3',4':3,4][1,4]oxazepino[5,6,7-de]quinazolin-11-yl)-3-cyano-7-fluorobenzo[b]thiophene-2-yl)carbamate (12 mg, 0.16 mmol) in 1,4-dioxane/hydrochloric acid mixed solution (4 M, 2.5 mL), was left to stand at room temperature for 6 hours. After completion of the reaction, the reaction mixture was concentrated under reduced pressure to obtain a residue. The obtained residue was purified by Prep-HPLC (column: XBridge XBridge C18 19*250 mm, 10 μm; mobile phase A: 0.1 % $NH_4HCO_3$/$H_2O$, B: acetonitrile; flow rate: 20 mL/min; gradient: 45 % to 50 %; retention time: 16 minutes, from 6.4 to 9 minutes) to obtain a desired compound 2-amino-4-((S)-12-chloro-10-fluoro-8-((1-(pyrrolidin-1-ylmethyl)cyclopropyl)methoxy)-4,5,14,14a-tetrahydro-1H,3H-[1,4]oxazepino[3',4':3,4][1,4]oxazepino[5,6,7-de]quinazolin-11-yl)-7-fluorobenzo[b]thiophene-3-carbonitrile (0.70 mg, 6.7 % yield).

$^1$H NMR (400 MHz, MeOD-d4) δ 8.55 (brs, 2.25H, FA), 7.22 (dd, *J* = 8.4, 5.1 Hz, 1H), 7.10 - 7.02 (m, 1H), 4.53 - 4.44 (m, 4H), 4.40 - 3.33 (m, 1H), 4.30 - 4.16 (m, 3H), 4.10 - 3.94 (m, 2H), 3.91 - 3.74 (m, 2H), 3.62 - 3.45 (m, 5H), 2.28 - 2.16 (m, 1H), 2.08 - 1.92 (m, 5H), 0.94 - 0.74 (m, 4H). MS: *m/z* = 653.0 (M+H$^+$, ESI+)

**Synthesis Reaction Equation of Example 11**

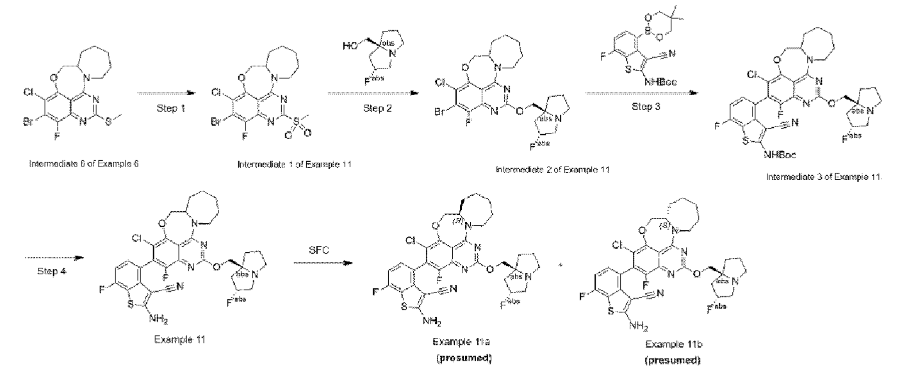


**Example 11: Preparation of 2-amino-4-(3-chloro-1-fluoro-13-(((2R,7aS)-2-fluorotetrahydro-1H-pyrrolizin-7a(5H)-yl)methoxy)-5a,6,7,8,9,10-hexahydro-5H-azepino[2',1':3,4][1,4]oxazepino[5,6,7-de]quinazolin-2-yl)-7-fluorobenzo[b]thiophene-3-carbonitrile**

Step 1) Intermediate 1 of Example 11: Preparation of 2-bromo-3-chloro-1-fluoro-13-(methylsulfonyl)-5a,6,7,8,9,10-hexahydro-5H-azepino[2',1':3,4][1,4]oxazepino[5,6,7-de]quinazoline

To a mixture of 2-bromo-3-chloro-1-fluoro-13-(methylthio)-5a,6,7,8,9,10-hexahydro-5H-azepino[2',1':3,4][1,4]oxazepino[5,6,7-de]quinazoline (300 mg, 0.70 mmol) in THF/$H_2O$ (1:1, 4 mL), oxone (2.14 g, 3.48 mmol) was added at 0 °C, and the reaction mixture was stirred at room temperature for 2 hours. The reaction was quenched with aqueous $Na_2SO_3$ (80 mL) and the solution was extracted with ethyl acetate (30 mL x 3). The combined organic phase was washed with aqueous $Na_2SO_3$ (20 mL × 2) and brine (20 mL), dried over anhydrous $Na_2SO_4$, filtered, and concentrated in vacuum to obtain a crude product 2-bromo-3-chloro-1-fluoro-13-(methylsulfonyl)-5a,6,7,8,9,10-hexahydro-5H-azepino[2',1':3,4][1,4]oxazepino[5,6,7-de]quinazoline (365 mg, crude compound) as a yellow solid. MS: *m*/*z* = 466.0(M+H$^+$, ESI+). The crude product was used in the next step without further purification.

Step 2) Intermediate 2 of Example 11: Preparation of 2-bromo-3-chloro-1-fluoro-13-(((2R,7aS)-2-fluorotetrahydro-1H-pyrrolizin-7a(5H)-yl)methoxy)-5a,6,7,8,9,10-hexahydro-5H-azepino[2',1':3,4][1,4]oxazepino[5,6,7-de]quinazoline

To a mixture of ((2R,7aS)-2-fluorotetrahydro-1H-pyrrolizin-7a(5H)-yl)methanol (125 mg, 0.79 mmol) in THF (2 mL), NaH (60 % in oil, 95 mg, 3.96 mmol) was added at 0 °C. The reaction mixture was stirred under $N_2$ gas at 0 °C for 10 minutes, and the above solution was added to a solution of 2-bromo-3-chloro-1-fluoro-13-(methylsulfonyl)-5a,6,7,8,9,10-hexahydro-13-(methylsulfonyl)-5a,6,7,8,9,10-hexahydro-5H-azepino[2',1':3,4][1,4]oxazepino[5,6,7-de]quinazoline (365 mg, 0.79 mmol) in THF (10 mL). The reaction mixture was stirred at 25 °C under $N_2$ gas for 16 hours. The reaction mixture was quenched with concentrated $NH_4Cl$(aq) (100 mL) and the mixture was extracted with ethyl acetate (50 mL x 3). The organic phase was washed with brine (30 mL), dried over anhydrous $Na_2SO_4$, filtered and concentrated to dryness in vacuum. The residue was purified by silica gel chromatography, eluted with petroleum ether/ethyl acetate = 1:2, to obtain a desired product, 2-bromo-3-chloro-1-fluoro-13-(((2R,7aS)-2-fluorotetrahydro-1H-pyrrolizin-7a(5H)-yl)methoxy)-5a,6,7,8,9,10-hexahydro-5H-azepino[2',1':3,4][1,4]oxazepino[5,6,7-de]quinazoline (319 mg, 74.7 % yield), as a yellow solid. MS: *m*/*z* = 545.0(M+H$^+$, ESI+)

Step 3) Intermediate 3 of Example 11: Preparation of tert-butyl(4-(3-chloro-1-fluoro-13-(((2R,7aS)-2-fluorotetrahydro-1H-pyrrolizin-7a(5H)-yl)methoxy)-5a,6,7,8,9,10-hexahydro-5H-azepino[2',1':3,4][1,4]oxazepino[5,6,7-de]quinazolin-2-yl)-3-cyano-7-fluorobenzo[b]thiophene-2-yl)carbamate

A mixture of 2-bromo-3-chloro-1-fluoro-13-(((2R,7aS)-2-fluorotetrahydro-1H-pyrrolizin-7a(5H)-yl)methoxy)-5a,6,7,8,9,10-hexahydro-5H-azepino[2',1':3,4][1,4]oxazepino[5,6,7-de]quinazoline (220 mg, 0.41 mmol), $Cs_2CO_3$ (396 mg, 1.22 mmol), and DPEphos-$PdCl_2$ (73 mg, 0.10 mmol) was purged with $N_2$ gas three times in 5 mL of toluene. The solution was stirred at 80 °C under $N_2$ gas for 10 minutes. Followed by the addition of tert-butyl (3-cyano-4-(5,5-dimethyl-1,3,2-dioxaborinan-2-yl)-7-fluorobenzothiophene-2-yl)carbamate (328 mg, 0.81 mmol) to the mixture. The mixture was purged three times with $N_2$ and stirred at 110 °C for 2 hours. The reactant was diluted with water (200 mL) and the solution was extracted with ethyl acetate (100 mL x 3). The combined organic phases were washed with brine (80 mL), dried over anhydrous $Na_2SO_4$, filtered and concentrated to dryness in vacuum. The residue was purified by silica gel chromatography and purified with petroleum ether/ethyl acetate = 1:2 to obtain a desired product, tert-butyl (4-(3-chloro-1-fluoro-13-(((2R,7aS)-2-fluorotetrahydro-1H-pyrrolizin-7a(5H)-yl)methoxy)-5a,6,7,8,9,10-hexahydro-5H-azepino[2',1':3,4][1,4]oxazepino[5,6,7-de]quinazolin-2-yl)-3-cyano-7-fluorobenzo[b]thiophene-2-yl)carbamate (176 mg, crude compound), as a yellow oil. MS: *m*/*z* = 755.4(M+H$^+$, ESI+)

Step 4) Intermediate 4 of Example 11: Preparation of 2-amino-4-(3-chloro-1-fluoro-13-(((2R,7aS)-2-fluorotetrahydro-1H-pyrrolizin-7a(5H)-yl)methoxy)-5a,6,7,8,9,10-hexahydro-5H-azepino[2',1':3,4][1,4]oxazepino[5,6,7-de]quinazolin-2-yl)-7-fluorobenzo[b]thiophene-3-carbonitrile

A mixture of tert-butyl (4-(3-chloro-1-fluoro-13-(((2R,7aS)-2-fluorotetrahydro-1H-pyrrolizin-7a(5H)-yl)methoxy)-5a,6,7,8,9,10-hexahydro-5H-azepino[2',1':3,4][1,4]oxazepino[5,6,7-de]quinazolin-2-yl)-3-cyano-7-fluorobenzo[b]thiophene-2-yl)carbamate (166 mg, 0.22 mmol) in HCl/dioxane (5 mL) was stirred at room temperature for 16 hours. The reactant was concentrated to dryness in vacuum. Preparative HPLC (Waters 2767/Qda, column: SunFire C18, 19*250 mm, 10 μm; mobile phase A: 0.1 % FA/$H_2O$, B: ACN, flow rate: 20 mL/min, gradient: 20 % to 30 %) was added to the residue to obtain desired products, Example 11a (40 mg) as a white solid and Example 11b (17.08 mg) as a white solid. HPLC results showed that Example 11a was not pure and was purified by preparative HPLC (Waters 2767/Qda, column: XBridge C18, 19*250 mm, 10 μm; mobile phase A: 10 mmol $NH_4HCO_3$/$H_2O$, B: ACN, flow rate: 20 mL/min, gradient: 30 % to 40 %, retention time: 7-8 min, 16 min) to obtain pure Example 11a (15.29 mg) as a white solid. MS: *m*/*z* = 655.4(M+H$^+$, ESI+)

HNMR(Example 11a): $^1$H NMR (400 MHz, DMSO-$d_6$) δ 8.04 (s, 2H), 7.30 - 7.21 (m, 1H), 7.18 - 7.10 (m, 1H),5.27 (d, *J* = 54.7 Hz, 1H), 4.86 - 4.71 (m, 1H), 4.70 - 4.58 (m, 1H), 4.52 - 4.41 (m,1H), 4.12 - 4.02 (m, 2H), 4.01 - 3.95 (m, 1H), 3.15 - 2.94 (m, 4H), 2.88 - 2.77 (m, 1H), 2.14- 1.45 (m, 14H)

HNMR(Example 11b): $^1$H NMR (400 MHz, DMSO-$d_6$) δ 8.14 (s, 0.40H-FA), 8.08 (s, 2H), 7.29 - 7.21 (m,1H), 7.19 - 7.04 (m, 1H), 5.29 (d, *J* = 53.8 Hz, 1H), 4.75 - 4.56 (m, 2H), 4.39 - 4.25 (m, 1H), 4.17 - 3.98 (m, 3H), 3.18 - 2.99 (m, 4H), 2.91 -

2.81 (m, 1H), 2.20 - 1.50 (m, 14H)

**Synthesis Reaction Equation of Example 12**

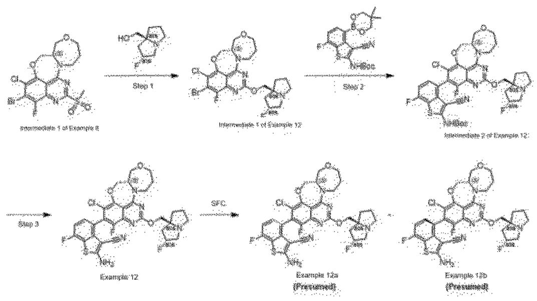


**Example 12: Preparation of 2-amino-4-((14aS)-12-chloro-10-fluoro-8-(((2R,7aS)-2-fluorotetrahydro-1H-pyrrolizin-7a(5H)-yl)methoxy)-4,5, 14, 14a-tetrahydro-1H,3H-[1,4]oxazepino[3',4':3,4][1,4]oxazepino[5,6,7-de]quinazolin-11-yl)-7-fluorobenzo[b]thiophene-3-carbonitrile**

Step 1) Intermediate 1 of Example 12: Preparation of (S)-11-bromo-12-chloro-10-fluoro-8-(((2R,7aS)-2-fluorotetrahydro-1H-pyrrolizin-7a(5H)-yl)methoxy)-4,5,14,14a-tetrahvdro-1H,3H-[1,4]oxazepino[3',4':3,4][1,4]oxazepino[5,6,7-de]quinazoline

A mixture of ((2R,7aS)-2-fluorotetrahydro-1H-pyrrolizin-7a(5H)-yl)methanol (78 mg, 0.49 mmol) in THF (5 mL) was added to NaH (60 % in oil, 60 mg, 2.5 mmol) at 0°C. The reaction mixture was stirred under $N_2$ gas at 0°C for 10 minutes, and the above solution was added to a solution of (S)-11-bromo-12-chloro-10-fluoro-8-(methylsulfonyl)-4,5,14,14a-tetrahydro-14-tetrahydro-12-chloro-10-fluoro-8-(methylsulfonyl)-4,5,14,14a-tetrahydro-1H,3H-[1,4]oxazepino[3',4':3,4][1,4]oxazepino[5,6,7-de]quinazoline (227 mg, 0.49 mmol) in THF (5 mL). The reaction mixture was stirred at 25 °C under $N_2$ gas for 16 hours. The reaction mixture was quenched with concentrated $NH_4Cl$(aq) (100 mL) and the mixture was extracted with ethyl acetate (50 mL x 3). The organic phase was washed with brine (30 mL), dried over anhydrous $Na_2SO_4$, filtered and concentrated to dryness in vacuum. The residue was purified by silica gel chromatography and eluted with ethyl acetate to obtain a desired product, (S)-11-bromo-12-chloro-10-fluoro-8-(((2R,7aS)-2-fluorotetrahydro-1H-pyrrolizin-7a(5H)-yl)methoxy)-4,5,14,14a-tetrahydro-1H,3H-[1,4]oxazepino[3',4':3,4][1,4]oxazepino[5,6,7-de]quinazoline (122 mg, 46 % yield) as a yellow oil. MS: *m*/*z* = 547.1(M+H$^+$, ESI+)

Step 2) Intermediate 2 of Example 12: Preparation of tert-butyl (4-((14aS)-12-chloro-10-fluoro-8-(((2R,7aS)-2-fluorotetrahydro-1H-pyrrolizin-7a(5H)-yl)methoxy)-4,5,14,14a-tetrahydro-1H,3H-[1,4]oxazepino[3',4':3,4][1,4]oxazepino[5,6,7-de]quinazolin-11-yl)-3-cyano-7-fluorobenzo[b]thiophene-2-yl)carbamate

A mixture of (S)-11-bromo-12-chloro-10-fluoro-8-(((2R,7aS)-2-fluorotetrahydro-1H-pyrrolizin-7a(5H)-yl)methoxy)-4,5,14,14a-tetrahydro-1H,3H-[1,4]oxazepino[3',4':3,4][1,4]oxazepino[5,6,7-de]quinazoline (91 mg, 0.17 mmol), tert-butyl(3-cyano-4-(5,5-dimethyl-1,3,2-dioxaborinan-2-yl)-7-fluorobenzo[b]thiophene-2-yl)carbamate (135 mg, 0.33 mmol), $K_3PO_4$ (107 mg, 0.5 mmol), KF (19 mg, 0.33 mmol), and DPEphos-$PdCl_2$ (24 mg, 0.034 mmol) was purged with $N_2$ gas three times in 4 mL of dioxane. Subsequently, the solution was stirred at 105 °C for 5 hours. The reaction mixture was cooled to room temperature and diluted with water (80 mL). The solution was extracted with ethyl acetate (40 mL x 3). The organic phase was washed with brine (20 mL), dried over anhydrous $Na_2SO_4$, filtered and concentrated to dryness in vacuum. The residue was purified by silica gel chromatography and eluted with ethyl acetate to obtain a desired product, tert-butyl (4-((14aS)-12-chloro-10-fluoro-8-(((2R,7aS)-2-fluorotetrahydro-1H)-pyrrolizin-7a(5H)-yl)methoxy)-4,5,14,14a-tetrahydro-1H,3H-[1,4]oxazepino[3',4':3,4][1,4]oxazepino[5,6,7-de]quinazolin-11-yl)-3-cyano-7-fluorobenzo[b]thiophene-2-yl)carbamate (40 mg, 31.7 % yield) as a yellow oil. MS: *m*/*z* = 757.3(M+H$^+$, ESI+)

Step 3) Example 12: Preparation of 2-amino-4-((14aS)-12-chloro-10-fluoro-8-(((2R,7aS)-2-fluorotetrahydro-1H-pyrrolizin-7a(5H)-yl)methoxy)-4,5,14,14a-tetrahydro-1H,3H-[1,4]oxazepino[3',4':3,4][1,4]oxazepino[5,6,7-de]quinazolin-11-yl)-7-fluorobenzo[b]thiophene-3-carbonitrile

A mixture of tert-butyl (4-((14aS)-12-chloro-10-fluoro-8-(((2R,7aS)-2-fluorotetrahydro-1H-pyrrolizin-7a(5H)-yl)methoxy)-4,5,14,14a-tetrahydro-1H,3H-[1,4]oxazepino[3',4':3,4][1,4]oxazepino[5,6,7-de]quinazolin-11-yl)-3-cyano-7-fluorobenzo[b]thiophene-2-yl)carbamate (40 mg, 0.05 mmol) in HCl/dioxane (2 mL) was stirred at room temperature for 16 hours. The solvent was removed under reduced pressure. Preparative HPLC (Waters 2767/Qda, column: SunFireSunfire C18, 19*250 mm, 10 μm; mobile phase A: 0.1 % FA/$H_2O$, B: ACN, flow rate: 20 mL/min; gradient: 20 % to 30 %) was added to the residue to obtain desired products, Example 12a (1.94 mg, 5.6 % yield) and Example 12b (1.32 mg, 3.8 % yield), as white solids. MS: $m/z$ = 657.3(M+H$^+$, ESI+)

HNMR(Example 12a): $^1$H NMR (400 MHz, $CD_3OD$) δ 7.15 (dd, $J$ = 8.4, 5.1 Hz, 1H), 7.02 -6.92 (m, 1H), 5.59 -5.33 (m, 1H), 5.06 - 4.96 (m, 1H), 4.69 - 4.36 (m, 4H), 4.27 - 4.10 (m, 2H), 4.01 - 3.69 (m,5H), 3.55 - 3.34 (m, 3H), 2.70 - 2.42 (m, 2H), 2.32 - 2.23 (m, 2H), 2.17 - 1.88 (m, 4H)

HNMR(Example 12b): $^1$H NMR (400 MHz, $CD_3OD$) δ 8.43 (brs, 0.61H-FA), 7.18 - 7.09 (m, 1H), 7.03 - 6.90(m, 1H), 5.52 - 5.31 (m, 1H), 5.04 - 4.92 (m, 1H), 4.73 - 4.63 (m, 2H), 4.59 - 4.37 (m, 4H), 4.26 - 4.18 (m, 1H), 4.17 - 4.08 (m, 1H), 3.99 - 3.89 (m, 1H), 3.85 - 3.58 (m, 4H), 3.57 - 3.43 (m, 2H), 2.57 - 2.37 (m, 2H), 2.35 - 2.24 (m, 1H), 2.22 - 2.11 (m,3H), 1.99 - 1.89 (m, 2H).

In a similar manner, compounds of Examples 1 to 15 of Table 1 below were prepared using appropriate samples for the preparation of the compounds described in each example.

[Table 1]

| Number | Structural Formula | IUPAC Name | Mass (M+H$^+$, ESI+) |
|---|---|---|---|
| 1 | 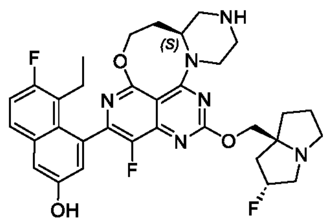 | 5-ethyl-6-fluoro-4-((S)-1-fluoro-12-(((2R,7aS)-2-fluorotetrahydro-1H-pyrrolizin-7a(5H)-yl)methoxy)-6,6a,7,8,9,10-hexahydro-5H-4-oxa-3,8,10a,11,13-pentaazabenzo[4,5]cycloocta[1,2,3-de]naphthalen-2-yl)naphthalen-2-ol | MS (LC-MS): 621.40 $m/z$ [M+H] |
| $^1$H NMR (400 MHz, DMSO-$d_6$) δ 0.76 (t, $J$ = 7.2 Hz, 2H), 0.85 (t, $J$ = 7.2 Hz, 1H), 1.75-1.79 (m, 2H), 1.79-1.81 (m, 2H), 1.99 (brs, 2H), 2.05 (brs, 1H), 2.12-2.28 (m, 2H), 2.61 (d, $J$ = 12.8 Hz, 1H), 2.82-2.86 (m, 2H), 2.93 (brs, 1H), 3.02 (s, 1H), 3.09 (d, $J$ = 4.12 Hz, 4H), 3.74-3.86 (m, 1H), 3.98 (d, $J$ = 10.26 Hz, 1H), 4.02-4.20 (m, 2H), 4.43-4.48 (m, 1H), 5.03 (t, $J$ = 12.8 Hz, 1H), 5.21 & 5.35 (brs, 1H), 6.91 & 7.11 (d, $J$ = 2.50 Hz, 1H), 7.28-7.30 (m, 1H), 7.31 (d, $J$ = 8.06 Hz, 1H), 7.74 (dd, $J$ = 8.88, 5.88 Hz, 1H), 9.92 (brs, 1H) | | | |
| 2 | 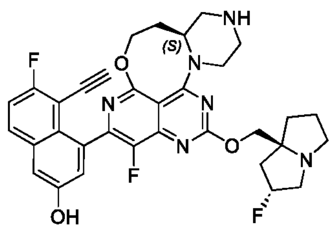 | 5-ethynyl-6-fluoro-4-((S)-1-fluoro-12-(((2R,7aS)-2-fluorotetrahydro-1H-pyrrolizin-7a(5H)-yl)methoxy)-6,6a,7,8,9,10-hexahydro-5H-4-oxa-3,8,10a,11,13-pentaazabenzo[4,5]cycloocta[1,2,3-de]naphthalen-2-yl)naphthalen-2-ol | MS (LC-MS): 617.31 $m/z$ [M+H] |
| $^1$H NMR (401 MHz, DMSO-$d_6$) δ 1.72-2.23 (m, 8H), 2.58-2.63 (m, 1H), 2.82-2.87 (m, 2H), 2.98-3.16 (m, 6H), 3.73 (dd, $J$ = 10.88, 1.38 Hz, 1H), 3.94-3.98 (m, 1H), 4.06-4.17 (m, 3H), 4.42 (dd, $J$ = 12.51, 5.50 Hz, 1H), 5.03 (d, $J$ = 12.51 Hz, 1H), 5.21-5.34 (m, 1H), 7.04-7.24 (m, 1H), 7.35-7.36 (m, 1H), 7.42-7.48 (m, 1H), 7.95 (dd, $J$ = 9.13, 5.88 Hz, 1H) | | | |
| 3 | 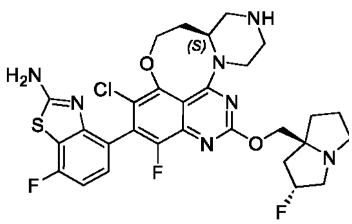 | 4-((14aS)-11-chloro-9-fluoro-7-(((2R,7aS)-2-fluorotetrahydro-1H-pyrrolizin-7a(5H)-yl)methoxy)-1,3,4,13,14,14a-hexahydro-2H-pyrazino[1',2':5,6][1,5]oxazocino[4,3, | MS (LCMS): 632.20 $m/z$ [M+H] |
| | | 2-de]quinazolin-10-yl)-7-fluorobenzo[d]thiazol-2-amine | |

(continued)

| Number | Structural Formula | IUPAC Name | Mass ($M+H^+$, ESI+) |
|---|---|---|---|
| $^1$H NMR (400 MHz, DMSO-$d_6$) 1.70-1.91 (m, 4H), 1.93-2.16 (m, 3H), 2.54-2.56 (m, 1H), 2.64-2.70 (m, 1H), 2.77-2.82 (m, 3H), 3.00-3.10 (m, 4H), 3.19-3.24 (m, 1H), 3.64-3.74 (m, 1H), 3.94 (dd, $J$ = 10.51, 2.25 Hz, 1H), 4.06 (dd, $J$ = 10.51, 2.75 Hz, 1H), 4.24 (q, $J$ = 11.6 Hz, 1H), 4.43 (brs, 1H), 4.55-4.93 (m, 1H), 5.20-5.34 (m, 1H), 7.01-7.06 (m, 1H), 7.14-7.23 (m, 1H), 7.89 (d, $J$ = 16.76 Hz, 2H) | | | |
| 4 | 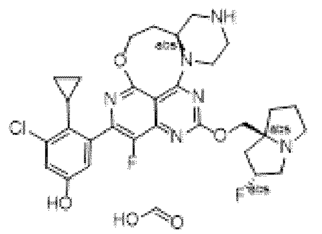 | 3-chloro-4-cyclopropyl-5-((S)-1-fluoro-12-(((2R,7aS)-2-fluorotetrahydro-1H-pyrrolizin-7a(5H)-yl)methoxy)-6,6a,7,8,9,10-hexahydro-5H-4-oxa-3,8,10a,11,13-pentaazabenzo[4,5]cycloocta[1,2,3-de]naphthalen-2-yl)phenol formate | MS: $m/z$ = 599.6($M+H^+$, ESI+) |
| $^1$H NMR (400 MHz, MeOD) δ 8.35 (brs, 2H, FA), 6.95 (d, $J$ = 2.4 Hz, 1H), 6.77 (s, 1H), 5.52 (d, $J$ = 52.6 Hz, 1H), 5.35 - 5.17 (m, 1H), 4.69 - 4.45 (m, 3H), 4.40 - 4.25 (m, 1H), 4.20 - 4.10 (m, 1H), 4.01 - 3.57 (m, 3H), 3.48 - 3.32 (m, 3H), 3.25 - 3.13 (m, 2H), 3.06 - 2.93 (m, 1H), 2.73 - 2.44 (m, 3H), 2.40 - 2.22 (m, 3H), 2.20 - 2.02 (m, 2H), 1.90 - 1.77 (m, 1H), 0.75 - 0.57 (m, 2H), 0.23 - 0.03 (m, 2H) | | | |
| 5 | 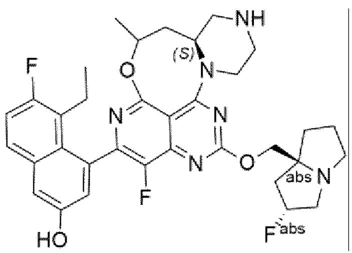 | 5-ethyl-6-fluoro-4-((6aS)-1-fluoro-12-(((2R,7aS)-2-fluorotetrahydro-1H-pyrrolizin-7a(5H)-yl)methoxy)-5-methyl-6,6a,7,8,9,10-hexahydro-5H-4-oxa-3,8,10a,11,13-pentaazabenzo[4,5]cycloocta[1,2,3-de]naphthalen-2-yl)naphthalen-2-ol | MS: $m/z$ = 635.4 ($M+H^+$, ESI+) |
| $^1$H NMR (400 MHz, MeOD) δ 8.49 (brs, 1H, FA), 7.71 - 7.57 (m, 1H), 7.32 - 6.90 (m, 3H), 5.55 - 5.20 (m, 2H), 4.64 - 4.52 (m, 1H), 4.41 (dd, $J$ = 59.4, 11.3 Hz, 2H), 4.02 (dd, $J$ = 30.6, 12.5 Hz, 1H), 3.66 - 3.40 (m, 3H), 3.26 - 2.92 (m, 5H), 2.91 - 2.65 (m, 2H), 2.60 - 2.34 (m, 3H), 2.28 - 2.08 (m, 4H), 2.05 - 1.92 (m, 1H), 1.88 - 1.74 (m, 1H), 1.47 (d, $J$ = 5.9 Hz, 3H), 0.98 - 0.82 (m, 3H) | | | |
| 6 | 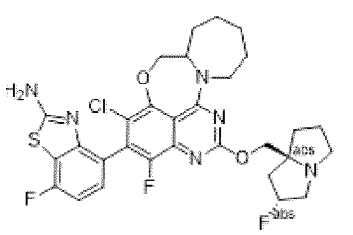 | 4-(3-chloro-1-fluoro-13-(((2R,7aS)-2-fluorotetrahydro-1H-pyrrolizin-7a(5H)-yl)methoxy)-5a,6,7,8,9,10-hexahydro-5H-azepino[2',1':3,4][1,4]oxazepino[5,6,7 -de]quinazolin-2-yl)-7-fluorobenzo[d]thiazol-2-amine | $m/z$ = 631.4 ($M+H^+$, ESI+) |
| $^1$H NMR (400 MHz, DMSO-$d_6$) δ 8.33 (s, 0.31H, FA), 7.96 - 7.85 (m, 2H), 7.20 (t, $J$ = 6.6 Hz, 1H), 7.04 (q, $J$ = 8.0 Hz, 1H), 5.27 (d, $J$ = 55.1 Hz, 1H), 4.87 - 4.59 (m, 2H), 4.48 - 4.37 (m, 1H), 4.11 - 3.95 (m, 3H), 3.14 - 2.96 (m, 4H), 2.87 - 2.76 (m, 1H), 2.14 - 1.55 (m, 13H), 1.30 - 1.16 (m, 1H) | | | |
| 7 | 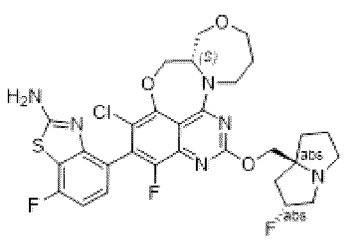 | 4-((14aS)-12-chloro-10-fluoro-8-(((2R,7aS)-2-fluorotetrahydro-1H-pyrrolizin-7a(5H)-yl)methoxy)-4,5,14,14a-tetrahydro-1H,3H-[1,4]oxazepino[3',4':3,4][1,4]oxazepin o[5,6,7-de]quinazolin-11-yl)-7-fluorobenzo[d]thiazol-2-amine | MS: $m/z$ = 633.3 ($M+H^+$, ESI+) |
| $^1$H NMR (400 MHz, $CD_3OD$) δ 8.56 (s, 0.72H-FA), 7.24 - 7.14 (m, 1H), 6.97 (t, $J$ = 8.8 Hz, 1H), 5.39 - 5.25 (m, 1H), 5.11 - 5.00 (m, 1H), 4.83 - 4.70 (m, 3H), 4.62 - 4.54 (m, 1H), 4.47 - 4.42 (m, 1H), 4.39 - 4.12 (m, 4H), 4.08 - 3.97 (m, 1H), 3.85 - 3.76 (m, 1H), 3.60 - 3.46 (m, 2H), 3.14 -3.05 (m, 1H), 2.36 - 2.17 (m, 4H), 2.06 - 1.87 (m, 4H) | | | |

(continued)

| Number | Structural Formula | IUPAC Name | Mass (M+H+, ESI+) |
|---|---|---|---|
| 8 | 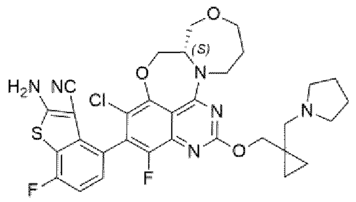 | 2-amino-4-((14aS)-12-chloro-10-fluoro-8-((1-(pyrrolidin-1-ylmethyl)cyclopropyl) methoxy)-4,5,14,14a-tetrahydro-1H,3H-[1,4] oxazepino[3',4':3,4][1,4]oxazepin o[5,6,7-de] quinazolin-11-yl)-7-fluorobenzo[b]thiophene-3-carbonitrile | MS: $m/z$ = 653.0 ($M+H^+$, ESI+) |
| $^1H$ NMR (400 MHz, MeOD-d4) δ 8.55 (brs, 2.25H, FA), 7.22 (dd, $J$ = 8.4, 5.1 Hz, 1H), 7.10 - 7.02 (m, 1H), 4.53 - 4.44 (m, 4H), 4.40 - 3.33 (m, 1H), 4.30 - 4.16 (m, 3H), 4.10 - 3.94 (m, 2H), 3.91 - 3.74 (m, 2H), 3.62 - 3.45 (m, 5H), 2.28 - 2.16 (m, 1H), 2.08 - 1.92 (m, 5H), 0.94 - 0.74 (m, 4H) | | | |
| 9 | 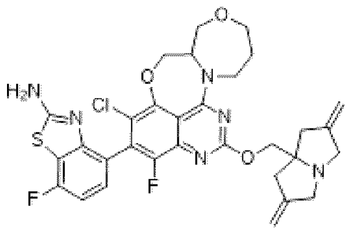 | 4-(12-chloro-8-((2,6-dimethylenetetrahydro-1H-pyrrolizin-7a(5H)-yl)methoxy)-10-fluoro-4,5,14,14a-tetrahydro-1H,3H-[1,4]oxazepino [3',4':3,4][1,4]oxazepin o[5,6,7-de]quinazolin-11-yl)-7-fluorobenzo[d]thiazol-2-amine | N/A |
| N/A | | | |
| 10 | 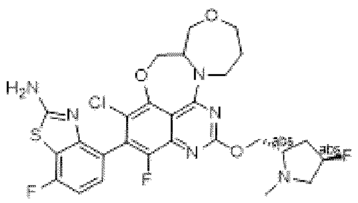 | 4-(12-chloro-10-fluoro-8-(((2S,4R)-4-fluoro-1-methylpyrrolidin-2-yl)methoxy)-4,5,14,14a-tetrahydro-1H,3H-[1,4]oxazepino[3',4':3,4][1,4] oxazepin o[5,6,7-de]quinazolin-11-yl)-7-fluorobenzo[d]thiazol-2-amine | N/A |
| N/A | | | |
| 11a (presume d) | 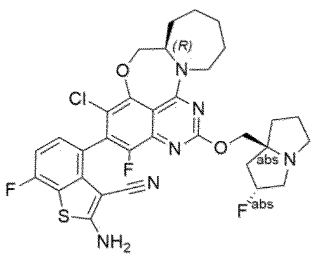 | 2-amino-4-(3-chloro-1-fluoro-13-(((2R,7aS)-2-fluorotetrahydro-1H-pyrrolizin-7a(5H)-yl)methoxy)-5a,6,7,8,9,10-hexahydro-5H-azepino [2',1':3,4][1,4]oxazepino[5,6,7 -de]quinazolin-2-yl)-7-fluorobenzo[b]thiophene-3-carbonitrile | MS: $m/z$ = 655.4 ($M+H^+$, ESI+) |
| $^1H$ NMR (400 MHz, DMSO-$d_6$) δ 8.04 (s, 2H), 7.30 - 7.21 (m, 1H), 7.18 - 7.10 (m, 1H),5.27 (d, $J$ = 54.7 Hz, 1H), 4.86 - 4.71 (m, 1H), 4.70 - 4.58 (m, 1H), 4.52 - 4.41 (m, 1H), 4.12 - 4.02 (m, 2H), 4.01 - 3.95 (m, 1H), 3.15 - 2.94 (m, 4H), 2.88 - 2.77 (m, 1H), 2.14- 1.45 (m, 14H) | | | |
| 11b (presum ed) | 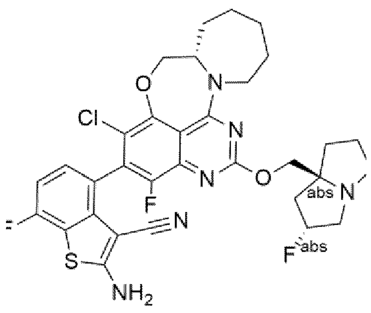 | 2-amino-4-(3-chloro-1-fluoro-13-(((2R,7aS)-2-fluorotetrahydro-1H-pyrrolizin-7a(5H)-yl)methoxy)-5a,6,7,8,9,10-hexahydro-5H-azepino [2',1':3,4][1,4]oxazepino[5,6 ,7-de]quinazolin-2-yl)-7-fluorobenzo[b]thiophene-3-carbonitrile | MS: $m/z$ = 655.4 ($M+H^+$, ESI+) |

(continued)

| Number | Structural Formula | IUPAC Name | Mass (M+H+, ESI+) |
|---|---|---|---|
| $^1$H NMR (400 MHz, DMSO-$d_6$) δ 8.14 (s, 0.40H-FA), 8.08 (s, 2H), 7.29 - 7.21 (m, 1H), 7.19 - 7.04 (m, 1H), 5.29 (d, *J* = 53.8 Hz, 1H), 4.75 - 4.56 (m, 2H), 4.39 - 4.25 (m, 1H), 4.17 - 3.98 (m, 3H), 3.18 - 2.99 (m, 4H), 2.91 - 2.81 (m, 1H), 2.20 - 1.50 (m, 14H) | | | |
| 12a (pre- sume d) | 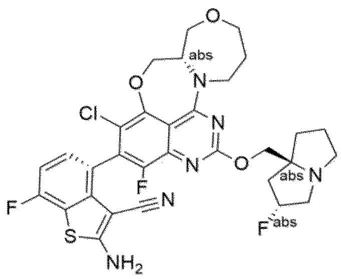 | 2-amino-4-((14aS)-12-chloro-10-fluoro-8-(((2R,7aS)-2-fluorotetrahydro-1H-pyrrolizin-7a(5H)-yl)methoxy)-4,5,14,14a-tetrahydro-1H,3H-[1,4]oxazepino[3',4':3,4][1,4]oxazepin o[5,6,7-de]quinazolin-11-yl)-7-fluorobenzo [b]thiophene-3-carbonitrile | MS: *m/z* = 657.3 (M+H+, ESI+) |
| $^1$H NMR (400 MHz, $CD_3OD$) δ 7.15 (dd, *J* = 8.4, 5.1 Hz, 1H), 7.02 - 6.92 (m, 1H), 5.59 -5.33 (m, 1H), 5.06 - 4.96 (m, 1H), 4.69 - 4.36 (m, 4H), 4.27 - 4.10 (m, 2H), 4.01 - 3.69 (m,5H), 3.55 - 3.34 (m, 3H), 2.70 - 2.42 (m, 2H), 2.32 - 2.23 (m, 2H), 2.17 - 1.88 (m, 4H) | | | |
| 12b (pre- sumed ) | 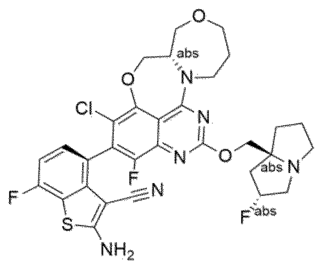 | 2-amino-4-((14aS)-12-chloro-10-fluoro-8-(((2R,7aS)-2-fluorotetrahydro-1H-pyrrolizin-7a(5H)-yl)methoxy)-4,5,14,14a-tetrahydro-1H,3H-[1,4]oxazepino[3',4':3,4][1,4]oxazepin o[5,6,7-de]quinazolin-11-yl)-7-fluorobenzo [b]thiophene-3-carbonitrile | MS: *m/z* = 657.3 (M+H+, ESI+) |
| $^1$H NMR (400 MHz, $CD_3OD$) δ 8.43 (brs, 0.61H-FA), 7.18 - 7.09 (m, 1H), 7.03 - 6.90(m, 1H), 5.52 - 5.31 (m, 1H), 5.04 - 4.92 (m, 1H), 4.73 - 4.63 (m, 2H), 4.59 - 4.37 (m, 4H), 4.26 - 4.18 (m, 1H), 4.17 - 4.08 (m, 1H), 3.99 - 3.89 (m, 1H), 3.85 - 3.58 (m, 4H), 3.57 - 3.43 (m, 2H), 2.57 - 2.37 (m, 2H), 2.35 - 2.24 (m, 1H), 2.22 - 2.11 (m,3H), 1.99 - 1.89 (m, 2H) | | | |
| 13 | 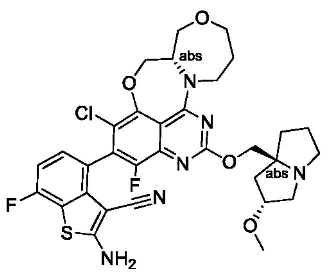 | 2-amino-4-((14aS)-12-chloro-10-fluoro-8-(((2R,7aS)-2-methoxytetrahydro-1H-pyrrolizin-7a(5H)-yl)methoxy)-4,5,14,14a-tetrahydro-1H,3H-[1,4]oxazepino[3',4':3,4][1,4]oxazepin o[5,6,7-de]quinazolin-11-yl)-7-fluorobenzo [b]thiophene-3-carbonitrile | N/A |
| N/A | | | |
| 14 | 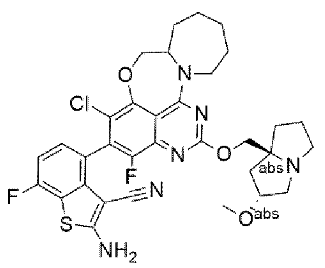 | 2-amino-4-(3-chloro-1-fluoro-13-(((2R,7aS)-2-methoxymethoxytetrahydro-1H-pyrrolizin-7a(5H)-yl)methoxy)-5a,6,7,8,9,10-hexahydro-5H-azepino[2',1':3,4][1,4]oxazepino[5,6,7-de]quinazolin-2-yl)-7-fluorobenzo[b]thiophene-3-carbonitrile | 667.3 (peak 1), 667.4 (peak 2) |
| N/A | | | |

(continued)

| Number | Structural Formula | IUPAC Name | Mass ($M+H^+$, ESI+) |
|---|---|---|---|
| 15 | 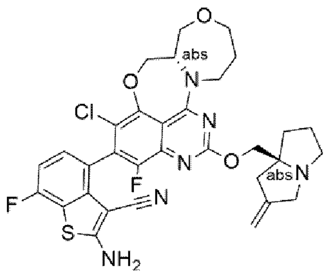 | 2-amino-4-((14aS)-12-chloro-10-fluoro-8-(((S)-2-methylenetetrahydro-1H-pyrrolizin-7a(5H)-yl)methoxy)-4,5,14,14a-tetrahydro-1H,3H-[1,4]oxazepino[3',4':3,4][1,4]oxazepino [5,6,7-de]quinazolin-11-yl)-7-fluorobenzo [b]thiophene-3-carbonitrile | 651.2 |
| N/A | | | |

**<Test Example 1: KRAS Nucleotide Exchange Assay>**

Test Objective

Evaluation of the inhibitory effect of compounds on SOS1-mediated nucleotide exchange activity of $KRAS^{WT}$, $KRAS^{G12D}$, and $KRAS^{G12V}$ mutants

Test Principle

A method to monitor SOS1-mediated exchange of unlabeled KRAS-bound GDP and fluorescently labeled GTP, based on energy transfer between the donor Tb cryptate-labeled GST antibody and the acceptor DY-647P1 GTP when they are in close proximity.

Test Conditions and Procedures

Material

GST-tagged KRAS WT or mutant proteins (amino acids 2-169),
SOS1(amino acids 564-1049),
Labeled GTP (GTP-DY-647P1),
Assay Buffer (20 mM HEPES pH 7.4, 150 mM NaCl, 5 mM $MgCl_2$, 1 mM DTT, 0.05% BSA, 0.0025% NP40)

Test Procedure

1. KRAS protein was diluted with assay buffer to 1.5 times the final concentration, mixed with Tb cryptate anti-GST antibody, and 10 μL was added to each assay well (final concentration was 20 nM for $KRAS^{WT}$, $KRAS^{G12D}$, and $KRAS^{G12V}$).
2. The compound was dissolved in DMSO and then diluted to prepare 100 times the final concentration.
3. Compounds were dispensed into assay wells using an ECHO acoustic dispenser (Beckman), gently mixed with the KRAS/Ab mixture, and incubated for 60 minutes.
4. SOS1 and labeled GTP were mixed and diluted to 3 times the final concentration with assay buffer, and 5 μL of the solution was added to the assay well to initiate the reaction (final concentration of labeled GTP was 0.15 μM, final concentration of SOS1 was 7.5 nM in WT, 12.5 nM in G12D, and 50 nM in G12V). Blank wells contained only assay buffer and labeled GTP.
5. The reaction was monitored at Ex/Em=(337/665; 337/620) using a Pherastar Plate Reader (BMG).
6. The HTRF signal was analyzed approximately 25 minutes after the start of the reaction (G12V reacted for 60 minutes).

7. Nucleotide exchange activity was expressed as a percentage difference compared to the DMSO reaction value, and the $IC_{50}$ value was calculated based on the four-parameter logistic equation in GraphPad 4.0 software.

$$HTRF\ signal = ems\ 665/ems\ 620*10000$$

The test results are shown in Table 2.

[Table 2]

| Test Results | | | | | |
|---|---|---|---|---|---|
| Example | G12D $IC_{50}$ (nM) | G12V $IC_{50}$ (nM) | G12C $IC_{50}$ (nM) | G13D $IC_{50}$ (nM) | WT $IC_{50}$ (nM) |
| 1 | 5.95 | - | - | - | 6.5 |
| 2 | 2.75 | 2.89 | - | - | - |
| 3 | 7.84 | - | - | - | - |
| 4 | 52.5 | - | - | - | - |
| 5 | 12.5 | - | - | - | - |
| 6 | 5.90 | 5.90 | 2.36 | 11.1 | - |

**<Test Example 2: KRAS NanoBRET Assay>**

Test Objective

NanoBRET target binding assessment of compounds to KRAS (WT, G12D or G12V) HEK293 cells

Test Conditions and Procedures

Compound Preparation

Test compounds were dissolved as 10 mM stocks, and reference compounds BI-2582 and MRTX1133 (MedChemExpress) were dissolved in DMSO as 10 mM and 1 mM stocks, respectively.

Cell Culture

NanoBRET KRAS (WT, G12D, or G12V)-NanoLuc Fusion vector and tracer K-2 were purchased from Promega, and the HEK293 cell line was purchased from ATCC. HEK293 cells were cultured in EMEM medium supplemented with 10 % FBS and 100 μg/mL penicillin-streptomycin at 37 °C in a humidified atmosphere of 5 % $CO_2$ and 95 % air.

Test Procedure

1. Transfect HEK293 cells with NanoBRET KRAS (WT, G12D or G12V)-NanoLuc Fusion Vector.
2. Adjust the density of transfected cells to 2 X$10^5$ cells/mL in Opti-MEM without phenol red and mix 20x K-2 tracer with the cells.
3. Dispense the cells and tracer mixture into 384 wells and place in an incubator at 37 °C, 5 % $CO_2$ for one hour, then leave at room temperature for 15 minutes.
4. The substrate and test compound solutions were treated in 384 wells including cells and tracers and reacted at room temperature for 15 minutes.
5. Measurements were made at the donor emission wavelength (460 nm) and acceptor emission wavelength (600 nm) using an Envision 2104 plate reader.
6. The BRET ratio was calculated by dividing the acceptor emission value (600 nm) by the donor emission value (460 nm), and the background was corrected by subtracting the BRET ratio that does not include the tracer.
6. The BRET response was calculated as the BRET ratio when treated with DMSO * 100 compared to the BRET ratio when treated with the compound.

7. $IC_{50}$ values were calculated based on the sigmoidal dose-response equation in the GraphPad Prism 4 program.

**<Test Example 3: Cell Proliferation Assay>**

Test Objective

Cell viability test of test compounds in AsPC-1 pancreatic cancer (KRAS G12D mutation) or SW480 colon cancer (KRAS G12V mutation) cells for 72 hours

Test Conditions and Procedures

Material

The reference compound Staurosporine was purchased from Sigma-Aldrich (Saint Louis, MI), and CellTiter-Glo® 2.0 Luminescent cell viability assay reagent (cat# G9243) was purchased from Promega (Madison, WI). AsPC-1 and SW480 cell lines were purchased from the American Type Culture Collection (Manassas, VA). AsPC-1 cells were cultured in RPMI-1640 (ATCC, cat#30-2001), and SW480 cells were cultured in DMEM (ATCC cat#30-2002), supplemented with 10% FBS (Sigma-Aldrich, cat#F2442) and 100 μg/mL penicillin-streptomycin (Sigma-Aldrich, cat#P4333). Cells were cultured at 37 °C in a humidified atmosphere of 5 % $CO_2$ and 95 % air.

Test Procedure

1. The test compound and reference compound Staurosporine were dissolved in DMSO solution to prepare 20 mM (test compound) and 10 mM (reference compound Staurosporine) in the Source Plate, and diluted 3-fold and 10 doses with DMSO.
2. Ten volumes of 125 nL test compound or 25 nL reference compound from the Source Plate were dispensed into wells of a 384-well culture plate (VWR, cat#82050-076) using an Echo 655.
3. 25 μL of culture medium including 2000 AsPC-1 or SW480 cells was dispensed into each 384-well cell culture plate.
4. Cells were cultured with compounds at 37 °C, 5 % $CO_2$for 72 hours.
5. 25 μL of CellTiter-Glo 2.0 reagent was added to each well of the plate.
6. The contents were mixed in an orbital shaker for 2 minutes and the luminescence signal was stabilized at room temperature for 15 minutes.
7. Luminescence signals were measured with an Envision 2104 Multilabel Reader (PerkinElmer, Santa Clara, CA), and the number of viable cells was determined by quantifying the ATP present in each culture medium.
8. $IC_{50}$ values were calculated based on the sigmoidal dose-response equation in the GraphPad Prism 4 program.

The test results are shown in Table 3.

[Table 3]

| Test Results | | |
|---|---|---|
| Example | AsPC-1 | SW480 |
| 1 | C | - |
| 2 | C | C |
| 3 | B | - |
| 6 | A | - |
| 7 | A | - |
| 11a | B | - |
| 11b | A | - |
| 12a | B | - |

(continued)

| Test Results | | |
|---|---|---|
| Example | AsPC-1 | SW480 |
| 12b | A | - |
| ($IC_{50}$ < 2 $\mu$M = A; $IC_{50} \geq$ 2 $\mu$M, < 20 $\mu$M = B; $IC_{50}$ > 20 $\mu$M = C) | | |

So far, the present disclosure has been looked at with respect to its embodiments. While the present disclosure has been specifically illustrated and described with reference to desirable examples and various alternative examples, it may be understood by a person of ordinary skill in the art that various changes in form and detail may be made therein without departing from the spirit and scope of the present disclosure.

## Claims

**1.** A compound of the following Formula 1, or a stereoisomer, diastereomer, enantiomer or atropisomer thereof, a solvate thereof, an isotopic variant thereof, a tautomer thereof, or a pharmaceutically acceptable salt thereof:

[Formula 1]

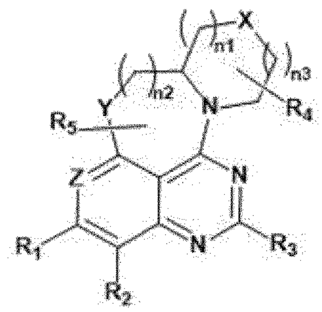

wherein in the formula above,

$R^1$ is phenyl, pyridinyl, naphthyl, indazolyl, benzothiazolyl, or benzothiophenyl, being unsubstituted or optionally substituted with one or more substituents independently selected from hydroxy, halogen, $C_{1-3}$ haloalkyl, $C_{1-3}$ alkyl, $C_{2-6}$ alkenyl, $C_{2-6}$ alkynyl, $C_{3-6}$ cycloalkyl, $C_{1-3}$ alkoxy, amino, and cyano;
$R^2$ is hydrogen or a halogen;
$R^3$ is hydrogen, $C_{1-3}$ alkyl, $C_{3-6}$ cycloalkyl, 4-10 membered heterocycle, or - O-$(L)_m$-$A_1$, wherein $A_1$ is $C_{1-3}$ alkyl, $C_{3-6}$ cycloalkyl, a 4-10 membered heterocycle, a 6-10 membered aryl, a 5-10 membered heteroaryl, or a 5-10 membered fused heteroaryl, each of which is unsubstituted or substituted with one or more $R^7$;
L is $C_{1-3}$ alkylene or $C_{3-8}$ cycloalkylene, each of which is unsubstituted or substituted with $R^7$;
each $R^7$ is independently a halogen, oxo(=O), =$CH_2$, -$OCF_3$, -$OCHF_2$, amino, cyano, -N($C_{1-3}$ alkyl)$_2$, -NH($C_{1-3}$ alkyl), a substituted or unsubstituted $C_{1-3}$ alkyl, a substituted or unsubstituted $C_{1-3}$ haloalkyl, a substituted or unsubstituted $C_{1-3}$ alkoxy, a substituted or unsubstituted $C_{3-4}$ cycloalkyl, or a substituted or unsubstituted heterocycle;
each $R^4$ is independently hydrogen, hydroxy, a halogen, $C_{1-3}$ haloalkyl, $C_{1-3}$ alkyl, or $C_{1-3}$ alkoxy;
X is O, $CH_2$ or $NR^8$;
$R^8$ is hydrogen or is $C_{1-6}$ alkyl, $C_{3-6}$ cycloalkyl or $C_{3-6}$ heterocycloalkyl optionally substituted 1-3 times with $R^9$;
$R^9$ is independently oxygen, hydroxy, -$C_{1-4}$ alkyl or -O-$C_{1-4}$ alkyl in each case;
Y is NH, O, S, SO, or $SO_2$;
Z is $CR^6$ or N;
$R^5$ is hydrogen, $C_{1-3}$ alkyl, cyano, or amino;
$R^6$ is hydrogen, hydroxy, a halogen, or $C_{1-3}$ alkyl;
n1, n2, n3, and m are each an integer of 1 to 3;
wherein heterocycles, heteroaryls, and fused heteroaryls each comprise one or more of N, S or O as a heteroatom.

**2.** The compound of claim 1,

wherein $R^1$ is phenyl, naphthyl, benzothiazolyl, or benzothiophenyl, being unsubstituted or optionally substituted with one or more substituents independently selected from hydroxy, halogen, $C_{1-3}$ haloalkyl, $C_{1-3}$ alkyl, $C_{2-6}$ alkenyl, $C_{2-6}$ alkynyl, $C_{3-6}$ cycloalkyl, $C_{1-3}$ alkoxy, amino, and cyano;
X is O, $CH_2$, or NH;
Y is O.

**3.** The compound of claim 1,
wherein $R^3$ is -O-$(L)_m$-$A_1$, where $A_1$ is a 4-10 membered heterocycle or a 5-10 membered fused heteroaryl, each of which is unsubstituted or substituted with one or more $R^7$.

**4.** The compound of claim 1,

wherein $R^1$ is

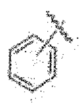 , 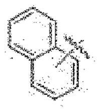 , 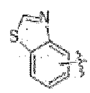 , or 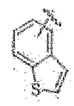 ,

being unsubstituted or optionally substituted with one or more substituents independently selected from hydroxy, halogen, $C_{1-3}$ alkyl, $C_{2-6}$ alkenyl, $C_{2-6}$ alkynyl, $C_{3-6}$ cycloalkyl, amino, and cyano;
$R^3$ is -O-$(L)_m$-$A_1$, where $A_1$ is a 4-10 membered heterocycle or a 5-10 membered fused heteroaryl, each of which is unsubstituted or substituted with one or more $R^7$;
L is $C_{1-3}$ alkylene or $C_{3-8}$ cycloalkylene;
each $R^7$ is independently a halogen, oxo (=O), $=CH_2$, $-OCF_3$, $-OCHF_2$, amino, cyano, $C_{1-3}$ alkyl, $C_{1-3}$ haloalkyl, or $C_{1-3}$ alkoxy;
$R^4$ is hydrogen;
X is O, $CH_2$, or NH;
Z is $CR^6$ or N;
$R^5$ is hydrogen or $C_{1-3}$ alkyl;
$R^6$ is a halogen;
and n1, n2, n3, and m are each independently an integer of 1 to 3.

**5.** The compound of claim 1,

wherein when Z is N,
$R^1$ is phenyl or naphthyl, being unsubstituted or optionally substituted with one or more substituents independently selected from hydroxy, a halogen, $C_{1-3}$ haloalkyl, $C_{1-3}$ alkyl, $C_{2-6}$ alkenyl, $C_{2-6}$ alkynyl, $C_{3-6}$ cycloalkyl, $C_{1-3}$ alkoxy, amino, and cyano;
wherein when Z is $CR^6$,
$R^1$ is benzothiazolyl or benzothiophenyl, being unsubstituted or optionally substituted with one or more substituents independently selected from hydroxy, halogen, $C_{1-3}$ haloalkyl, $C_{1-3}$ alkyl, $C_{2-6}$ alkenyl, $C_{2-6}$ alkynyl, $C_{3-6}$ cycloalkyl, $C_{1-3}$ alkoxy, amino, and cyano.

**6.** The compound of claim 1, wherein $R^3$ is -O-$(L)_m$-$A_2$, where $A_2$ is

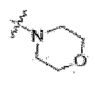 ,

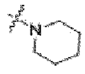 , 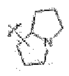 , 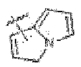 , 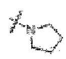 , 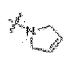 , or 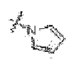 ,

each of which is unsubstituted or optionally substituted with one or more substituents independently selected from

a halogen, $C_{1-3}$ alkyl, $C_{1-3}$ alkoxy, and $=CH_2$; and $-(L)_m-$ is methylene or

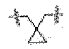.

**7.** The compound of claim 1,

wherein $R^1$ is

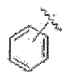, 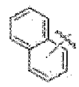, 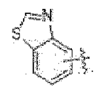, or 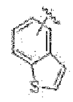,

being unsubstituted or optionally substituted with one or more substituents independently selected from hydroxy, a halogen, $C_{1-3}$ alkyl, $C_{2-6}$ alkenyl, $C_{2-6}$ alkynyl, $C_{3-6}$ cycloalkyl, amino, and cyano;
$R^2$ is a halogen;
$R^3$ is $-O-(L)_m-A_1$, where $A_1$ is

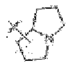, 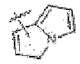, 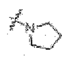, 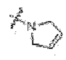, or 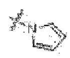,

each of which is unsubstituted or optionally substituted with one or more substituents independently selected from a halogen, $C_{1-3}$ alkyl, $C_{1-3}$ alkoxy, and $=CH_2$, where $-(L)_m-$ is methylene or

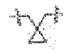;

$R^4$ is hydrogen;
X is O, $CH_2$, or NH;
Y is O;
Z is CH, $C(C_{1-3}$ alkyl), or N;
and $R^5$ is hydrogen or $C_{1-3}$ alkyl.

**8.** The compound of claim 1, wherein the compound of Formula 1 is any one compound selected from the group consisting of the following compounds, or a stereoisomer, diastereomer, enantiomer, or atropisomer thereof, a solvate thereof, an isotopic variant thereof, a tautomer thereof, or a pharmaceutically acceptable salt thereof:

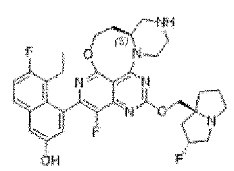, 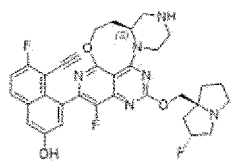,

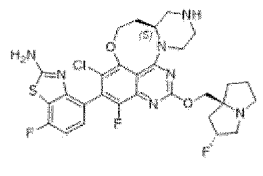, 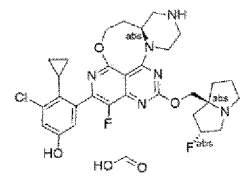,

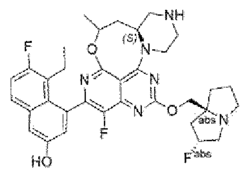,
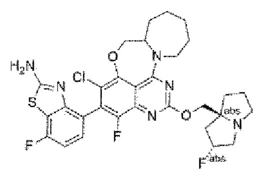,

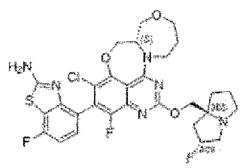,
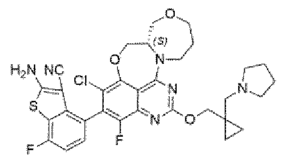,

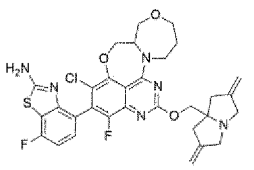,
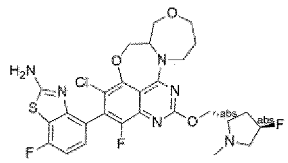,

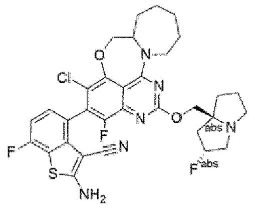,
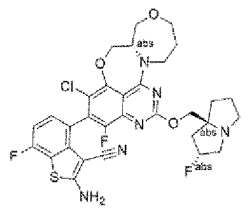,

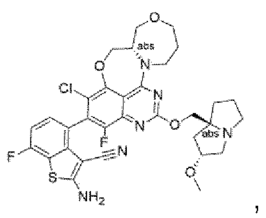,
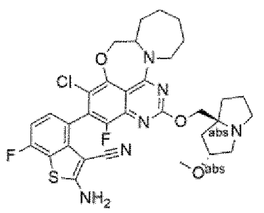,

and

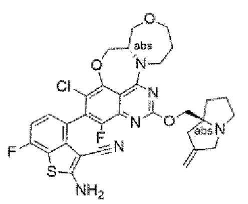.

**9.** A pharmaceutical composition for treating cancer, comprising, as an active ingredient, a compound of any one of claims 1 to 8, or a stereoisomer, diastereomer, enantiomer, or atropisomer thereof, a solvate thereof, an isotopic variant thereof, a tautomer thereof, or a pharmaceutically acceptable salt thereof.

**10.** The pharmaceutical composition of claim 9, exhibiting KRAS protein inhibitory activity.

## INTERNATIONAL SEARCH REPORT

International application No.
**PCT/KR2023/014444**

**A. CLASSIFICATION OF SUBJECT MATTER**

**C07D 498/22**(2006.01)i; **A61K 31/519**(2006.01)i; **A61P 35/00**(2006.01)i

According to International Patent Classification (IPC) or to both national classification and IPC

**B. FIELDS SEARCHED**

Minimum documentation searched (classification system followed by classification symbols)

C07D 498/22(2006.01); A61K 31/553(2006.01); A61P 35/00(2006.01); C07D 471/00(2006.01); C07D 471/04(2006.01); C07D 498/14(2006.01); C07D 498/16(2006.01); C07D 519/00(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal), STN (registry, caplus) & keywords: 테트라헤테로사이클 화합물(tetraheterocyclic compound), KRAS 저해제(KRAS inhibitor), 암(cancer)

**C. DOCUMENTS CONSIDERED TO BE RELEVANT**

| Category* | Citation of document, with indication, where appropriate, of the relevant passages | Relevant to claim No. |
|---|---|---|
| X | WO 2022-188729 A1 (JACOBIO PHARMACEUTICALS CO., LTD.) 15 September 2022 (2022-09-15) See claims 1, 29, 31-32 and 34; pages 1 and 28; and table 10. | 1-10 |
| X | WO 2022-194245 A1 (GENFLEET THERAPEUTICS (SHANGHAI) INC. et al.) 22 September 2022 (2022-09-22) See abstract; claims 1 and 13-15; and page 62, table C. | 1-10 |
| X | WO 2022-173678 A1 (GENENTECH, INC.) 18 August 2022 (2022-08-18) See claims 1, 65 and 78-82; and pages 1 and 61. | 1-10 |
| A | KR 10-2021-0006948 A (ASTRAZENECA AB) 19 January 2021 (2021-01-19) See entire document. | 1-10 |
| A | WO 2021-108683 A1 (REVOLUTION MEDICINES, INC.) 03 June 2021 (2021-06-03) See entire document. | 1-10 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **22 December 2023** | **26 December 2023** |
| Name and mailing address of the ISA/KR<br>**Korean Intellectual Property Office**<br>**Government Complex-Daejeon Building 4, 189 Cheongsa-ro, Seo-gu, Daejeon 35208**<br>Facsimile No. **+82-42-481-8578** | Authorized officer<br><br>Telephone No. |

Form PCT/ISA/210 (second sheet) (July 2022)

**INTERNATIONAL SEARCH REPORT**
**Information on patent family members**

International application No.
**PCT/KR2023/014444**

| Patent document cited in search report | | | Publication date (day/month/year) | Patent family member(s) | | | Publication date (day/month/year) |
|---|---|---|---|---|---|---|---|
| WO | 2022-188729 | A1 | 15 September 2022 | None | | | |
| WO | 2022-194245 | A1 | 22 September 2022 | CA | 3211725 | A1 | 22 September 2022 |
| WO | 2022-173678 | A1 | 18 August 2022 | CN | 116867792 | A | 10 October 2023 |
| | | | | TW | 202241912 | A | 01 November 2022 |
| | | | | US | 2022-0281893 | A1 | 08 September 2022 |
| KR | 10-2021-0006948 | A | 19 January 2021 | CN | 112074520 | A | 11 December 2020 |
| | | | | EP | 3790884 | A1 | 17 March 2021 |
| | | | | JP | 2021-532061 | A | 25 November 2021 |
| | | | | JP | 7138724 | B2 | 16 September 2022 |
| | | | | TW | 202012415 | A | 01 April 2020 |
| | | | | US | 11407765 | B2 | 09 August 2022 |
| | | | | US | 2021-0221823 | A1 | 22 July 2021 |
| | | | | WO | 2019-215203 | A1 | 14 November 2019 |
| WO | 2021-108683 | A1 | 03 June 2021 | CN | 114980976 | A | 30 August 2022 |
| | | | | EP | 4065231 | A1 | 05 October 2022 |
| | | | | JP | 2023-505100 | A | 08 February 2023 |
| | | | | US | 2023-0100838 | A1 | 30 March 2023 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- *Scientific Reports*, vol. 6 (1), 21949
- *J Cancer Metastasis Treat*, 2021, vol. 7, 26
- *Cancer Biol Ther.*, August 2006, vol. 5 (8), 928-932